(19) Europäisches Patentamt
European Patent Office
Office européen des brevets

(11) EP 2 848 939 A1

(12) EUROPEAN PATENT APPLICATION

(43) Date of publication:
**18.03.2015 Bulletin 2015/12**

(51) Int Cl.:
***G01N 33/574*** *(2006.01)*

(21) Application number: **14188048.4**

(22) Date of filing: **18.07.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**19.07.2010 EP 10170008**
**19.07.2010 EP 10170004**
**17.11.2010 US 414853 P**
**16.06.2011 US 201161497753 P**

(62) Document number(s) of the earlier application(s) in accordance with Art. 76 EPC:
**11732484.8 / 2 596 358**

(27) Previously filed application:
**18.07.2011 PCT/EP2011/062228**

(71) Applicant: **F. Hoffmann-La Roche AG**
**4070 Basel (CH)**

(72) Inventors:
- **Andres, Herbert**
  **82377 Penzberg (DE)**
- **De Haas, Sanne Lysbet**
  **4056 Basel (CH)**
- **Elliott, Rebecca**
  **South San Francisco, CA 94080 (US)**
- **Karl, Johann**
  **82380 Peissenberg (DE)**
- **Meng, Yu-Ju**
  **South San Francisco, CA 94080 (US)**
- **Plowman, Gregory**
  **San Carlos, CA 94070 (US)**
- **Scherer, Stefan**
  **1028 Preverenges (CH)**
- **Wild, Norbert**
  **82538 Geretsried/Gelting (DE)**

(74) Representative: **Burger, Alexander et al**
**Roche Diagnostics GmbH**
**Patent Department (LPP.....6164)**
**P.O.Box 11 52**
**82372 Penzberg (DE)**

Remarks:
This application was filed on 08-10-2014 as a divisional application to the application mentioned under INID code 62.

(54) **Method to identify a patient with an increased likelihood of responding to an anti-cancer therapy**

(57) The invention provides methods for identifying patient who may benefit from treatment with an anti-cancer therapy comprising a VEGF antagonist. The invention also provides methods for monitoring a patient's response to the anti-cancer therapy. The invention also provides kits and articles of manufacture for use in the methods.

Figure 12

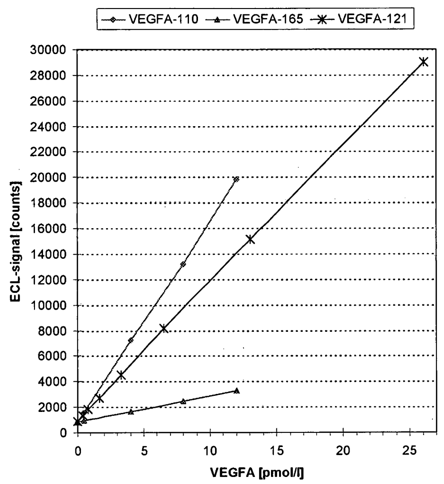


EP 2 848 939 A1

SEQUENCE LISTING

<110> Genentech, Inc.
F. Hoffmann-La Roche AG

<120> METHOD TO IDENTIFY A PATIENT WITH AN INCREASED LIKELIHOOD OF RESPONDING TO AN ANTI-CANCER THERAPY

<130> 27121 WO-WN

<150> US 61/414853
<151> 2010-11-17

<150> US 61/497753
<151> 2011-06-16

<150> EP 10170004.5
<151> 2010-07-19

<150> EP 10170008.6
<151> 2010-07-19

<160> 3

<170> PatentIn version 3.5

<210> 1
<211> 232
<212> PRT
<213> Homo sapiens

<400> 1

Met Asn Phe Leu Leu Ser Trp Val His Trp Ser Leu Ala Leu Leu Leu
1 5 10 15

Tyr Leu His His Ala Lys Trp Ser Gln Ala Ala Pro Met Ala Glu Gly
20 25 30

Gly Gly Gln Asn His His Glu Val Val Lys Phe Met Asp Val Tyr Gln
35 40 45

Arg Ser Tyr Cys His Pro Ile Glu Thr Leu Val Asp Ile Phe Gln Glu
50 55 60

Tyr Pro Asp Glu Ile Glu Tyr Ile Phe Lys Pro Ser Cys Val Pro Leu
65 70 75 80

Met Arg Cys Gly Gly Cys Cys Asn Asp Glu Gly Leu Glu Cys Val Pro
85 90 95

Thr Glu Glu Ser Asn Ile Thr Met Gln Ile Met Arg Ile Lys Pro His
100 105 110

Gln Gly Gln His Ile Gly Glu Met Ser Phe Leu Gln His Asn Lys Cys
115 120 125

Glu Cys Arg Pro Lys Lys Asp Arg Ala Arg Gln Glu Lys Lys Ser Val

130 135 140

Arg Gly Lys Gly Lys Gly Gln Lys Arg Lys Arg Lys Lys Ser Arg Tyr
145 150 155 160

Lys Ser Trp Ser Val Tyr Val Gly Ala Arg Cys Cys Leu Met Pro Trp
165 170 175

Ser Leu Pro Gly Pro His Pro Cys Gly Pro Cys Ser Glu Arg Arg Lys
180 185 190

His Leu Phe Val Gln Asp Pro Gln Thr Cys Lys Cys Ser Cys Lys Asn
195 200 205

Thr Asp Ser Arg Cys Lys Ala Arg Gln Leu Glu Leu Asn Glu Arg Thr
210 215 220

Cys Arg Cys Asp Lys Pro Arg Arg
225 230

<210> 2
<211> 1356
<212> PRT
<213> Homo sapiens

<400> 2

Met Gln Ser Lys Val Leu Leu Ala Val Ala Leu Trp Leu Cys Val Glu
1 5 10 15

Thr Arg Ala Ala Ser Val Gly Leu Pro Ser Val Ser Leu Asp Leu Pro
20 25 30

Arg Leu Ser Ile Gln Lys Asp Ile Leu Thr Ile Lys Ala Asn Thr Thr
35 40 45

Leu Gln Ile Thr Cys Arg Gly Gln Arg Asp Leu Asp Trp Leu Trp Pro
50 55 60

Asn Asn Gln Ser Gly Ser Glu Gln Arg Val Glu Val Thr Glu Cys Ser
65 70 75 80

Asp Gly Leu Phe Cys Lys Thr Leu Thr Ile Pro Lys Val Ile Gly Asn
85 90 95

Asp Thr Gly Ala Tyr Lys Cys Phe Tyr Arg Glu Thr Asp Leu Ala Ser
100 105 110

Val Ile Tyr Val Tyr Val Gln Asp Tyr Arg Ser Pro Phe Ile Ala Ser
115 120 125

```
Val Ser Asp Gln His Gly Val Val Tyr Ile Thr Glu Asn Lys Asn Lys
    130                 135                 140

Thr Val Val Ile Pro Cys Leu Gly Ser Ile Ser Asn Leu Asn Val Ser
145                 150                 155                 160

Leu Cys Ala Arg Tyr Pro Glu Lys Arg Phe Val Pro Asp Gly Asn Arg
                165                 170                 175

Ile Ser Trp Asp Ser Lys Lys Gly Phe Thr Ile Pro Ser Tyr Met Ile
            180                 185                 190

Ser Tyr Ala Gly Met Val Phe Cys Glu Ala Lys Ile Asn Asp Glu Ser
        195                 200                 205

Tyr Gln Ser Ile Met Tyr Ile Val Val Val Val Gly Tyr Arg Ile Tyr
    210                 215                 220

Asp Val Val Leu Ser Pro Ser His Gly Ile Glu Leu Ser Val Gly Glu
225                 230                 235                 240

Lys Leu Val Leu Asn Cys Thr Ala Arg Thr Glu Leu Asn Val Gly Ile
                245                 250                 255

Asp Phe Asn Trp Glu Tyr Pro Ser Ser Lys His Gln His Lys Lys Leu
            260                 265                 270

Val Asn Arg Asp Leu Lys Thr Gln Ser Gly Ser Glu Met Lys Lys Phe
        275                 280                 285

Leu Ser Thr Leu Thr Ile Asp Gly Val Thr Arg Ser Asp Gln Gly Leu
    290                 295                 300

Tyr Thr Cys Ala Ala Ser Ser Gly Leu Met Thr Lys Lys Asn Ser Thr
305                 310                 315                 320

Phe Val Arg Val His Glu Lys Pro Phe Val Ala Phe Gly Ser Gly Met
                325                 330                 335

Glu Ser Leu Val Glu Ala Thr Val Gly Glu Arg Val Arg Ile Pro Ala
            340                 345                 350

Lys Tyr Leu Gly Tyr Pro Pro Pro Glu Ile Lys Trp Tyr Lys Asn Gly
        355                 360                 365

Ile Pro Leu Glu Ser Asn His Thr Ile Lys Ala Gly His Val Leu Thr
    370                 375                 380
```

```
Ile Met Glu Val Ser Glu Arg Asp Thr Gly Asn Tyr Thr Val Ile Leu
385                 390                 395                 400

Thr Asn Pro Ile Ser Lys Glu Lys Gln Ser His Val Val Ser Leu Val
                405                 410                 415

Val Tyr Val Pro Pro Gln Ile Gly Glu Lys Ser Leu Ile Ser Pro Val
            420                 425                 430

Asp Ser Tyr Gln Tyr Gly Thr Thr Gln Thr Leu Thr Cys Thr Val Tyr
        435                 440                 445

Ala Ile Pro Pro Pro His His Ile His Trp Tyr Trp Gln Leu Glu Glu
    450                 455                 460

Glu Cys Ala Asn Glu Pro Ser Gln Ala Val Ser Val Thr Asn Pro Tyr
465                 470                 475                 480

Pro Cys Glu Glu Trp Arg Ser Val Glu Asp Phe Gln Gly Gly Asn Lys
                485                 490                 495

Ile Glu Val Asn Lys Asn Gln Phe Ala Leu Ile Glu Gly Lys Asn Lys
            500                 505                 510

Thr Val Ser Thr Leu Val Ile Gln Ala Ala Asn Val Ser Ala Leu Tyr
        515                 520                 525

Lys Cys Glu Ala Val Asn Lys Val Gly Arg Gly Glu Arg Val Ile Ser
    530                 535                 540

Phe His Val Thr Arg Gly Pro Glu Ile Thr Leu Gln Pro Asp Met Gln
545                 550                 555                 560

Pro Thr Glu Gln Glu Ser Val Ser Leu Trp Cys Thr Ala Asp Arg Ser
                565                 570                 575

Thr Phe Glu Asn Leu Thr Trp Tyr Lys Leu Gly Pro Gln Pro Leu Pro
            580                 585                 590

Ile His Val Gly Glu Leu Pro Thr Pro Val Cys Lys Asn Leu Asp Thr
        595                 600                 605

Leu Trp Lys Leu Asn Ala Thr Met Phe Ser Asn Ser Thr Asn Asp Ile
    610                 615                 620

Leu Ile Met Glu Leu Lys Asn Ala Ser Leu Gln Asp Gln Gly Asp Tyr
625                 630                 635                 640
```

Val Cys Leu Ala Gln Asp Arg Lys Thr Lys Lys Arg His Cys Val Val
645 650 655

Arg Gln Leu Thr Val Leu Glu Arg Val Ala Pro Thr Ile Thr Gly Asn
660 665 670

Leu Glu Asn Gln Thr Thr Ser Ile Gly Glu Ser Ile Glu Val Ser Cys
675 680 685

Thr Ala Ser Gly Asn Pro Pro Pro Gln Ile Met Trp Phe Lys Asp Asn
690 695 700

Glu Thr Leu Val Glu Asp Ser Gly Ile Val Leu Lys Asp Gly Asn Arg
705 710 715 720

Asn Leu Thr Ile Arg Arg Val Arg Lys Glu Asp Glu Gly Leu Tyr Thr
725 730 735

Cys Gln Ala Cys Ser Val Leu Gly Cys Ala Lys Val Glu Ala Phe Phe
740 745 750

Ile Ile Glu Gly Ala Gln Glu Lys Thr Asn Leu Glu Ile Ile Ile Leu
755 760 765

Val Gly Thr Ala Val Ile Ala Met Phe Phe Trp Leu Leu Leu Val Ile
770 775 780

Ile Leu Arg Thr Val Lys Arg Ala Asn Gly Gly Glu Leu Lys Thr Gly
785 790 795 800

Tyr Leu Ser Ile Val Met Asp Pro Asp Glu Leu Pro Leu Asp Glu His
805 810 815

Cys Glu Arg Leu Pro Tyr Asp Ala Ser Lys Trp Glu Phe Pro Arg Asp
820 825 830

Arg Leu Lys Leu Gly Lys Pro Leu Gly Arg Gly Ala Phe Gly Gln Val
835 840 845

Ile Glu Ala Asp Ala Phe Gly Ile Asp Lys Thr Ala Thr Cys Arg Thr
850 855 860

Val Ala Val Lys Met Leu Lys Glu Gly Ala Thr His Ser Glu His Arg
865 870 875 880

Ala Leu Met Ser Glu Leu Lys Ile Leu Ile His Ile Gly His His Leu
885 890 895

Asn Val Val Asn Leu Leu Gly Ala Cys Thr Lys Pro Gly Gly Pro Leu
900 905 910

Met Val Ile Val Glu Phe Cys Lys Phe Gly Asn Leu Ser Thr Tyr Leu
915 920 925

Arg Ser Lys Arg Asn Glu Phe Val Pro Tyr Lys Thr Lys Gly Ala Arg
930 935 940

Phe Arg Gln Gly Lys Asp Tyr Val Gly Ala Ile Pro Val Asp Leu Lys
945 950 955 960

Arg Arg Leu Asp Ser Ile Thr Ser Ser Gln Ser Ser Ala Ser Ser Gly
965 970 975

Phe Val Glu Glu Lys Ser Leu Ser Asp Val Glu Glu Glu Glu Ala Pro
980 985 990

Glu Asp Leu Tyr Lys Asp Phe Leu Thr Leu Glu His Leu Ile Cys Tyr
995 1000 1005

Ser Phe Gln Val Ala Lys Gly Met Glu Phe Leu Ala Ser Arg Lys
1010 1015 1020

Cys Ile His Arg Asp Leu Ala Ala Arg Asn Ile Leu Leu Ser Glu
1025 1030 1035

Lys Asn Val Val Lys Ile Cys Asp Phe Gly Leu Ala Arg Asp Ile
1040 1045 1050

Tyr Lys Asp Pro Asp Tyr Val Arg Lys Gly Asp Ala Arg Leu Pro
1055 1060 1065

Leu Lys Trp Met Ala Pro Glu Thr Ile Phe Asp Arg Val Tyr Thr
1070 1075 1080

Ile Gln Ser Asp Val Trp Ser Phe Gly Val Leu Leu Trp Glu Ile
1085 1090 1095

Phe Ser Leu Gly Ala Ser Pro Tyr Pro Gly Val Lys Ile Asp Glu
1100 1105 1110

Glu Phe Cys Arg Arg Leu Lys Glu Gly Thr Arg Met Arg Ala Pro
1115 1120 1125

Asp Tyr Thr Thr Pro Glu Met Tyr Gln Thr Met Leu Asp Cys Trp
1130 1135 1140

His Gly Glu Pro Ser Gln Arg Pro Thr Phe Ser Glu Leu Val Glu
1145 1150 1155

His Leu Gly Asn Leu Leu Gln Ala Asn Ala Gln Gln Asp Gly Lys
1160 1165 1170

Asp Tyr Ile Val Leu Pro Ile Ser Glu Thr Leu Ser Met Glu Glu
1175 1180 1185

Asp Ser Gly Leu Ser Leu Pro Thr Ser Pro Val Ser Cys Met Glu
1190 1195 1200

Glu Glu Glu Val Cys Asp Pro Lys Phe His Tyr Asp Asn Thr Ala
1205 1210 1215

Gly Ile Ser Gln Tyr Leu Gln Asn Ser Lys Arg Lys Ser Arg Pro
1220 1225 1230

Val Ser Val Lys Thr Phe Glu Asp Ile Pro Leu Glu Glu Pro Glu
1235 1240 1245

Val Lys Val Ile Pro Asp Asp Asn Gln Thr Asp Ser Gly Met Val
1250 1255 1260

Leu Ala Ser Glu Glu Leu Lys Thr Leu Glu Asp Arg Thr Lys Leu
1265 1270 1275

Ser Pro Ser Phe Gly Gly Met Val Pro Ser Lys Ser Arg Glu Ser
1280 1285 1290

Val Ala Ser Glu Gly Ser Asn Gln Thr Ser Gly Tyr Gln Ser Gly
1295 1300 1305

Tyr His Ser Asp Asp Thr Asp Thr Thr Val Tyr Ser Ser Glu Glu
1310 1315 1320

Ala Glu Leu Leu Lys Leu Ile Glu Ile Gly Val Gln Thr Gly Ser
1325 1330 1335

Thr Ala Gln Ile Leu Gln Pro Asp Ser Gly Thr Thr Leu Ser Ser
1340 1345 1350

Pro Pro Val
1355

<210> 3
<211> 221

<212> PRT
<213> Homo sapiens

<400> 3

```
Met Pro Val Met Arg Leu Phe Pro Cys Phe Leu Gln Leu Leu Ala Gly
1               5                   10                  15

Leu Ala Leu Pro Ala Val Pro Pro Gln Gln Trp Ala Leu Ser Ala Gly
            20                  25                  30

Asn Gly Ser Ser Glu Val Glu Val Val Pro Phe Gln Glu Val Trp Gly
        35                  40                  45

Arg Ser Tyr Cys Arg Ala Leu Glu Arg Leu Val Asp Val Val Ser Glu
    50                  55                  60

Tyr Pro Ser Glu Val Glu His Met Phe Ser Pro Ser Cys Val Ser Leu
65                  70                  75                  80

Leu Arg Cys Thr Gly Cys Cys Gly Asp Glu Asn Leu His Cys Val Pro
                85                  90                  95

Val Glu Thr Ala Asn Val Thr Met Gln Leu Leu Lys Ile Arg Ser Gly
            100                 105                 110

Asp Arg Pro Ser Tyr Val Glu Leu Thr Phe Ser Gln His Val Arg Cys
        115                 120                 125

Glu Cys Arg His Ser Pro Gly Arg Gln Ser Pro Asp Met Pro Gly Asp
    130                 135                 140

Phe Arg Ala Asp Ala Pro Ser Phe Leu Pro Pro Arg Arg Ser Leu Pro
145                 150                 155                 160

Met Leu Phe Arg Met Glu Trp Gly Cys Ala Leu Thr Gly Ser Gln Ser
                165                 170                 175

Ala Val Trp Pro Ser Ser Pro Val Pro Glu Glu Ile Pro Arg Met His
            180                 185                 190

Pro Gly Arg Asn Gly Lys Lys Gln Gln Arg Lys Pro Leu Arg Glu Lys
        195                 200                 205

Met Lys Pro Glu Arg Cys Gly Asp Ala Val Pro Arg Arg
    210                 215                 220
```

## Description

### Related Applications

The present application is related to EP 10170004.5 and EP 10170008.6, both filed July 19, 2010 and U.S. Provisional Application Nos. 61/414,853; 61/414,859; and 61/414,861 all filed November 17, 2010, the disclosures of each of which are hereby incorporated by reference in their entirety for all purposes.

### Field of the Invention

The present invention is directed to methods for identifying which patients will most benefit from treatment with anti-cancer agents and monitoring patients for their sensitivity and responsiveness to treatment with anti-cancer agents.

### Background of the Invention

Cancer is one of the most deadly threats to human health. In the U.S. alone, cancer affects nearly 1.3 million new patients each year, and is the second leading cause of death after cardiovascular disease, accounting for approximately 1 in 4 deaths. Solid tumors are responsible for most of those deaths. Although there have been significant advances in the medical treatment of certain cancers, the overall 5-year survival rate for all cancers has improved only by about 10% in the past 20 years. Cancers, or malignant tumors, metastasize and grow rapidly in an uncontrolled manner, making timely detection and treatment extremely difficult.

Depending on the cancer type, patients typically have several treatment options available to them including chemotherapy, radiation and antibody-based drugs. Diagnostic methods useful for predicting clinical outcome from the different treatment regimens would greatly benefit clinical management of these patients.

Thus, there is a need for more effective means for determining which patients will respond to which treatment and for incorporating such determinations into more effective treatment regimens for patients with anti-cancer therapies, whether used as single agents or combined with other agents.

### Summary of the Invention

The present invention provides methods for identifying patients who will respond to treatment with anti-cancer agents, e.g., VEGF A antagonists such as, for example bevacizumab.

One embodiment of the invention provides methods of identifying a patient who may benefit from treatment with an anti-cancer therapy comprising a VEGF antagonist, the methods comprising: determining an expression level of $VEGF_{110}$ in a sample obtained from the patient, wherein a level of $VEGF_{110}$ in the sample obtained from the patient at or above a reference level (e.g., as compared to a reference sample) indicates that the patient may benefit from treatment with the anti-cancer therapy. In some embodiments, the cancer is selected from the group consisting of: colorectal cancer, glioblastoma, renal cancer, ovarian cancer, breast cancer (including, e.g., locally advanced, recurrent or metastatic HER-2 negative breast cancer), pancreatic cancer (including, e.g., metastatic pancreatic cancer), gastric cancer and lung cancer. In some embodiments, the sample obtained from the patient is a member selected from the group consisting of: whole blood, plasma, serum, and combinations thereof. In some embodiments, the $VEGF_{110}$ level is a protein level. In some embodiments, the $VEGF_{110}$ protein level is determined by measuring $VEGF_{110}$ plasma protein level. In some embodiments, a plasma level of $VEGF_{110}$ that is at or above a reference level, indicates that the patient may benefit from the anti-cancer therapy, is more likely to be responsive to the anti-cancer therapy, or has increased likelihood of benefit from the anti-cancer therapy. In some embodiments, the methods further comprise administering an effective amount of an anti-cancer therapy comprising a VEGF-A antagonist to said patient. In some embodiments, the methods further comprise administering an effective amount of a second anti-cancer therapy selected from the group consisting of: a cytotoxic agent, a chemotherapeutic agent, a growth inhibitory agent, and anti-angiogenic agents, and combinations thereof. In some embodiments, the second anti-cancer therapy and the VEGF-A antagonist are administered concurrently. In some embodiments, the second anti-cancer therapy and the VEGF-A antagonist are administered sequentially. In some embodiments, the methods further comprise administering an effective amount of a third anti-cancer therapy selected from the group consisting of: a cytotoxic agent, a chemotherapeutic agent, a growth inhibitory agent, and anti-angiogenic agents, and combinations thereof. In some embodiments, the third anti-cancer therapy, the second anti-cancer therapy and the VEGF-A antagonist are administered concurrently. In some embodiments, the third anti-cancer therapy, the second anti-cancer therapy and the VEGF-A antagonist are administered sequentially. In some embodiments, the VEGF-A antagonist is an antibody. In some embodiments, the antibody is bevacizumab. In some embodiments, the cancer is breast cancer (including, e.g., locally advanced, recurrent or metastatic HER-2 negative breast cancer) and the second anti-cancer therapy is docetaxel. In some embodiments, the cancer is pancreatic cancer (including, e.g., metastatic pancreatic cancer), the second anti-cancer therapy is gemcitabine, and the third anti-cancer therapy is erlotinib. In some embodiments, the cancer is gastric cancer, the second anti-cancer therapy is capecitabine, and the third anti-cancer therapy is cisplatin. In some embodiment, the cancer is lung cancer, the second anti-cancer therapy is gemcitabine, and the third anti-cancer therapy is cisplatin.

A further embodiment of the invention provides methods of predicting responsiveness of a patient suffering from cancer to treatment with an anti-cancer therapy comprising a VEGF-A antagonist, the methods comprising: determining an expression level of $VEG_{110}$ in a sample obtained from the patient, wherein a level of $VEGF_{110}$ in the sample obtained from the patient at or above a reference level (e.g., as compared to a reference sample) indicates that the patient is more likely to be responsive to treatment with the anti-cancer therapy. In some embodiments, the cancer is selected from the group consisting of: colorectal cancer, glioblastoma, renal cancer, ovarian cancer, breast cancer (including, e.g., locally advanced, recurrent or metastatic HER-2 negative breast cancer), pancreatic cancer (including, e.g., metastatic pancreatic cancer), gastric cancer, and lung cancer. In some embodiments, the sample obtained from the patient is a member selected from the group consisting of: whole blood, plasma, serum, and combinations thereof. In some embodiments, the $VEGF_{110}$ protein level is determined by measuring $VEGF_{110}$ plasma protein level. In some embodiments, a plasma level of $VEGF_{110}$ that is at or above a reference level, indicates that the patient may benefit from the anti-cancer therapy, is more likely to be responsive to the anti-cancer therapy, or has increased likelihood of benefit from the anti-cancer therapy. In some embodiments, the methods further comprise administering an effective amount of an anti-cancer therapy comprising a VEGF-A antagonist to said patient. In some embodiments, the methods further comprise administering an effective amount of a second anti-cancer therapy selected from the group consisting of: a cytotoxic agent, a chemotherapeutic agent, a growth inhibitory agent, and anti-angiogenic agents, and combinations thereof. In some embodiments, the second anti-cancer therapy and the VEGF-A antagonist are administered concurrently. In some embodiments, the second anti-cancer therapy and the VEGF-A antagonist are administered sequentially. In some embodiments, the methods further comprise administering an effective amount of a third anti-cancer therapy selected from the group consisting of: a cytotoxic agent, a chemotherapeutic agent, a growth inhibitory agent, and anti-angiogenic agents, and combinations thereof. In some embodiments, the third anti-cancer therapy, the second anti-cancer therapy and the VEGF-A antagonist are administered concurrently. In some embodiments, the third anti-cancer therapy, the second anti-cancer therapy and the VEGF-A antagonist are administered sequentially. In some embodiments, the VEGF-A antagonist is an antibody. In some embodiments, the antibody is bevacizumab. In some embodiments, the antibody is bevacizumab. In some embodiments, the cancer is breast cancer (including, e.g., locally advanced, recurrent or metastatic HER-2 negative breast cancer) and the second anti-cancer therapy is docetaxel. In some embodiments, the cancer is pancreatic cancer (including, e.g., metastatic pancreatic cancer), the second anti-cancer therapy is gemcitabine, and the third anti-cancer therapy is erlotinib. In some embodiments, the cancer is gastric cancer, the second anti-cancer therapy is capecitabine, and the third anti-cancer therapy is cisplatin. In some embodiment, the cancer is lung cancer, the second anti-cancer therapy is gemcitabine, and the third anti-cancer therapy is cisplatin.

Yet another embodiment of the invention provides methods for determining the likelihood that a patient with cancer will exhibit benefit from anti-cancer therapy comprising a VEGF-A antagonist, the methods comprising: determining expression an level of $VEGF_{110}$ in a sample obtained from the patient, wherein a level of $VEGF_{110}$ in the sample obtained from the patient at or above a reference level (e.g., as compared to a reference sample) indicates that the patient has increased likelihood of benefit from the anti-cancer therapy. In some embodiments, the cancer is selected from the group consisting of: colorectal cancer, glioblastoma, renal cancer, ovarian cancer, breast cancer (including, e.g., locally advanced, recurrent or metastatic HER-2 negative breast cancer), pancreatic cancer (including, e.g., metastatic pancreatic cancer), gastric cancer, and lung cancer. In some embodiments, the sample obtained from the patient is a member selected from the group consisting of: whole blood, plasma, serum, and combinations thereof. In some embodiments, the $VEGF_{110}$ protein level is determined by measuring $VEGF_{110}$ plasma protein level. In some embodiments, a plasma level of $VEGF_{110}$ that is at or above a reference level, indicates that the patient may benefit from the anti-cancer therapy, is more likely to be responsive to the anti-cancer therapy, or has increased likelihood of benefit from the anti-cancer therapy. In some embodiments, the methods further comprise administering an effective amount of an anti-cancer therapy comprising a VEGF-A antagonist to said patient. In some embodiments, the methods further comprise administering an effective amount of a second anti-cancer therapy selected from the group consisting of: a cytotoxic agent, a chemotherapeutic agent, a growth inhibitory agent, and anti-angiogenic agents, and combinations thereof. In some embodiments, the second anti-cancer therapy and the VEGF-A antagonist are administered concurrently. In some embodiments, the second anti-cancer therapy and the VEGF-A antagonist are administered sequentially. In some embodiments, the methods further comprise administering an effective amount of a third anti-cancer therapy selected from the group consisting of: a cytotoxic agent, a chemotherapeutic agent, a growth inhibitory agent, and anti-angiogenic agents, and combinations thereof. In some embodiments, the third anti-cancer therapy, the second anti-cancer therapy and the VEGF-A antagonist are administered concurrently. In some embodiments, the third anti-cancer therapy, the second anti-cancer therapy and the VEGF-A antagonist are administered sequentially. In some embodiments, the VEGF-A antagonist is an antibody. In some embodiments, the antibody is bevacizumab. In some embodiments, the antibody is bevacizumab. In some embodiments, the cancer is breast cancer (including, e.g., locally advanced, recurrent or metastatic HER-2 negative breast cancer) and the second anti-cancer therapy is docetaxel. In some embodiments, the cancer is pancreatic cancer (including, e.g., metastatic pancreatic cancer), the second anti-cancer therapy is gemcitabine, and the third anti-cancer therapy is erlotinib. In some embodiments, the cancer is gastric cancer, the second anti-cancer therapy is capecitabine, and the third anti-cancer therapy is cisplatin. In some embodiment, the cancer is lung cancer, the second anti-cancer therapy is gemcitabine, and the third anti-cancer therapy is cisplatin.

Even another embodiment of the invention provides methods for optimizing the therapeutic efficacy of an anti-cancer therapy comprising a VEGF-A antagonist, the methods comprising: determining an expression level of $VEGF_{110}$ in a sample obtained from the patient, wherein a level of $VEGF_{110}$ in the sample obtained from the patient at or a above a reference level (e.g., as compared to a reference sample) indicates that the patient has increased likelihood of benefit from the anti-cancer therapy. In some embodiments, the cancer is selected from the group consisting of: colorectal cancer, glioblastoma, renal cancer, ovarian cancer, breast cancer (including, e.g., locally advanced, recurrent or metastatic HER-2 negative breast cancer), pancreatic cancer (including, e.g., metastatic pancreatic cancer), gastric cancer, and lung cancer. In some embodiments, the sample obtained from the patient is a member selected from the group consisting of: whole blood, plasma, serum, and combinations thereof. In some embodiments, the $VEGF_{110}$ protein level is determined by measuring $VEGF_{110}$ plasma protein level. In some embodiments, a plasma level of $VEGF_{110}$ that is at or above a reference level, indicates that the patient may benefit from the anti-cancer therapy, is more likely to be responsive to the anti-cancer therapy, or has increased likelihood of benefit from the anti-cancer therapy. In some embodiments, the methods further comprise administering an effective amount of an anti-cancer therapy comprising a VEGF-A antagonist to said patient. In some embodiments, the methods further comprise administering an effective amount of a second anti-cancer therapy selected from the group consisting of: a cytotoxic agent, a chemotherapeutic agent, a growth inhibitory agent, and anti-angiogenic agents, and combinations thereof. In some embodiments, the second anti-cancer therapy and the VEGF-A antagonist are administered concurrently. In some embodiments, the second anti-cancer therapy and the VEGF-A antagonist are administered sequentially. In some embodiments, the methods further comprise administering an effective amount of a third anti-cancer therapy selected from the group consisting of: a cytotoxic agent, a chemotherapeutic agent, a growth inhibitory agent, and anti-angiogenic agents, and combinations thereof. In some embodiments, the third anti-cancer therapy, the second anti-cancer therapy and the VEGF-A antagonist are administered concurrently. In some embodiments, the third anti-cancer therapy, the second anti-cancer therapy and the VEGF-A antagonist are administered sequentially. In some embodiments, the VEGF-A antagonist is an antibody. In some embodiments, the antibody is bevacizumab. In some embodiments, the antibody is bevacizumab. In some embodiments, the cancer is breast cancer (including, e.g., locally advanced, recurrent or metastatic HER-2 negative breast cancer) and the second anti-cancer therapy is docetaxel. In some embodiments, the cancer is pancreatic cancer (including, e.g., metastatic pancreatic cancer), the second anti-cancer therapy is gemcitabine, and the third anti-cancer therapy is erlotinib. In some embodiments, the cancer is gastric cancer, the second anti-cancer therapy is capecitabine, and the third anti-cancer therapy is cisplatin. In some embodiment, the cancer is lung cancer, the second anti-cancer therapy is gemcitabine, and the third anti-cancer therapy is cisplatin.

A further embodiment of the invention provides methods for treating cancer in a patient, the methods comprising determining that a sample obtained from the patient has a level at or above a reference level (e.g., as compared to a reference sample) of $VEGF_{110}$, and administering an effective amount of an anti-cancer therapy comprising a VEGF-A antagonist to said patient, whereby the cancer is treated. In some embodiments, the cancer is selected from the group consisting of: colorectal cancer, glioblastoma, renal cancer, ovarian cancer, breast cancer (including, e.g., locally advanced, recurrent or metastatic HER-2 negative breast cancer), pancreatic cancer (including, e.g., metastatic pancreatic cancer), gastric cancer, and lung cancer. In some embodiments, the sample obtained from the patient is a member selected from the group consisting of: whole blood, plasma, serum, and combinations thereof. In some embodiments, the $VEGF_{110}$ protein level is determined by measuring $VEGF_{110}$ plasma protein level. In some embodiments, a plasma level of $VEGF_{110}$ that is at or above a reference level, indicates that the patient may benefit from the anti-cancer therapy, is more likely to be responsive to the anti-cancer therapy, or has increased likelihood of benefit from the anti-cancer therapy. In some embodiments, the methods further comprise administering an effective amount of a second anti-cancer therapy selected from the group consisting of: a cytotoxic agent, a chemotherapeutic agent, a growth inhibitory agent, and anti-angiogenic agents, and combinations thereof. In some embodiments, the second anti-cancer therapy and the VEGF-A antagonist are administered concurrently. In some embodiments, the second anti-cancer therapy and the VEGF-A antagonist are administered sequentially. In some embodiments, the methods further comprise administering an effective amount of a third anti-cancer therapy selected from the group consisting of: a cytotoxic agent, a chemotherapeutic agent, a growth inhibitory agent, and anti-angiogenic agents, and combinations thereof. In some embodiments, the third anti-cancer therapy, the second anti-cancer therapy and the VEGF-A antagonist are administered concurrently. In some embodiments, the third anti-cancer therapy, the second anti-cancer therapy and the VEGF-A antagonist are administered sequentially. In some embodiments, the VEGF-A antagonist is an antibody. In some embodiments, the antibody is bevacizumab. In some embodiments, the antibody is bevacizumab. In some embodiments, the cancer is breast cancer (including, e.g., locally advanced, recurrent or metastatic HER-2 negative breast cancer) and the second anti-cancer therapy is docetaxel. In some embodiments, the cancer is pancreatic cancer (including, e.g., metastatic pancreatic cancer), the second anti-cancer therapy is gemcitabine, and the third anti-cancer therapy is erlotinib. In some embodiments, the cancer is gastric cancer, the second anti-cancer therapy is capecitabine, and the third anti-cancer therapy is cisplatin. In some embodiment, the cancer is lung cancer, the second anti-cancer therapy is gemcitabine, and the third anti-cancer therapy is cisplatin.

Another embodiment of the invention provides kits for determining whether a patient may benefit from treatment with an anti-cancer therapy comprising a VEGF-A antagonist, the kits comprising a set of compounds to determine the level of at least one biomarker to predict responsiveness of a patient to treatment with an anti-cancer therapy comprising a VEGF-A antagonist, wherein a level of $VEGF_{110}$ at or above a reference level (e.g., the level of $VEGF_{110}$ in a reference sample) indicates that the patient may benefit from treatment with an anti-cancer therapy comprising a VEGF-A antagonist. In some embodiments, the compounds are proteins. In some embodiments, the proteins are antibodies.

A further embodiment of the invention provides a set of compounds for detecting levels of $VEGF_{110}$, the set comprising at least one compound capable of specifically binding to $VEGF_{110}$. Preferably the set of compounds is used to predict responsiveness of a patient to treatment with an anti-cancer therapy comprising a VEGF-A antagonist. In some embodiments, the compounds are proteins. In some embodiments, the proteins are antibodies.

These and other embodiments are further described by the detailed description that follows.

## Brief Description of the Drawings

**Figure 1:** Kaplan Meier Curve for Progression Free Survival for the overall biomarker population for bevacizumab (low or high dose) plus docetaxel therapy versus placebo plus docetaxel therapy for patients being treated for locally advanced, recurrent or metastatic HER-2 negative breast cancer. Short-dash line represents placebo plus docetaxel. Solid line represents low dose bevacizumab (7.5 mg/kg every 3 weeks) plus docetaxel. Long-dash line represents high dose bevacizumab (15 mg/kg every 3 weeks) plus docetaxel.

**Figure 2:** Forest Plot of hazard ratio of progression-free survival before start of subsequent anti-neoplastic therapy by Biomarker (Placebo and Low Dose Bevacizumab), a dichotomized analysis, for bevacizumab (low dose) plus docetaxel therapy versus placebo plus docetaxel therapy for patients being treated for locally advanced, recurrent or metastatic HER-2 negative breast cancer.

**Figure 3:** Forest Plot of hazard ratio of progression-free survival before start of subsequent anti-neoplastic therapy by Biomarker (Placebo and High Dose Bevacizumab), a dichotomized analysis, for bevacizumab (high dose) plus docetaxel therapy versus placebo plus docetaxel therapy for patients being treated for locally advanced, recurrent or metastatic HER-2 negative breast cancer.

**Figure 4:** Kaplan Meier Curve of progression-free survival before start of subsequent anti-neoplastic therapy for low expression level (<125 pg/ml) VEGFA, (Figure 4A), and high expression level (≥125 pg/ml) VEGFA, (Figure 4B), for bevacizumab (low or high dose) plus docetaxel therapy versus placebo plus docetaxel therapy for patients being treated for locally advanced, recurrent or metastatic HER-2 negative breast cancer. Short-dash line represents placebo plus docetaxel. Solid line represents low dose bevacizumab (7.5 mg/kg every 3 weeks) plus docetaxel. Long-dash line represents high dose bevacizumab (15 mg/kg every 3 weeks) plus docetaxel.

**Figure 5:** Kaplan Meier Curve of progression free survival before start of subsequent anti-neoplastic therapy for low expression level (<11 ng/ml) VEGFR2, (Figure 5A), and high expression level (≥11 ng/ml) VEGFR2, (Figure 5B), for bevacizumab (low or high dose) plus docetaxel therapy versus placebo plus docetaxel therapy for patients being treated for locally advanced, recurrent or metastatic HER-2 negative breast cancer. Short-dash line represents placebo plus docetaxel. Solid line represents low dose bevacizumab (7.5 mg/kg every 3 weeks) plus docetaxel. Long-dash line represents high dose bevacizumab (15 mg/kg every 3 weeks) plus docetaxel.

**Figure 6:** Kaplan Meier Curve of progression free survival before start of subsequent anti-neoplastic therapy for combined low expression level (Formula 1 < -0.132) and combined high expression level (Formula 1 ≥ -0.132) of VEGFA and VEGFR2 for bevacizumab (low or high dose) plus docetaxel therapy versus placebo plus docetaxel therapy for patients being treated for locally advanced, recurrent or metastatic HER-2 negative breast cancer. Solid line represents placebo plus docetaxel. Long-dash represents low dose bevacizumab (7.5 mg/kg every 3 weeks) plus docetaxel. Short-dash line represents high dose bevacizumab (15 mg/kg every 3 weeks) plus docetaxel.

**Figure 7**: Kaplan Meier Curve of progression free survival before start of subsequent anti-neoplastic therapy for combined low expression level (Formula 2 < -0.006) and combined high expression level (Formula 2 ≥ -0.006) of VEGFA and PLGF for bevacizumab (low or high dose) plus docetaxel therapy versus placebo plus docetaxel therapy for patients being treated for locally advanced, recurrent or metastatic HER-2 negative breast cancer. Solid line represents placebo plus docetaxel. Long-dash line represents low dose bevacizumab (7.5 mg/kg every 3 weeks) plus docetaxel. Short-dash line represents high dose bevacizumab (15 mg/kg every 3 weeks) plus docetaxel.

**Figure 8:** SEQ ID NO:1, Exemplary amino acid sequence of VEGFA.

**Figure 9:** SEQ ID NO:2, Exemplary amino acid sequence of VEGFR2.

**Figure 10:** SEQ ID NO:3, Exemplary amino acid sequence of PLGF.

**Figure 11:** Measurements of increasing concentrations of $VEGF_{111}$, $VEGF_{121}$, $VEGF_{165}$ and $VEGF_{189}$ as measured on an IMPACT chip.

**Figure 12:** Measurements of increasing concentrations of $VEGF_{110}$, $VEGF_{121}$, and $VEGF_{165}$ as measured using the Elecsys® Assay on the automated Elecsys® analyzer.

**Figure 13:** Kaplan Meier Curves for Overall Survival (Figure 13A) and for Progression Free Survival (Figure 13B) for the marker VEGFA, for both high (>111 pg/ml) and low (≤111 pg/ml) expression levels, for bevacizumab plus capecitabine/cisplatin therapy versus control placebo plus capecitabine/cisplatin therapy for patients being treated for inoperable locally advanced/metastatic gastric/gastro-oesophageal adenocarcinoma.

**Figure 14:** Kaplan Meier Curves for association with treatment effect on Overall Survival (Figure 14A) and for Progression Free Survival (Figure 14B) for the marker pVEGFA, for both high (>111 pg/ml) and low (≤111 pg/ml) expression levels, for bevacizumab plus capecitabine/cisplatin therapy versus control placebo plus capecitabine/cisplatin therapy for patients from the Asian-Pacific regions being treated for inoperable locally advanced/metastatic gastric/gastro-oesophageal adenocarcinoma.

**Figure 15:** Kaplan Meier Curves for association with treatment effect on Overall Survival (Figure 15A) and for Progression Free Survival (Figure 15B) for the marker VEGFA, for both high (>111 pg/ml) and low (≤111 pg/ml) expression levels, for bevacizumab plus capecitabine/cisplatin therapy versus control placebo plus capecitabine/cisplatin therapy for patients from non-Asian-Pacific regions being treated for inoperable locally advanced/metastatic gastric/gastro-oesophageal adenocarcinoma.

**Figure 16:** Kaplan Meier Curves for Overall Survival (Figure 16A) and for Progression Free Survival (Figure 16B) for bevacizumab plus gemcitabine-erlotinib therapy versus control placebo plus gemcitabine-erlotinib therapy for patients being treated for metastatic pancreatic cancer. In the figures, the solid line represents bevacizumab/gemcitabine-erlotinib treatment and the dashed line represents placebo/gemcitabine-erlotinib treatment.

**Figure 17:** Kaplan Meier Curves for association with treatment effect on Overall Survival for the marker VEGFA (Figure 17A) and for association with treatment effect on Progression free survival for the marker VEGFA (Figure 17B), for both high (≥152.9 pg/ml) and low (< 152.9 pg/ml) expression levels, for bevacizumab plus gemcitabine-erlotinib therapy versus control placebo plus gemcitabine-erlotinib therapy for patients being treated for metastatic pancreatic cancer. In the figures, the solid line represents bevacizumab/gemcitabine-erlotinib treatment and the dashed line represents placebo/gemcitabine-erlotinib treatment.

**Figure 18:** Kaplan Meier Curves for association with treatment effect on Overall Survival for the markers VEGFA and VEGFR2 (Figure 18A), as a combined expression level for both high (Formula 1 ≥ -0.1) and low (Formula 1 < -0.1) expression levels, and VEGFA and PLGF (Figure 18B), as a combined expression level for both high (Formula 2 ≥ -0.042) and low (Formula 2 < -0.042) expression levels, for bevacizumab plus gemcitabine-erlotinib therapy versus control placebo plus gemcitabine-erlotinib therapy for patients being treated for metastatic pancreatic cancer. In the figures, the solid line represents bevacizumab/gemcitabine-erlotinib treatment and the dashed line represents placebo/gemcitabine-erlotinib treatment.

**Figure 19:** Kaplan Meier Curves for association with treatment effect on Progression Free Survival for the markers VEGFA and VEGFR2 (Figure 19A), as a combined expression level for both high (Formula 1 ≥ -0.1) and low (Formula 1 < -0.1) expression levels, and VEGFA and PLGF (Figure 19B), as a combined expression level for both high (Formula 2 ≥ -0.042) and low (Formula 2 < -0.042) expression levels, for bevacizumab plus gemcitabine-erlotinib therapy versus control placebo plus gemcitabine-erlotinib therapy for patients being treated for metastatic pancreatic cancer. In the figures, the solid line represents bevacizumab/gemcitabine-erlotinib treatment and the dashed line represents placebo/gemcitabine-erlotinib treatment.

**Figure 20:** Kaplan Meier Curve for association with treatment effect on Overall Survival for the markers for the markers VEGFA, VEGFR2 and PLGF (Figure 20A), as a combined expression level for both high (Formula 3 ≥ 0.837) and low (Formula 3 < 0.837) expression levels, and for association with treatment effect on Progression Free Survival for the makers VEGFA, VEGFR2 and PLGF (Figure 20B), as a combined expression level for both high (Formula 3 ≥ 0.837) and low (Formula 3 < 0.837) expression levels, for bevacizumab plus gemcitabine-erlotinib therapy versus control placebo plus gemcitabine-erlotinib therapy for patients being treated for metastatic pancreatic cancer.. In the figure, the solid line represents bevacizumab/gemcitabine-erlotinib treatment and the dashed line represents placebo/gemcitabine-erlotinib treatment.

**Figure 21** Data from EDTA- and Citrate samples from the same patients measured twice with the IMPACT assay. The VEGFA concentration is about 40% higher for EDTA-plasma than for Citrate with a Spearman correlation for the EDTA-Citrate method comparison of about 0.8.

## Detailed Description of the Preferred Embodiments

### I. Introduction

The invention provides methods for identifying patients having an increased likelihood of responding to an anti-cancer therapy comprising a VEGF antagonist.

### II. Definitions

In certain embodiments, the term "increase" or "above" refers to a level above the reference level or to an overall increase of 5%, 10%, 20%, 25%, 30%, 40%, 50%, 60%, 70%, 80%, 85%, 90%, 95%, 100% or greater, in $VEGF_{110}$ level detected by the methods described herein, as compared to the $VEGF_{110}$ level from a reference sample. In certain embodiments, the term increase refers to the increase in $VEGF_{110}$ level wherein, the increase is at least about 1.5-, 1.75-, 2-, 3-, 4-, 5-, 6-, 7-, 8-, 9-, 10-, 15-, 20-, 25-, 30-, 40-, 50-, 60-, 70-, 75-, 80-, 90-, or 100-fold higher as compared to the $VEGF_{110}$ level e.g. predetermined from a reference sample. In one preferred embodiment the term increased level relates to a value at or above a reference level.

In certain embodiments, the term "decrease" or "below" herein refers to a level below the reference level or to an overall reduction of 5%, 10%, 20%, 25%, 30%, 40%, 50%, 60%, 70%, 80%, 85%, 90%, 95%, 96%, 97%, 98%, 99% or greater, in $VEGF_{110}$ level detected by the methods described herein, as compared to the $VEGF_{110}$ level from a reference sample. In certain embodiments, the term decrease refers to the decrease in $VEGF_{110}$ level, wherein the decreased level is at most about 0.9-, 0.8-, 0.7-, 0.6-, 0.5-, 0.4-, 0.3-, 0.2-, 0.1-, 0.05-, or 0.01-fold the $VEGF_{110}$ level from the reference sample or lower.

In certain embodiments, the term "at a reference level" refers to a level that is the same as $VEGF_{110}$ level detected by the methods described herein, from a reference sample.

In certain embodiments, the term "reference level" herein refers to a predetermined value. As the skilled artisan will appreciate the reference level is predetermined and set to meet the requirements in terms of e.g. specificity and/or sensitivity. These requirements can vary, e.g. from regulatory body to regulatory body. It may for example be that assay sensitivity or specificity, respectively, has to be set to certain limits, e.g. 80%, 90% or 95%. These requirements may also be defined in terms of positive or negative predictive values. Nonetheless, based on the teaching given in the present invention it will always be possible to arrive at the reference level meeting those requirements. In one embodiment the reference level is determined in healthy individuals. The reference value in one embodiment has been predetermined in the disease entity to which the patient belongs. In certain embodiments the reference level can e.g. be set to any percentage between 25% and 75% of the overall distribution of the values in a disease entity investigated. In other embodiments the reference level can e.g. be set to the median, tertiles or quartiles as determined from the overall distribution of the values in a disease entity investigated. In one embodiment the reference level is set to the median value as determined from the overall distribution of the values in a disease entity investigated.

In the context of the present invention, "VEGF", VEGFA", or "VEGF-A" refers to vascular endothelial growth factor protein A, exemplified by SEQ ID NO:1, shown in FIGURE 8 (Swiss Prot Accession Number P15692, Gene ID (NCBI): 7422). The term "VEGFA" encompasses the protein having the amino acid sequence of SEQ ID NO:1 as well as homologues and isoforms thereof. The term "VEGF-A" also encompasses the known isoforms, e.g., splice isoforms, of VEGF-A, *e.g.*, $VEGF_{121}$, $VEGF_{145}$, $VEGF_{165}$, $VEGF_{189}$ and $VEGF_{206}$, together with the naturally occurring allelic and processed forms thereof, including the 110-amino acid human vascular endothelial cell growth factor generated by plasmin cleavage of $VEGF_{165}$ as described in Ferrara Mol. Biol. Cell 21:687 (2010) and Leung et al. Science 246:1306 (1989), and Houck et al. Mol. Endocrin. 5:1806 (1991). In the context of the invention, the term "VEGF-A" also encompasses variants and/or homologues thereof, as well as fragments of VEGF-A, provided that the variant proteins (including isoforms), homologous proteins and/or fragments are recognized by one or more VEGF-A specific antibodies, such as antibody clone 3C5 and 26503, which are available from Bender Relia Tech and R&D Systems, respectively and A4.6.1 as described in Kim et al., Growth Factors 7(1): 53-64 (1992). In the context of the invention, the term "isoform" of VEGF, VEGFA or VEGF-A refers to both splice isoforms and forms generated by enzymatic cleavage (e.g., plasmin).

In the context of the present invention, "VEGFR2" refers to vascular endothelial growth factor receptor 2, exemplified by SEQ ID NO:2, shown in FIGURE 9 (Swiss Prot Accession Number P35968, Gene ID (NCBI): 3791). The term "VEGFR2" encompasses the protein having the amino acid sequence of SEQ ID NO:2 as well as homologues and isoforms thereof. In the context of the invention, the term "VEGFR2" also encompasses proteins having at least 85%, at least 90% or at least 95% homology to the amino acid sequence of SEQ ID NO:2, or to the amino acid sequences of the variants and/or homologues thereof, as well as fragments of the sequences, provided that the variant proteins (including isoforms), homologous proteins and/or fragments are recognized by one or more VEGFR2 specific antibodies, such as antibody clone 89115 and 89109, which are available from R&D Systems.

In the context of the present invention, "PLGF" refers to placental growth factor exemplified by SEQ ID NO:3, shown in FIGURE 10 (Swiss Prot Accession Number P49763, Gene ID (NCBI): 5228). The term "PLGF" encompasses the protein having the amino acid sequence of SEQ ID NO:3 as well as homologues and isoforms thereof. In the context of the invention, the term "PLGF" also encompasses proteins having at least 85%, at least 90% or at least 95% homology to the amino acid sequence of SEQ ID NO:3, or to the amino acid sequences of the variants and/or homologues thereof, as well as fragments of the sequences, provided that the variant proteins (including isoforms), homologous proteins and/or fragments are recognized by one or more PLGF specific antibodies, such as antibody clone 2D6D5 and 6A11D2, which are available from Roche Diagnostics GmbH.

The term "VEGF" also refers to VEGFs from non-human species such as mouse, rat or primate. Sometimes the VEGF from a specific species are indicated by terms such as hVEGF for human VEGF, mVEGF for murine VEGF, and etc. The term "VEGF" is also used to refer to truncated forms of the polypeptide comprising amino acids 8 to 109 or 1 to 109 of the 165-amino acid human vascular endothelial cell growth factor. Reference to any such forms of VEGF may be identified in the present application, e.g., by "VEGF (8-109)," "VEGF (1-109)" or "$VEGF_{165}$." The amino acid positions for a "truncated" native VEGF are numbered as indicated in the native VEGF sequence. For example, amino acid position 17 (methionine) in truncated native VEGF is also position 17 (methionine) in native VEGF. The truncated native VEGF has binding affinity for the KDR and Flt-1 receptors comparable to native VEGF. According to a preferred embodiment, the VEGF is a human VEGF.

"VEGF biological activity" includes binding to any VEGF receptor or any VEGF signaling activity such as regulation of both normal and abnormal angiogenesis and vasculogenesis (Ferrara and Davis-Smyth (1997) Endocrine Rev. 18:4-25; Ferrara (1999) J. Mol. Med. 77:527-543); promoting embryonic vasculogenesis and angiogenesis (Carmeliet et al. (1996) Nature 380:435-439; Ferrara et al. (1996) Nature 380:439-442); and modulating the cyclical blood vessel proliferation in the female reproductive tract and for bone growth and cartilage formation (Ferrara et al. (1998) Nature Med. 4:336-340; Gerber et al. (1999) Nature Med. 5:623-628). In addition to being an angiogenic factor in angiogenesis and vasculogenesis, VEGF, as a pleiotropic growth factor, exhibits multiple biological effects in other physiological processes, such as endothelial cell survival, vessel permeability and vasodilation, monocyte chemotaxis and calcium influx (Ferrara and Davis-Smyth (1997), *supra* and Cebe-Suarez et al. Cell. Mol. Life Sci. 63:601-615 (2006)). Moreover, recent studies have reported mitogenic effects of VEGF on a few non-endothelial cell types, such as retinal pigment epithelial cells, pancreatic duct cells, and Schwann cells. Guerrin et al. (1995) J. Cell Physiol. 164:385-394; Oberg-Welsh et al. (1997) Mol. Cell. Endocrinol. 126:125-132; Sondell et al. (1999) J. Neurosci. 19:5731-5740.

A "VEGF antagonist" or "VEGF-specific antagonist" refers to a molecule capable of binding to VEGF, reducing VEGF expression levels, or neutralizing, blocking, inhibiting, abrogating, reducing, or interfering with VEGF biological activities, including, but not limited to, VEGF binding to one or more VEGF receptors and VEGF mediated angiogenesis and endothelial cell survival or proliferation. Included as VEGF-specific antagonists useful in the methods of the invention are polypeptides that specifically bind to VEGF, polypeptides that specifically bind VEGF receptors, anti-VEGF antibodies and antigen-binding fragments thereof, receptor molecules and derivatives which bind specifically to VEGF thereby sequestering its binding to one or more receptors, fusions proteins (e.g., VEGF-Trap (Regeneron)), and $VEGF_{121}$-gelonin (Peregrine). VEGF-specific antagonists also include antagonist variants of VEGF polypeptides, antisense nucleobase oligomers directed to VEGF, small RNA molecules directed to VEGF, RNA aptamers, peptibodies, and ribozymes against VEGF, nucleic acids that hybridize under stringent conditions to nucleic acid sequences that encode VEGF or VEGF receptor (e.g., RNAi), immunoadhesins, anti-VEGF receptor antibodies and VEGF receptor antagonists such as small molecule inhibitors of the VEGFR tyrosine kinases, According to one preferred embodiment, the VEGF antagonist binds to VEGF and inhibits VEGF-induced endothelial cell proliferation in vitro. According to one preferred embodiment, the VEGF antagonist binds to VEGF or a VEGF receptor with greater affinity than a non-VEGF or non-VEGF receptor. According to one preferred embodiment, the VEG antagonist binds to VEGF or a VEGF receptor with a Kd of between 1uM and IpM. According to another preferred embodiment, the VEGF antagonist binds to VEGF or a VEGF receptor between 500nM and IpM. VEGF-specific antagonists also include nonpeptide small molecules that bind to VEGF and are capable of blocking, inhibiting, abrogating, reducing, or interfering with VEGF biological activities. Thus, the term "VEGF activities" specifically includes VEGF mediated biological activities of VEGF. In certain embodiments, the VEGF antagonist reduces or inhibits, by at least 10%, 20%, 30%, 40%, 50%, 60%, 70%, 80%, 90% or more, the expression level or biological activity of VEGF.

According to a preferred embodiment, the VEGF antagonist is selected from a polypeptide such as an antibody, a peptibody, an immunoadhesin, a small molecule or an aptamer. In a preferred embodiment, the antibody is an anti-VEGF antibody such as bevacizumab (AVASTIN®) or an anti-VEGF receptor antibody such as an anti-VEGFR2 or an anti-VEGFR3 antibody. Other examples of VEGF antagonists include: VEGF-Trap, Mucagen, PTK787, SU11248, AG-013736, Bay 439006 (sorafenib), ZD-6474, CP632, CP-547632, AZD-2171, CDP-171, SU-14813, CHIR-258, AEE-788, SB786034, BAY579352, CDP-791, EG-3306, GW-786034, RWJ-417975/CT6758 and KRN-633.

An "anti-VEGF antibody" is an antibody that binds to VEGF with sufficient affinity and specificity. In certain embodiments, the antibody selected will normally have a sufficiently binding affinity for VEGF, for example, the antibody may bind hVEGF with a $K_d$ value of between 100 nM-1 pM. Antibody affinities may be determined by a surface plasmon resonance based assay (such as the BIAcore assay as described in PCT Application Publication No. WO2005/012359); enzyme-linked immunoabsorbent assay (ELISA); and competition assays (e.g. RIA's), for example. Preferably, the anti-VEGF antibody of the invention can be used as a therapeutic agent in targeting and interfering with diseases or conditions wherein the VEGF activity is involved. An anti-VEGF antibody will usually not bind to other VEGF homologues such as VEGF-B or VEGF-C, nor other growth factors such as P1GF, PDGF or bFGF. A preferred anti-VEGF antibody is a monoclonal antibody that binds to the same epitope as the monoclonal anti-VEGF antibody A4.6.1 produced by hybridoma ATCC HB 10709. More preferably the anti-VEGF antibody is a recombinant humanized anti-VEGF monoclonal antibody generated according to Presta et al. (1997) Cancer Res. 57:4593-4599, including but not limited to the antibody known as bevacizumab (BV; Avastin®). According to another embodiment, anti-VEGF antibodies that can be used include, but are not limited to the antibodies disclosed in WO 2005/012359. According to one embodiment, the anti-VEGF antibody comprises the variable heavy and variable light region of any one of the antibodies disclosed in Figures 24, 25, 26, 27 and 29 of WO 2005/012359 (e.g., G6, G6-23, G6-31, G6-23.1, G6-23.2, B20, B20-4 and B20.4.1). In another preferred embodiment, the anti-VEGF antibody known as ranibizumab is the VEGF antagonist administered for ocular disease such as diabetic neuropathy and AMD

In certain embodiment, the anti-VEGF antibody can be used as a therapeutic agent in targeting and interfering with diseases or conditions wherein the VEGF activity is involved. Also, the antibody may be subjected to other biological activity assays, e.g., in order to evaluate its effectiveness as a therapeutic. Such assays are known in the art and depend on the target antigen and intended use for the antibody. Examples include the HUVEC inhibition assay; tumor cell growth inhibition assays (as described in WO 89/06692, for example); antibody-dependent cellular cytotoxicity (ADCC) and complement-mediated cytotoxicity (CDC) assays (US Patent 5,500,362); and agonistic activity or hematopoiesis assays (see WO 95/27062). An anti-VEGF antibody will usually not bind to other VEGF homologues such as VEGF-B or VEGF-C, nor other growth factors such as P1GF, PDGF or bFGF. In one embodiment, anti-VEGF antibody is a monoclonal antibody that binds to the same epitope as the monoclonal anti-VEGF antibody A4.6.1 produced by hybridoma ATCC HB 10709. In another embodiment, the anti-VEGF antibody is a recombinant humanized anti-VEGF monoclonal antibody generated according to Presta et al. (1997) Cancer Res. 57:4593-4599, including but not limited to the antibody known as bevacizumab (BV; AVASTIN®).

The anti-VEGF antibody "Bevacizumab (BV)," also known as "rhuMAb VEGF" or "AVASTIN®," is a recombinant humanized anti-VEGF monoclonal antibody generated according to Presta et al. (1997) Cancer Res. 57:4593-4599. It comprises mutated human IgG1 framework regions and antigen-binding complementarity-determining regions from the murine anti-hVEGF monoclonal antibody A.4.6.1 that blocks binding of human VEGF to its receptors. Approximately 93% of the amino acid sequence of Bevacizumab, including most of the framework regions, is derived from human IgG1, and about 7% of the sequence is derived from the murine antibody A4.6.1. Bevacizumab has a molecular mass of about 149,000 daltons and is glycosylated. Bevacizumab and other humanized anti-VEGF antibodies are further described in U.S. Pat. No. 6,884,879 and WO 2005/044853.

The anti-VEGF antibody Ranibizumab or the LUCENTIS® antibody or rhuFab V2 is a humanized, affinity-matured anti-human VEGF Fab fragment. Ranibizumab is produced by standard recombinant technology methods in Escherichia coli expression vector and bacterial fermentation. Ranibizumab is not glycosylated and has a molecular mass of ~48,000 daltons. See WO98/45331 and US2003/0190317.

The two best characterized VEGF receptors are VEGFR1 (also known as Flt-1) and VEGFR2 (also known as KDR and FLK-1 for the murine homolog). The specificity of each receptor for each VEGF family member varies but VEGF-A binds to both Flt-1 and KDR. The full length Flt-1 receptor includes an extracellular domain that has seven Ig domains, a transmembrane domain, and an intracellular domain with tyrosine kinase activity. The extracellular domain is involved in the binding of VEGF and the intracellular domain is involved in signal transduction.

VEGF receptor molecules, or fragments thereof, that specifically bind to VEGF can be used as VEGF inhibitors that bind to and sequester the VEGF protein, thereby preventing it from signaling. In certain embodiments, the VEGF receptor molecule, or VEGF binding fragment thereof, is a soluble form, such as sFlt-1. A soluble form of the receptor exerts an inhibitory effect on the biological activity of the VEGF protein by binding to VEGF, thereby preventing it from binding to its natural receptors present on the surface of target cells. Also included are VEGF receptor fusion proteins, examples of which are described below.

A chimeric VEGF receptor protein is a receptor molecule having amino acid sequences derived from at least two different proteins, at least one of which is a VEGF receptor protein (e.g., the flt-1 or KDR receptor), that is capable of binding to and inhibiting the biological activity of VEGF. In certain embodiments, the chimeric VEGF receptor proteins of the present invention consist of amino acid sequences derived from only two different VEGF receptor molecules; however, amino acid sequences comprising one, two, three, four, five, six, or all seven Ig-like domains from the extracellular ligand-binding region of the flt-1 and/or KDR receptor can be linked to amino acid sequences from other unrelated proteins, for example, immunoglobulin sequences. Other amino acid sequences to which Ig-like domains are combined will be readily apparent to those of ordinary skill in the art. Examples of chimeric VEGF receptor proteins include, but not limited to, soluble Flt-1/Fc, KDR/Fc, or Flt-1/KDR/Fc (also known as VEGF Trap). (*See* for example PCT Application Publication No. WO97/44453).

A soluble VEGF receptor protein or chimeric VEGF receptor proteins includes VEGF receptor proteins which are not fixed to the surface of cells via a transmembrane domain. As such, soluble forms of the VEGF receptor, including chimeric receptor proteins, while capable of binding to and inactivating VEGF, do not comprise a transmembrane domain and thus generally do not become associated with the cell membrane of cells in which the molecule is expressed.

Additional VEGF inhibitors are described in, for example in WO 99/24440, PCT International Application PCT/IB99/00797, in WO 95/21613, WO 99/61422, U.S. Pat. No. 6,534,524, U.S. Pat. No. 5,834,504, WO 98/50356, U.S. Pat. No. 5,883,113, U.S. Pat. No. 5,886,020, U.S. Pat. No. 5,792,783, U.S. Pat. No. 6,653,308, WO 99/10349, WO 97/32856, WO 97/22596, WO 98/54093, WO 98/02438, WO 99/16755, and WO 98/02437, all of which are herein incorporated by reference in their entirety.

The term "B20 series polypeptide" as used herein refers to a polypeptide, including an antibody that binds to VEGF. B20 series polypeptides includes, but not limited to, antibodies derived from a sequence of the B20 antibody or a B20-derived antibody described in US Publication No. 2006/0280747, US Publication No. 2007/0141065 and/or US Publication No. 2007/0020267, the content of these patent applications are expressly incorporated herein by reference. In one embodiment, B20 series polypeptide is B20-4.1 as described in US Publication No. 20060280747, US Publication No. 20070141065 and/or US Publication No. 20070020267. In another embodiment, B20 series polypeptide is B20-4.1.1 described in US Patent Application 60/991,302, the entire disclosure of which is expressly incorporated herein by reference.

The term "G6 series polypeptide" as used herein refers to a polypeptide, including an antibody that binds to VEGF. G6 series polypeptides includes, but not limited to, antibodies derived from a sequence of the G6 antibody or a G6-derived antibody described in US Publication No. 2006/0280747, US Publication No. 2007/0141065 and/or US Publication No. 20070020267. G6 series polypeptides, as described in US Publication No. 2006/0280747, US Publication No. 2007/0141065 and/or US Publication No. 2007/0020267 include, but not limited to, G6-8, G6-23 and G6-31.

For additional antibodies *see* U.S. Pat. Nos. 7,060,269, 6,582,959, 6,703,020; 6,054,297; WO98/45332; WO 96/30046; WO94/10202; EP 0666868B1; U.S. Patent Application Publication Nos. 2006/009360, 2005/0186208, 2003/0206899, 2003/0190317, 2003/0203409, and 2005/0112126; and Popkov et al., Journal of Immunological Methods 288:149-164 (2004). In certain embodiments, other antibodies include those that bind to a functional epitope on human VEGF comprising of residues F17, M18, D19, Y21, Y25, Q89, I91, K101, E103, and C104 or, alternatively, comprising residues F17, Y21, Q22, Y25, D63, I83 and Q89.

Other anti-VEGF antibodies are also known, and described, for example, in Liang et al., J Biol Chem 281, 951-961 (2006).

An "effective response" of a patient or a patient's "responsiveness" or "sensitivity" to treatment with an anti-cancer agent refers to the clinical or therapeutic benefit imparted to a patient at risk for or suffering from cancer from or as a result of the treatment with an anti-cancer agent, such as, e.g., an anti-VEGF-A antibody. Such benefit includes cellular or biological responses, a complete response, a partial response, a stable disease (without progression or relapse), or a response with a later relapse of the patient from or as a result of the treatment with the antagonist. For example, an effective response can be reduced tumor size, progression-free survival, or overall survival.

"Antagonists as used herein refer to compounds or agents which inhibit or reduce the biological activity of the molecule to which they bind. Antagonists include antibodies, synthetic or native-sequence peptides, immunoadhesins, and small-molecule antagonists that bind to VEGF, optionally conjugated with or fused to another molecule. A "blocking" antibody or an "antagonist" antibody is one which inhibits or reduces biological activity of the antigen it binds.

An "agonist antibody," as used herein, is an antibody which partially or fully mimics at least one of the functional activities of a polypeptide of interest.

The term "antibody" herein is used in the broadest sense and specifically covers monoclonal antibodies, polyclonal antibodies, multispecific antibodies (*e.g.* bispecific antibodies) formed from at least two intact antibodies, and antibody fragments so long as they exhibit the desired biological activity.

In certain embodiments, an antibody used as a VEGF antagonist in a method provided herein is a multispecific antibody, e.g. a bispecific antibody. Multispecific antibodies are monoclonal antibodies that have binding specificities for at least two different sites. In certain embodiments, one of the binding specificities is for VEGF and the other is for any other antigen. In certain embodiments, bispecific antibodies may bind to two different epitopes of VEGF. Bispecific antibodies may also be used to localize cytotoxic agents to cells which express VEGF. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein, C. and Cuello, A.C., Nature 305 (1983) 537-540, WO 93/08829, and Traunecker, A. et al., EMBO J. 10 (1991) 3655-3659), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004); cross-linking two or more antibodies or fragments (see, e.g., US Patent No. 4,676,980, and Brennan, M. et al., Science 229 (1985) 81-83); using leucine zippers to produce bi-specific antibodies (see, e.g., Kostelny, S.A. et al., J. Immunol. 148 (1992) 1547-1553; using "diabody" technology for making bispecific antibody fragments (see, e.g., Holliger, P. et al., Proc. Natl. Acad. Sci. USA 90 (1993) 6444-6448); and using single-chain Fv (sFv) dimers (see,e.g. Gruber, M et al., J. Immunol. 152 (1994) 5368-5374); and preparing trispecific antibodies as described, e.g., in Tutt, A. et al., J. Immunol. 147 (1991) 60-69).

Engineered antibodies with three or more functional antigen binding sites, including "Octopus antibodies," are also included herein (see, e.g. US 2006/0025576).

The antibody or fragment herein also includes a "Dual Acting FAb" or "DAF" comprising an antigen binding site that binds to VEGF as well as another, different antigen (see, US 2008/0069820, for example).

The antibody or fragment an antibody used as a VEGF antagonist in a method provided herein provided herein also includes multispecfic antibodies as described in WO2009/080251, WO2009/080252, WO2009/080253, WO2009/080254, WO2010/112193, WO2010/115589, WO2010/136172, WO 2010/145792, and WO 2010/145793. Examples of bispecific VEGF antibodies are described e.g. in WO2010/040508 (VEGF-ANG2), PCT/EP2011/054504 (VEGF-ANG2), WO2005/087812 (VEGF-PDGF), WO2009120922 (VEGF-PDGFR beta), WO2011/039370 (VEGF-Dll4).

An "isolated" antibody is one which has been identified and separated and/or recovered from a component of its natural environment. Contaminant components of its natural environment are materials which would interfere with research, diagnostic or therapeutic uses for the antibody, and may include enzymes, hormones, and other proteinaceous or nonproteinaceous solutes. In some embodiments, an antibody is purified (1) to greater than 95% by weight of antibody as determined by, for example, the Lowry method, and in some embodiments, to greater than 99% by weight; (2) to a degree sufficient to obtain at least 15 residues of N-terminal or internal amino acid sequence by use of, for example, a spinning cup sequenator, or (3) to homogeneity by SDS-PAGE under reducing or nonreducing conditions using, for example, Coomassie blue or silver stain. Isolated antibody includes the antibody *in situ* within recombinant cells since at least one component of the antibody's natural environment will not be present. Ordinarily, however, isolated antibody will be prepared by at least one purification step.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light-chain variable domain is aligned with the variable domain of the heavy chain. Particular amino acid residues are believed to form an interface between the light-chain and heavy-chain variable domains.

The "variable region" or "variable domain" of an antibody refers to the amino-terminal domains of the heavy or light chain of the antibody. The variable domain of the heavy chain may be referred to as "VH." The variable domain of the light chain may be referred to as "VL." These domains are generally the most variable parts of an antibody and contain the antigen-binding sites.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions (HVRs) both in the light-chain and the heavy-chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen-binding site of antibodies (see Kabat et al., Sequences of Proteins of Immunological Interest, Fifth Edition, National Institute of Health, Bethesda, MD (1991)). The constant domains are not involved directly in the binding of an antibody to an antigen, but exhibit various effector functions, such as participation of the antibody in antibody-dependent cellular toxicity.

The "light chains" of antibodies (immunoglobulins) from any vertebrate species can be assigned to one of two clearly distinct types, called kappa ($\kappa$) and lambda ($\lambda$), based on the amino acid sequences of their constant domains.

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), *e.g.*, $IgG_1$, $IgG_2$, $IgG_3$, $IgG_4$, $IgA_1$, and $IgA_2$. The heavy-chain constant domains that correspond to the different classes of immunoglobulins are called $\alpha$, $\delta$, $\varepsilon$, $\gamma$, and $\mu$, respectively. The subunit structures and three-dimensional configurations of different classes of immunoglobulins are well known and described generally in, for example, Abbas et al. Cellular and Mol. Immunology, 4th ed. (W. B. Saunders, Co., 2000). An antibody may be part of a larger fusion molecule, formed by covalent or non-covalent association of the antibody with one or more other proteins or peptides.

The terms "full-length antibody," "intact antibody," and "whole antibody" are used herein interchangeably to refer to an antibody in its substantially intact form, not antibody fragments as defined below. The terms particularly refer to an antibody with heavy chains that contain an Fc region.

A "naked antibody" for the purposes herein is an antibody that is not conjugated to a cytotoxic moiety or radiolabel.

"Antibody fragments" comprise a portion of an intact antibody, preferably comprising the antigen-binding region thereof. Examples of antibody fragments include Fab, Fab', $F(ab')_2$, and Fv fragments; diabodies; linear antibodies; single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields a $F(ab')_2$ fragment that has two antigen-combining sites and is still capable of cross-linking antigen.

"Fv" is the minimum antibody fragment which contains a complete antigen-binding site. In one embodiment, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three HVRs of each variable domain interact to define an antigen-binding site on the surface of the VH-VL dimer. Collectively, the six HVRs confer antigen-binding specificity to the antibody. However, even a single variable domain (or half of an Fv comprising only three HVRs specific for an antigen) has the ability to recognize and bind antigen, although at a lower affinity than the entire binding site.

The Fab fragment contains the heavy- and light-chain variable domains and also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody-hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known.

"Single-chain Fv" or "scFv" antibody fragments comprise the VH and VL domains of an antibody, wherein these domains are present in a single polypeptide chain. Generally, the scFv polypeptide further comprises a polypeptide linker between the VH and VL domains that enables the scFv to form the desired structure for antigen binding. For a review of scFv, see, *e.g.*, Plueckthun, in The Pharmacology of Mono-clonal Antibodies, vol. 113, Rosenburg and Moore eds. (Springer-Verlag, New York: 1994), pp 269-315.

The term "diabodies" refers to antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 404097; WO 1993/01161; Hudson et al., Nat. Med. 9:129-134 (2003); and Holliger et al., PNAS USA 90: 6444-6448 (1993). Triabodies and tetrabodies are also described in Hudson et al., Nat. Med. 9:129-134 (2003).

The term "monoclonal antibody" as used herein refers to an antibody obtained from a population of substantially homogeneous antibodies, *i.e.*, the individual antibodies comprising the population are identical except for possible mutations, *e.g.*, naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target-binding sequence, to improve its production in cell culture, to reduce its immunogenicity *in vivo,* to create a multispecific antibody, *etc.*, and that an antibody comprising the altered target binding sequence is also a monoclonal antibody of this invention. In contrast to polyclonal antibody preparations, which typically include different antibodies directed against different determinants (epitopes), each monoclonal antibody of a monoclonal-antibody preparation is directed against a single determinant on an antigen. In addition to their specificity, monoclonal-antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (*e.g.*, Kohler and Milstein., Nature, 256:495-97 (1975); Hongo et al., Hybridoma, 14 (3): 253-260 (1995), Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, *e.g.*, U.S. Patent No. 4,816,567), phage-display technologies (see, *e.g.,* Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, PNAS USA 101(34): 12467-12472 (2004); and Lee et al., J. Immunol. Methods 284(1-2): 119-132(2004), and technologies for producing human or human-like antibodies in animals that have parts or all of the human immunoglobulin loci or genes encoding human immunoglobulin sequences (see, *e.g.,* WO 1998/24893; WO 1996/34096; WO 1996/33735; WO 1991/10741; Jakobovits et al., PNAS USA 90: 2551 (1993); Jakobovits et al., Nature 362: 255-258 (1993); Bruggemann et al., Year in Immunol. 7:33 (1993); U.S. Patent Nos. 5,545,807; 5,545,806; 5,569,825; 5,625,126; 5,633,425; and 5,661,016; Marks et al., Bio/Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996); and Lonberg and Huszar, Intern. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (*e.g.*, U.S. Pat. No. 4,816,567 and Morrison et al., PNAS USA 81:6851-6855 (1984)). Chimeric antibodies include PRIMATIZED® antibodies wherein the antigen-binding region of the antibody is derived from an antibody produced by, *e.g.*, immunizing macaque monkeys with the antigen of interest.

"Humanized" forms of non-human (*e.g.*, murine) antibodies are chimeric antibodies that contain minimal sequence derived from non-human immunoglobulin. In one embodiment, a humanized antibody is a human immunoglobulin (recipient antibody) in which residues from a HVR of the recipient are replaced by residues from a HVR of a non-human species (donor antibody) such as mouse, rat, rabbit, or nonhuman primate having the desired specificity, affinity, and/or capacity. In some instances, FR residues of the human immunoglobulin are replaced by corresponding non-human residues. Furthermore, humanized antibodies may comprise residues that are not found in the recipient antibody or in the donor antibody. These modifications may be made to further refine antibody performance. In general, a humanized antibody will comprise substantially all of at least one, and typically two, variable domains, in which all or substantially all of the hypervariable loops correspond to those of a non-human immunoglobulin, and all, or substantially all, of the FRs are those of a human immunoglobulin sequence. The humanized antibody optionally will also comprise at least a portion of an immunoglobulin constant region (Fc), typically that of a human immunoglobulin. For further details, see, *e.g.,* Jones et al., Nature 321:522-525 (1986); Riechmann et al., Nature 332:323-329 (1988); and Presta, Curr. Op. Struct. Biol. 2:593-596 (1992). See also, for example, Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994); and U.S. Pat. Nos. 6,982,321 and 7,087,409.

A "human antibody" is one which possesses an amino-acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, *e.g.,* immunized xenomice (see, *e.g.*, U.S. Pat. Nos. 6,075,181 and 6,150,584 regarding XENOMOUSE™ technology). See also, for example, Li et al., PNAS USA, 103:3557-3562 (2006) regarding human antibodies generated via a human B-cell hybridoma technology.

The term "hypervariable region," "HVR," or "HV," when used herein refers to the regions of an antibody-variable domain which are hypervariable in sequence and/or form structurally defined loops. Generally, antibodies comprise six HVRs; three in the VH (H1, H2, H3), and three in the VL (L1, L2, L3). In native antibodies, H3 and L3 display the most diversity of the six HVRs, and H3 in particular is believed to play a unique role in conferring fine specificity to antibodies. See, *e.g.*, Xu et al. Immunity 13:37-45 (2000); Johnson and Wu in Methods in Molecular Biology 248:1-25 (Lo, ed., Human Press, Totowa, NJ, 2003). Indeed, naturally occurring camelid antibodies consisting of a heavy chain only are functional and stable in the absence of light chain. See, *e.g.,* Hamers-Casterman et al., Nature 363:446-448 (1993) and Sheriff et al., Nature Struct. Biol. 3:733-736 (1996).

A number of HVR delineations are in use and are encompassed herein. The HVRs that are Kabat complementarity-determining regions (CDRs) are based on sequence variability and are the most commonly used (Kabat et al., Sequences of Proteins of Immunological Interest, 5th Ed. Public Health Service, National Institutes of Health, Bethesda, MD (1991)). Chothia refers instead to the location of the structural loops (Chothia and Lesk J. Mol. Biol. 196:901-917 (1987)). The AbM HVRs represent a compromise between the Kabat CDRs and Chothia structural loops, and are used by Oxford Molecular's AbM antibody-modeling software. The "contact" HVRs are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B (Kabat Numbering) |
| H1 | H31-H35 | H26-H35 | H26-H32 | H30-H35 (Chothia Numbering) |
| H2 | H50-H65 | H50-H58 | H53-H55 | H47-H58 |
| H3 | H95-H102 | H95-H102 | H96-H101 | H93-H101 |

HVRs may comprise "extended HVRs" as follows: 24-36 or 24-34 (L1), 46-56 or 50-56 (L2), and 89-97 or 89-96 (L3) in the VL, and 26-35 (H1), 50-65 or 49-65 (H2), and 93-102, 94-102, or 95-102 (H3) in the VH. The variable-domain residues are numbered according to Kabat *et al*., *supra,* for each of these extended-HVR definitions.

"Framework" or "FR" residues are those variable-domain residues other than the HVR residues as herein defined.

The expression "variable-domain residue-numbering as in Kabat" or "amino-acid-position numbering as in Kabat," and variations thereof, refers to the numbering system used for heavy-chain variable domains or light-chain variable domains of the compilation of antibodies in Kabat *et al*., *supra.* Using this numbering system, the actual linear amino acid sequence may contain fewer or additional amino acids corresponding to a shortening of, or insertion into, a FR or HVR of the variable domain. For example, a heavy-chain variable domain may include a single amino acid insert (residue 52a according to Kabat) after residue 52 of H2 and inserted residues *(e.g.* residues 82a, 82b, and 82c, *etc.* according to Kabat) after heavy-chain FR residue 82. The Kabat numbering of residues may be determined for a given antibody by alignment at regions of homology of the sequence of the antibody with a "standard" Kabat numbered sequence.

An "affinity-matured" antibody is one with one or more alterations in one or more HVRs thereof which result in an improvement in the affinity of the antibody for antigen, compared to a parent antibody which does not possess those alteration(s). In one embodiment, an affinity-matured antibody has nanomolar or even picomolar affinities for the target antigen. Affinity-matured antibodies are produced by procedures known in the art. For example, Marks et al., Bio/Technology 10:779-783 (1992) describes affinity maturation by VH- and VL-domain shuffling. Random mutagenesis of HVR and/or framework residues is described by, for example: Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

"Growth-inhibitory" antibodies are those that prevent or reduce proliferation of a cell expressing an antigen to which the antibody binds.

Antibodies that "induce apoptosis" are those that induce programmed cell death,, as determined by standard apoptosis assays, such as binding of annexin V, fragmentation of DNA, cell shrinkage, dilation of endoplasmic reticulum, cell fragmentation, and/or formation of membrane vesicles (called apoptotic bodies).

Antibody "effector functions" refer to those biological activities attributable to the Fc region (a native-sequence Fc region or amino-acid-sequence-variant Fc region) of an antibody, and vary with the antibody isotype. Examples of antibody effector functions include: C1q binding and complement- dependent cytotoxicity (CDC); Fc-receptor binding; antibody-dependent cell-mediated cytotoxicity (ADCC); phagocytosis; down-regulation of cell-surface receptors (e.g. B-cell receptor); and B-cell activation.

The term "Fc region" herein is used to define a C-terminal region of an immunoglobulin heavy chain, including native-sequence Fc regions and variant Fc regions. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue.

Unless indicated otherwise herein, the numbering of the residues in an immunoglobulin heavy chain is that of the EU index as in Kabat *et al*., *supra.* The "EU index as in Kabat" refers to the residue numbering of the human IgG1 EU antibody.

A "functional Fc region" possesses an "effector function" of a native-sequence Fc region. Exemplary "effector functions" include C1q binding; CDC; Fc-receptor binding; ADCC; phagocytosis; down-regulation of cell-surface receptors *(e.g.* B-cell receptor; BCR), *etc.* Such effector functions generally require the Fc region to be combined with a binding domain (*e.g.* an antibody-variable domain) and can be assessed using various assays as disclosed, for example, in definitions herein.

A "native-sequence Fc region" comprises an amino acid sequence identical to the amino acid sequence of an Fc region found in nature. Native-sequence human Fc regions include a native-sequence human IgG1 Fc region (non-A and A allotypes); native-sequence human IgG2 Fc region; native-sequence human IgG3 Fc region; and native-sequence human IgG4 Fc region, as well as naturally occurring variants thereof.

A "variant Fc region" comprises an amino acid sequence which differs from that of a native- sequence Fc region by virtue of at least one amino acid modification, preferably one or more amino acid substitution(s). Preferably, the variant Fc region has at least one amino acid substitution compared to a native-sequence Fc region or to the Fc region of a parent polypeptide, *e.g.* from about one to about ten amino acid substitutions, and preferably from about one to about five amino acid substitutions in a native- sequence Fc region or in the Fc region of the parent polypeptide. The variant Fc region herein will preferably possess at least about 80% homology with a native-sequence Fc region and/or with an Fc region of a parent polypeptide, and most preferably at least about 90% homology therewith, more preferably at least about 95% homology therewith.

The term "Fc-region-comprising antibody" refers to an antibody that comprises an Fc region. The C-terminal lysine (residue 447 according to the EU numbering system) of the Fc region may be removed, for example, during purification of the antibody or by recombinant engineering the nucleic acid encoding the antibody. Accordingly, a composition comprising an antibody having an Fc region according to this invention can comprise an antibody with K447, with all K447 removed, or a mixture of antibodies with and without the K447 residue.

"Fc receptor" or "FcR" describes a receptor that binds to the Fc region of an antibody. In some embodiments, an FcR is a native-human FcR. In some embodiments, an FcR is one which binds an IgG antibody (a gamma receptor) and includes receptors of the FcγRI, FcγRII, and FcγRIII subclasses, including allelic variants and alternatively spliced forms of those receptors. FcyRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see, *e.g.*, Daëron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

The term "Fc receptor" or "FcR" also includes the neonatal receptor, FcRn, which is responsible for the transfer of maternal IgGs to the fetus (Guyer et al., J. Immunol. 117:587 (1976) and Kim et al., J. Immunol. 24:249 (1994)) and regulation of homeostasis of immunoglobulins. Methods of measuring binding to FcRn are known (see, *e.g.,* Ghetie and Ward, Immunology Today, 18 (12):592-8 (1997); Ghetie et al., Nature Biotechnology, 15 (7):637-40 (1997); Hinton et al., J. Biol. Chem.,279(8):6213-6 (2004); WO 2004/92219 (Hinton et al.).

Binding to human FcRn *in vivo* and serum half-life of human FcRn high-affinity binding polypeptides can be assayed, *e.g.*, in transgenic mice or transfected human cell lines expressing human FcRn, or in primates to which the polypeptides with a variant Fc region are administered. WO 2000/42072 (Presta) describes antibody variants with improved or diminished binding to FcRs. See, also, for example, Shields et al. J. Biol. Chem. 9(2): 6591-6604 (2001).

"Binding affinity" generally refers to the strength of the sum total of noncovalent interactions between a single binding site of a molecule (*e.g.*, an antibody) and its binding partner (*e.g.*, an antigen). Unless indicated otherwise, as used herein, "binding affinity" refers to intrinsic binding affinity which reflects a 1:1 interaction between members of a binding pair (*e.g.*, antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative and exemplary embodiments for measuring binding affinity are described in the following.

In one embodiment, the "Kd" or "Kd value" according to this invention is measured by a radiolabeled antigen-binding assay (RIA) performed with the Fab version of an antibody of interest and its antigen as described by the following assay. Solution-binding affinity of Fabs for antigen is measured by equilibrating Fab with a minimal concentration of ($^{125}$I)-labeled antigen in the presence of a titration series of unlabeled antigen, then capturing bound antigen with an anti-Fab antibody-coated plate (see, *e.g.,* Chen et al., J. Mol. Biol. 293:865-881 (1999)). To establish conditions for the assay, microtiter plates (DYNEX Technologies, Inc.) are coated overnight with 5 μg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [$^{125}$I]-antigen are mixed with serial dilutions of a Fab of interest (*e.g.*, consistent with assessment of the anti-VEGF antibody, Fab-12, in Presta et al., Cancer Res. 57:4593-4599 (1997)). The Fab of interest is then incubated overnight; however, the incubation may continue for a longer period (*e.g.*, about 65 hours) to ensure that equilibrium is reached. Thereafter, the mixtures are transferred to the capture plate for incubation at room temperature (*e.g.*, for one hour). The solution is then removed and the plate washed eight times with 0.1% TWEEN-20™ surfactant in PBS. When the plates have dried, 150 μl/well of scintillant (MICROSCINT-20™; Packard) is added, and the plates are counted on a TOPCOUNT™ gamma counter (Packard) for ten minutes. Concentrations of each Fab that give less than or equal to 20% of maximal binding are chosen for use in competitive binding assays.

According to another embodiment, the Kd or Kd value is measured by using surface-plasmon resonance assays using a BIACORE®-2000 or a BIACORE®-3000 instrument (BIAcore, Inc., Piscataway, NJ) at 25°C with immobilized antigen CM5 chips at ~10 response units (RU). Briefly, carboxymethylated dextran biosensor chips (CM5, BIAcore Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 μg/ml (~0.2 μM) before injection at a flow rate of 5 μl/minute to achieve approximately ten response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% TWEEN 20™ surfactant (PBST) at 25°C at a flow rate of approximately 25 μl/min. Association rates ($k_{on}$) and dissociation rates ($k_{off}$) are calculated using a simple one-to-one Langmuir binding model (BIAcore® Evaluation Software version 3.2) by simultaneously fitting the association and dissociation sensorgrams. The equilibrium dissociation constant (Kd) is calculated as the ratio $k_{off}/k_{on}$. See, *e.g.*, Chen et al., J. Mol. Biol. 293:865-881 (1999). If the on-rate exceeds $10^6$ $M^{-1}s^{-1}$ by the surface-plasmon resonance assay above, then the on-rate can be determined by using a fluorescent quenching technique that measures the increase or decrease in fluorescence-emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow-equipped spectrophotometer (Aviv Instruments) or a 8000-series SLM-AMINCO™ spectrophotometer (ThermoSpectronic) with a stirred cuvette.

An "on-rate," "rate of association," "association rate," or "$k_{on}$" according to this invention can also be determined as described above using a BIACORE®-2000 or a BIACORE®-3000 system (BIAcore, Inc., Piscataway, NJ).

The term "substantially similar" or "substantially the same," as used herein, denotes a sufficiently high degree of similarity between two numeric values (for example, one associated with an antibody of the invention and the other associated with a reference/comparator antibody), such that one of skill in the art would consider the difference between the two values to be of little or no biological and/or statistical significance within the context of the biological characteristic measured by said values (*e.g.*, Kd values). The difference between said two values is, for example, less than about 50%, less than about 40%, less than about 30%, less than about 20%, and/or less than about 10% as a function of the reference/comparator value.

The phrase "substantially reduced," or "substantially different," as used herein, denotes a sufficiently high degree of difference between two numeric values (generally one associated with a molecule and the other associated with a reference/comparator molecule) such that one of skill in the art would consider the difference between the two values to be of statistical significance within the context of the biological characteristic measured by said values (*e.g.*, Kd values). The difference between said two values is, for example, greater than about 10%, greater than about 20%, greater than about 30%, greater than about 40%, and/or greater than about 50% as a function of the value for the reference/comparator molecule.

In certain embodiments, the humanized antibody useful herein further comprises amino acid alterations in the IgG Fc and exhibits increased binding affinity for human FcRn over an antibody having wild-type IgG Fc, by at least 60 fold, at least 70 fold, at least 80 fold, more preferably at least 100 fold, preferably at least 125 fold, even more preferably at least 150 fold to about 170 fold.

A "disorder" or "disease" is any condition that would benefit from treatment with a substance/molecule or method of the invention. This includes chronic and acute disorders or diseases including those pathological conditions which predispose the mammal to the disorder in question. Non-limiting examples of disorders to be treated herein include cancer (e.g., malignant and benign tumors; non-leukemias and lymphoid malignancies); neuronal, glial, astrocytal, hypothalamic and other glandular, macrophagal, epithelial, stromal and blastocoelic disorders; and inflammatory, immunologic and other angiogenesis-related disorders.

The terms "cell proliferative disorder" and "proliferative disorder" refer to disorders that are associated with some degree of abnormal cell proliferation. In one embodiment, the cell proliferative disorder is cancer. In one embodiment, the cell proliferative disorder is angiogenesis.

"Tumor", as used herein, refers to all neoplastic cell growth and proliferation, whether malignant or benign, and all pre-cancerous and cancerous cells and tissues. The terms "cancer", "cancerous", "cell proliferative disorder", "proliferative disorder" and "tumor" are not mutually exclusive as referred to herein.

The terms "cancer" and "cancerous" refer to or describe the physiological condition in mammals that is typically characterized by unregulated cell proliferation. Examples of cancer include but are not limited to, carcinoma, lymphoma, blastoma, sarcoma, and leukemia. More particular examples of such cancers include squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including, e.g., gastrointestinal cancer), pancreatic cancer (including, e.g., metastic pancreatic cancer), glioblastoma, cervical cancer, ovarian cancer, liver cancer, bladder cancer, hepatoma, breast cancer (including locally advanced, recurrent or metastatic HER-2 negative breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome.

The term "anti-neoplastic composition" or "anti-cancer composition" or "anti-cancer agent" refers to a composition useful in treating cancer comprising at least one active therapeutic agent, *e.g.*, "anti-cancer agent." Examples of therapeutic agents (anti-cancer agents) include, but are limited to, *e.g.,* chemotherapeutic agents, growth inhibitory agents, cytotoxic agents, agents used in radiation therapy, anti-angiogenesis agents, anti-lymphangiogenesis agents, apoptotic agents, anti-tubulin agents, and other-agents to treat cancer, such as anti-HER-2 antibodies, anti-CD20 antibodies, an epidermal growth factor receptor (EGFR) antagonist *(e.g.,* a tyrosine kinase inhibitor), HER1/EGFR inhibitor (*e.g.*, erlotinib (Tarceva™), platelet derived growth factor inhibitors (*e.g.*, Gleevec™ (Imatinib Mesylate)), a COX-2 inhibitor (*e.g.*, celecoxib), interferons, cytokines, antagonists (*e.g.*, neutralizing antibodies) that bind to one or more of the following targets ErbB2, ErbB3, ErbB4, PDGFR-beta, BlyS, APRIL, BCMA VEGF, or VEGF receptor(s), TRAIL/Apo2, and other bioactive and organic chemical agents, etc. Combinations thereof are also included in the invention.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or disorder.

An "effective amount" refers to an amount effective, at dosages and for periods of time necessary, to achieve the desired therapeutic or prophylactic result.

A "therapeutically effective amount" of a substance/molecule of the invention, agonist or antagonist may vary according to factors such as the disease state, age, sex, and weight of the individual, and the ability of the substance/molecule, agonist or antagonist to elicit a desired response in the individual. A therapeutically effective amount is also one in which any toxic or detrimental effects of the substance/molecule, agonist or antagonist are outweighed by the therapeutically beneficial effects. The term "therapeutically effective amount" refers to an amount of an antibody, polypeptide or antagonist of this invention effective to "treat" a disease or disorder in a mammal (aka patient). In the case of cancer, the therapeutically effective amount of the drug can reduce the number of cancer cells; reduce the tumor size or weight; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug can prevent growth and/or kill existing cancer cells, it can be cytostatic and/or cytotoxic. In one embodiment, the therapeutically effective amount is a growth inhibitory amount. In another embodiment, the therapeutically effective amount is an amount that extends the survival of a patient. In another embodiment, the therapeutically effective amount is an amount that improves progression free survival of a patient. "Progression free survival" as used herein refers to the length of time during and after treatment during which, according to the assessment of the treating physician or investigator, the patient's disease does not become worse, *i.e.*, does not progress.

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount is less than the therapeutically effective amount.

The term "cytotoxic agent" as used herein refers to a substance that inhibits or prevents the function of cells and/or causes destruction of cells. The term is intended to include radioactive isotopes (*e.g.*, $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu), chemotherapeutic agents e.g. methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anti-cancer agents disclosed below. Other cytotoxic agents are described below. A tumoricidal agent causes destruction of tumor cells.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (*e.g.*, calicheamicin, especially calicheamicin gammalI and calicheamicin omegaI1 (see, *e.g.*, Angew. Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, *e.g.*, TAXOL® paclitaxel (Bristol-Myers Squibb Oncology, Princeton, N.J.), ABRAXANE™ Cremophor-free, albumin-engineered nanoparticle formulation of paclitaxel (American Pharmaceutical Partners, Schaumberg, Illinois), and TAXOTERE® doxetaxel (Rhône-Poulenc Rorer, Antony, France); chloranbucil; gemcitabine (GEMZAR®); 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluorometlhylornithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN™) combined with 5-FU and leucovovin. Additional chemotherapeutic agents include the cytotoxic agents useful as antibody drug conjugates, such as maytansinoids (DM1, for example) and the auristatins MMAE and MMAF, for example.

"Chemotherapeutic agents" also include "anti-hormonal agents" that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX® tamoxifen), EVISTA® raloxifene, droloxifene, 4-hydroxytamoxifen, trioxifene, keoxifene, LY117018, onapristone, and FARESTON® toremifene; anti-progesterones; estrogen receptor down-regulators (ERDs); agents that function to suppress or shut down the ovaries, for example, leutinizing hormone-releasing hormone-(LHRH) agonists such as LUPRON® and ELIGARD® leuprolide acetate, goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole. In addition, such definition of chemotherapeutic agents includes bisphosphonates such as clodronate (for example, BONEFOS® or OSTAC®), DIDROCAL® etidronate, NE-58095, ZOMETA® zoledronic acid/zoledronate, FOSAMAX® alendronate, AREDIA® pamidronate, SKELID® tiludronate, or ACTONEL® risedronate; as well as troxacitabine (a 1,3-dioxolane nucleoside cytosine analog); antisense oligonucleotides, particularly those that inhibit expression of genes in signaling pathways implicated in abherant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; LURTOTECAN® topoisomerase 1 inhibitor; ABARELIX® rmRH; lapatinib ditosylate (an ErbB-2 and EGFR dual tyrosine kinase small-molecule inhibitor also known as GW572016); and pharmaceutically acceptable salts, acids or derivatives of any of the above.

A "growth inhibitory agent" when used herein refers to a compound or composition which inhibits growth and/or proliferation of a cell. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as the anthracycline antibiotic doxorubicin ((8S-cis)-10-[(3-amino-2,3,6-trideoxy-α-L-lyxo-hexapyranosyl)oxy]-7,8,9,10-tetrahydro-6,8,11-trihydroxy-8-(hydroxyacetyl)-1-methoxy-5,12-naphthacenedione), epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and anti-neoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

As used herein, the term "patient" refers to any single animal, more preferably a mammal (including such non-human animals as, for example, dogs, cats, horses, rabbits, zoo animals, cows, pigs, sheep, and non-human primates) for which treatment is desired. Most preferably, the patient herein is a human.

A "subject" herein is any single human subject, including a patient, eligible for treatment who is experiencing or has experienced one or more signs, symptoms, or other indicators of an angiogenic disorder. Intended to be included as a subject are any subjects involved in clinical research trials not showing any clinical sign of disease, or subjects involved in epidemiological studies, or subjects once used as controls. The subject may have been previously treated with an anti-cancer agent, or not so treated. The subject may be naive to an additional agent(s) being used when the treatment herein is started, *i.e.*, the subject may not have been previously treated with, for example, an anti-neoplastic agent, a chemotherapeutic agent, a growth inhibitory agent, a cytotoxic agent at "baseline" (*i.e.*, at a set point in time before the administration of a first dose of an anti-cancer in the treatment method herein, such as the day of screening the subject before treatment is commenced). Such "naïve" subjects are generally considered to be candidates for treatment with such additional agent(s).

The term "pharmaceutical formulation" refers to a sterile preparation that is in such form as to permit the biological activity of the medicament to be effective, and which contains no additional components that are unacceptably toxic to a subject to which the formulation would be administered.

A "sterile" formulation is aseptic or free from all living microorganisms and their spores.

A "package insert" is used to refer to instructions customarily included in commercial packages of therapeutic products or medicaments, that contain information about the indications, usage, dosage, administration, contraindications, other therapeutic products to be combined with the packaged product, and/or warnings concerning the use of such therapeutic products or medicaments, *etc.*

A "kit" is any manufacture (e.g. a package or container) comprising at least one reagent, *e.g.*, a medicament for treatment of an angiogenic disorder, or a probe for specifically detecting a biomarker gene or protein of the invention. The manufacture is preferably promoted, distributed, or sold as a unit for performing the methods of the present invention.

For purposes of non-response to medicament(s), a subject who experiences "a clinically unacceptably high level of toxicity" from previous or current treatment with one or more medicaments experiences one or more negative side-effects or adverse events associated therewith that are considered by an experienced clinician to be significant, such as, for example, serious infections, congestive heart failure, demyelination (leading to multiple sclerosis), significant hypersensitivity, neuropathological events, high degrees of autoimmunity, a cancer such as endometrial cancer, non-Hodgkin's lymphoma, breast cancer, prostate cancer, lung cancer, ovarian cancer, or melanoma, tuberculosis (TB), *etc.*

By "reducing the risk of a negative side effect" is meant reducing the risk of a side effect resulting from treatment with the antagonist herein to a lower extent than the risk observed resulting from treatment of the same patient or another patient with a previously administered medicament. Such side effects include those set forth above regarding toxicity, and are preferably infection, cancer, heart failure, or demyelination.

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to various embodiments herein, one may use the results of an analytical assay to determine whether a specific therapeutic regimen using an anti-cancer agent, such as anti-VEGF antibody, should be performed.

### III. Methods

The present invention provides methods for identifying patients who may benefit from treatment with an anti-cancer therapy comprising a VEGF antagonist, methods of predicting responsiveness of a patient suffering from cancer to treatment with an anti-cancer therapy comprising a VEGF-A antagonist, methods for determining the likelihood that a patient with cancer will exhibit benefit from anti-cancer therapy comprising a VEGF-A antagonist, and methods for optimizing the therapeutic efficacy of an anti-cancer therapy comprising a VEGF-A antagonist

The methods comprise determining an expression level of $VEGF_{110}$ in a sample obtained from the patient, wherein a level of $VEGF_{110}$ in the sample obtained from the patient at or above a reference level indicates that the patient may benefit from treatment with the anti-cancer therapy comprising a VEGF antagonist, that the patient has increased likelihood of benefit from the anti-cancer therapy, or that the patient is more likely to be responsive to treatment with the anti-cancer therapy. In some embodiments, the methods further comprise administering an anti-cancer therapy comprising a VEGF antagonist to the patient.

The invention further provides methods for treating cancer in a patient. The methods comprise determining that a sample obtained from the patient has a level of $VEGF_{110}$at or above a reference level, and administering an effective amount of an anti-cancer therapy comprising a VEGF antagonist to said patient, whereby the cancer is treated. In some embodiments, the methods further comprise administering additional agent(s) (e.g., a second, third, or fourth agent) to the patient.

### A. Detection Methods

The disclosed methods and assays provide for convenient, efficient, and potentially cost-effective means to obtain data and information useful in assessing appropriate or effective therapies for treating patients. For example, according to the methods of the invention, a patient could provide a blood sample before treatment with anti-cancer therapy comprising a VEGF antagonist and the level of $VEGF_{110}$ in the sample could be determined and compared to the level of $VEGF_{110}$ in a reference sample or to a predetermined reference value, respectively. Patients with an increased level of $VEGF_{110}$ are identified as patients likely to respond to anti-cancer therapy comprising a VEGF antagonist, such as an anti-VEGF antibody. The methods may be conducted in a variety of assay formats, including assays detecting protein expression (such enzyme immunoassays) and biochemical assays detecting appropriate activity. Determination of expression or the presence of such biomarkers in the samples is predictive that the patient providing the sample will be sensitive to the biological effects of a VEGF antagonist. Typically an expression level of $VEGF_{110}$ in a sample obtained from the patient at or above a reference level indicates that a patient will respond to or be sensitive to treatment with a VEGF antagonist.

One of skill in the medical arts, particularly pertaining to the application of diagnostic tests and treatment with therapeutics, will recognize that biological systems are somewhat variable and not always entirely predictable, and thus many good diagnostic tests or therapeutics are occasionally ineffective. Thus, it is ultimately up to the judgment of the attending physician to determine the most appropriate course of treatment for an individual patient, based upon test results, patient condition and history, and his or her own experience. There may even be occasions, for example, when a physician will choose to treat a patient with a VEGF antagonist even when a patient is not predicted to be particularly sensitive to VEGF antagonists, based on data from diagnostic tests or from other criteria, particularly if all or most of the other obvious treatment options have failed, or if some synergy is anticipated when given with another treatment.

In further expressed embodiments, the present invention provides a method of predicting the sensitivity of a patient to treatment with an anti-cancer therapy comprising a VEGF antagonist, or predicting whether a patient will respond effectively to treatment with an anti-cancer therapy comprising a VEGF antagonist, comprising assessing the level of $VEGF_{110}$ in the sample; and predicting the sensitivity of the patient to inhibition by a VEGF antagonist, wherein an expression level of $VEGF_{110}$ at or above a reference level correlates with high sensitivity of the patient to effective response to treatment with a VEGF antagonist.

The sample may be taken from a patient who is suspected of having, or is diagnosed as having an cancer, including, e.g., colorectal cancer, glioblastoma, renal cancer, ovarian cancer, breast cancer (including, e.g., locally advanced, recurrent or metastatic HER-2 negative breast cancer), pancreatic cancer (including, e.g., metastatic pancreatic cancer), gastric cancer and lung cancer, and hence is likely in need of treatment or from a normal individual who is not suspected of having any disorder. For assessment of marker expression, patient samples, such as those containing cells, or proteins or nucleic acids produced by these cells, may be used in the methods of the present invention. In the methods of this invention, the level of a biomarker can be determined by assessing the amount (e.g. absolute amount or concentration) of the markers in a sample, preferably assessed in bodily fluids or excretions containing detectable levels of biomarkers. Bodily fluids or secretions useful as samples in the present invention include, e.g., blood, lymphatic fluid, sputum, ascites, or any other bodily secretion or derivative thereof. The word blood is meant to include whole blood, plasma, serum, or any derivative of blood. Assessment of a biomarker in such bodily fluids or excretions can sometimes be preferred in circumstances where an invasive sampling method is inappropriate or inconvenient. However, the sample to be tested herein is preferably blood, most preferably blood plasma. In one embodiment, the sample is EDTA-plasma. In one embodiment, the sample is citrate-plasma.

The sample may be frozen, fresh, fixed (*e.g.* formalin fixed), centrifuged, and/or embedded *(e.g.* paraffin embedded), *etc.* The cell sample can, of course, be subjected to a variety of well-known post-collection preparative and storage techniques (*e.g.*, nucleic acid and/or protein extraction, fixation, storage, freezing, ultrafiltration, concentration, evaporation, centrifugation, *etc*.) prior to assessing the amount of the marker in the sample. Likewise, biopsies may also be subjected to post-collection preparative and storage techniques, e.g., fixation.

### A. Detection of $VEGF_{110}$

$VEGF_{110}$ protein can be detected using any method known in the art. For example, tissue or cell samples from mammals can be conveniently assayed for, *e.g*., proteins using Westerns, ELISAs, etc.Many references are available to provide guidance in applying the above techniques (Kohler et al., Hybridoma Techniques (Cold Spring Harbor Laboratory, New York, 1980); Tijssen, Practice and Theory of Enzyme Immunoassays (Elsevier, Amsterdam, 1985); Campbell, Monoclonal Antibody Technology (Elsevier, Amsterdam, 1984); Hurrell, Monoclonal Hybridoma Antibodies: Techniques and Applications (CRC Press, Boca Raton, FL, 1982); and Zola, Monoclonal Antibodies: A Manual of Techniques, pp. 147-158 (CRC Press, Inc., 1987)).

If reference is made to the detection or level of $VEGF_{110}$ this means that the $VEGF_{110}$ isoform i.e. the cleavage product $VEGF_{110}$ is measured.

As to detection of $VEGF_{110}$ protein, various assays are available. For example, the sample may be contacted with an antibody or an antibody combination (e.g. in a sandwich assay) preferentially or specifically binding the short VEGF-A isoform $VEGF_{110}$. Preferably $VEGF_{110}$ is detected with an at least 3-fold higher sensitivity as compared to the longer isoforms, especially as compared to $VEGF_{165}$. In one embodiment an antibody having at least 3-fold higher sensitivity for $VEGF_{110}$ as compared to VEGF165 is used. Such at least 3-fold higher sensitivity for $VEGF_{110}$ is assessed by comparing $VEGF_{165}$ (purity at least 90% by SDS-PAGE and concentration determined by OD 280nm) and the cleavage product $VEGF_{110}$ (purity at least 90% by SDS-PAGE and concentration determined by OD 280nm) using the same reagents. If in this comparison the signal obtained for $VEGF_{165}$ is only one third or less of the signal as obtained with $VEGF_{110}$, then $VEGF_{110}$ is detected with an at least 3-fold higher sensitivity. As the skilled artisan will appreciate the signal is preferably read of at about 50% of the maximal signal. Also preferred the sensitivity for $VEGF_{110}$ is at least 4-fold, 5-fold, 6-fold, 7-fold, 8-fold or 9-fold higher as compared to the long isoforms, especially as compared to $VEGF_{165}$.

In one preferred embodiment $VEGF_{110}$ is specifically detected.

Such specific detection is e.g. possible if antibodies, especially monoclonal antibodies are used and employed that bind to the free C-terminal end of $VEGF_{110}$, respectively. Such $VEGF_{110}$ anti C-terminus antibody does not bind to any VEGF-A isoform comprising amino acid $_{110}$ as part of a longer polypeptide chain or to shorter VEGF-A fragments ending e.g. at amino acid 109. Specific binding to $VEGS_{110}$ in the above sense is acknowledged, if the antibody used exhibits less than 10% cross-reactivity with a shorter fragment of VEGF not having amino acid 110 at its C-terminus and less than 10% cross-reactivity with those VEGF-A isoforms not having a free C-terminal amino acid 110. Also preferred the cross-reactivity will be less than 5%, 4%, 3%, 2% and 1%, respectively, for both shorter fragments of VEGF and VEGF isoforms not having a free C-terminal amino acid 110.

Appropriate specific antibodies only binding the short VEGF cleavage product $VEGS_{110}$ can be obtained according to standard procedures. Usually a peptide representing or comprising the C-terminal most at least 4, 5, 6, 7, 8, 9, 10 or more amino acids of $VEGF_{110}$ will be synthesized, optionally coupled to a carrier and used for immunization. Specific polyclonal antibodies can be obtained by appropriate immunosorption steps. Monoclonal antibodies can easily be screened for reactivity with $VEGF_{110}$, and appropriate low cross-reactivity. Low cross-reactivity in terms of the $VEGF_{110}$-specific antibody can be assessed for both shorter fragments of $VEGF_{110}$ (e.g. lacking the C-terminal amino acid of $VEGF_{110}$) and VEGF-A isoforms not having a free C-terminal amino acid of $VEGF_{110}$.

Various measurement methods are at stake and, for example, the sample may be contacted with an antibody for $VEGF_{110}$ under conditions sufficient for an antibody-$VEGF_{110}$ complex to form, and then detecting both these complexes. The presence of $VEGF_{110}$ protein may be detected in a number of ways, such as by Western blotting (with or without immunoprecipitation), 2-dimensional SDS-PAGE, immunoprecipitation, fluorescence activated cell sorting (FACS), flow cytometry, and ELISA procedures for assaying a wide variety of tissues and samples, including plasma or serum. A wide range of immunoassay techniques using such an assay format are available, see, *e.g.,* U.S. Pat. Nos. 4,016,043, 4,424,279, and 4,018,653. These include both single-site and two-site or "sandwich" assays of the non-competitive types, as well as in the traditional competitive binding assays. These assays also include direct binding of a labeled antibody to a target biomarker.

Sandwich assays are among the most useful and commonly used assays. A number of variations of the sandwich assay technique exist, and all are intended to be encompassed by the present invention. Briefly, in a typical forward assay, an unlabelled antibody is immobilized on a solid substrate, and the sample to be tested brought into contact with the bound molecule. Immobilization of this capture antibody can be by direct adsorption to a solid phase or indirectly, e.g. via a specific binding pair, e.g. via the streptavidin-biotin binding pair. After a suitable period of incubation, for a period of time sufficient to allow formation of an antibody-antigen complex, a second antibody specific to the antigen (i.e., $VEGF_{110}$), labeled with a reporter molecule capable of producing a detectable signal is then added and incubated, allowing time sufficient for the formation of another complex of antibody-antigen-labeled antibody. Any unreacted material is washed away, and the presence of the $VEGF_{110}$ is determined by observation of a signal produced by the reporter molecule. The results may either be qualitative, by simple observation of the visible signal, or may be quantitated by comparing with a control sample containing known amounts of biomarker. In an alternative set-up the antibody specific for $VEGF_{110}$, respectively, is immobilized and an antibody binding to $VEGF_{110}$, optionally carrying a reporter molecule may be used to detect the target molecules.

Variations on the forward assay include a simultaneous assay, in which both sample and labeled antibody are added simultaneously to the bound antibody. These techniques are well known to those skilled in the art, including any minor variations as will be readily apparent. In a typical forward sandwich assay, a first antibody is either covalently or passively bound to a solid surface. The solid surface is typically glass or a polymer, the most commonly used polymers being cellulose, polyacrylamide, nylon, polystyrene, polyvinyl chloride, or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (*e.g.* 2-40 minutes or overnight if more convenient) and under suitable conditions (*e.g.,* from room temperature to 40°C such as between 25° C and 32° C inclusive) to allow for binding between the first or capture antibody and the corresponding antigen. Following the incubation period, the solid phase, comprising the first or capture antibody and bound thereto the antigen is washed, and incubated with a secondary or labeled antibody binding to another epitope on the antigen. The second antibody is linked to a reporter molecule which is used to indicate the binding of the second antibody to the complex of first antibody and the antigen of interest

An alternative, competitive method involves immobilizing $VEGF_{110}$ on a solid phase and then exposing the immobilized target together with the sample to a specific antibody to $VEGF_{110}$, respectively, which may or may not be labeled with a reporter molecule. Depending on the amount of target and the strength of the reporter molecule signal, a competition by the target molecule may be detectable directly via such labeled antibody. Alternatively, a second labeled antibody, specific to the first antibody is exposed to the target-first antibody complex to form a target-first antibody-second antibody tertiary complex. The complex is detected by the signal emitted by the reporter molecule. By "reporter molecule", as used in the present specification, is meant a molecule which, by its chemical nature, provides an analytically identifiable signal which allows the detection of antigen-bound antibody. The most commonly used reporter molecules in this type of assay are either enzymes, fluorophores or radionuclide containing molecules (*i.e.,* radioisotopes) and chemiluminescent molecules.

In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, beta-galactosidase, and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labeled antibody is added to the first antibody-molecular marker complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of $VEGF_{110}$ which was present in the sample. Alternately, fluorescent compounds, such as fluorescein and rhodamine, may be chemically coupled to antibodies without altering their binding capacity. When activated by illumination with light of a particular wavelength, the fluorochrome-labeled antibody adsorbs the light energy, inducing a state to excitability in the molecule, followed by emission of the light at a characteristic color visually detectable with a light microscope. As in the EIA, the fluorescent labeled antibody is allowed to bind to the first antibody-molecular marker complex. After washing off the unbound reagent, the remaining tertiary complex is then exposed to the light of the appropriate wavelength, the fluorescence observed indicates the presence of $VEGF_{110}$. Immunofluorescence and EIA techniques are both very well established in the art. However, other reporter molecules, such as radioisotope, chemiluminescent or bioluminescent molecules, may also be employed. Immunoassays for detecting VEGF are described in, e.g., U.S. Patent Nos. 6,855,508 and 7,541,160 and U.S. Patent Publication No. 2010/0255515. Suitable platforms for detecting VEGF are described in, e.g., EP 0939319 and EP 1610129.

## B. Kits

For use in detection of $VEGF_{110}$, kits or articles of manufacture are also provided by the invention. Such kits can be used to determine if a subject will be effectively responsive to an anti-cancer therapy comprising a VEGF antagonist. These kits may comprise a carrier means being compartmentalized to receive in close confinement one or more container means such as vials, tubes, and the like, each of the container means comprising one of the separate elements to be used in the method. For example, one of the container means may comprise a probe that is or can be detectably labeled. Such probe may be an antibody specific for $VEGF_{110}$ protein. The kit may also have containers comprising a reporter-means, such as a biotin-binding protein, *e.g*., avidin or streptavidin, bound to a reporter molecule, such as an enzymatic, florescent, or radioisotope label.

Such kit will typically comprise the container described above and one or more other containers comprising materials desirable from a commercial and user standpoint, including buffers, diluents, filters, needles, syringes, and package inserts with instructions for use. A label may be present on the container to indicate that the composition is used for a specific application, and may also indicate directions for either *in vivo* or *in vitro* use, such as those described above.

The kits of the invention have a number of embodiments. A typical embodiment is a kit comprising a container, a label on said container, and a composition contained within said container, wherein the composition includes an antibody that specifically binds to $VEGF_{110}$ and the label on said container indicates that the composition can be used to evaluate the presence of $VEGF_{110}$ in a sample, and wherein the kit includes instructions for using the antibody for evaluating the presence of $VEGF_{110}$ in a particular sample type. The kit can further comprise a set of instructions and materials for preparing a sample and applying antibody to the sample. The kit may include both a primary and secondary antibody, wherein the secondary antibody is conjugated to a label, *e.g.,* an enzymatic label.

Other optional components of the kit include one or more buffers (*e.g.,* block buffer, wash buffer, substrate buffer, *etc.*), other reagents such as substrate (*e.g*., chromogen) that is chemically altered by an enzymatic label, epitope retrieval solution, control samples (positive and/or negative controls), control slide(s), *etc.* Kits can also include instructions for interpreting the results obtained using the kit.

In one further specific embodiment, for antibody-based kits, the kit can comprise, for example: (1) a first antibody (*e.g*., attached to a solid support or capable of binding to a solid support) that binds to $VEGF_{110}$; (2) a second, different antibody that preferentially binds to the $VEGF_{110}$, respectively. Preferably the later antibody is labeled with the same reporter molecule. Of course it is also possible to exchange the first for the second antibody and vice versa, when designing such assay.

## B. Methods of Treatment

Some methods of the invention further comprise administering a VEGF antagonist to a patient with an increased level of $VEGF_{110}$ compared to a reference sample. Other embodiments of the invention provide methods of treating a patient with an antic-cancer therapy comprising a VEGF antagonist. Dosage regimens may be adjusted to provide the optimum desired response (*e.g*., a therapeutic response). For example, a dose may be administered, several divided doses may be administered over time or the dose may be proportionally reduced or increased as indicated by exigencies of the therapeutic situation.

A physician having ordinary skill in the art can readily determine and prescribe the effective amount of the pharmaceutical composition required, depending on such factors as the particular type of anti-cancer agent. For example, the physician could start with doses of such anti-cancer agent, such as an anti-VEGF-A antibody, employed in the pharmaceutical composition at levels lower than that required in order to achieve the desired therapeutic effect and gradually increase the dosage until the desired effect is achieved. The effectiveness of a given dose or treatment regimen of the antagonist can be determined, for example, by assessing signs and symptoms in the patient using standard measures of efficacy.

In yet another embodiment, the subject is treated with the same anti-cancer agent, such as an anti-VEGF-A antibody at least twice. Thus, the initial and second antagonist exposures are preferably with the same antagonist, and more preferably all antagonist exposures are with the same antagonist, *i.e*., treatment for the first two exposures, and preferably all exposures, is with one type of anti-cancer agent, for example, an antagonist that binds to VEGF, such as an anti-VEGF antibody, *e.g*., all with bevacizumab.

In all the inventive methods set forth herein, the anti-cancer agent (such as an antibody that binds to VEGF) may be unconjugated, such as a naked antibody, or may be conjugated with another molecule for further effectiveness, such as, for example, to improve half-life.

One preferred anti-cancer agent herein is a chimeric, humanized, or human antibody, e.g., an anti-VEGF antibody, and preferably bevacizumab.

In another embodiment, the VEGF antagonist (e.g., an anti-VEGF antibody) is the only medicament administered to the subject.

In one embodiment, the antagonist is an anti-VEGF antibody that is administered at a dose of about 100 or 400 mg every 1, 2, 3, or 4 weeks or is administered a dose of about 1, 3, 5, 7.5, 10, 15, or 20 mg/kg every 1, 2, 3, or 4 weeks. The dose may be administered as a single dose or as multiple doses (e.g., 2 or 3 doses), such as infusions.

In yet another aspect, the invention provides, after the diagnosis step, a method of determining whether to continue administering an anti-cancer agent (e.g., an anti-VEGF antibody) to a subject diagnosed with cancer comprising measuring reduction in tumor size, using imaging techniques, such as radiography and/or MRI, after administration of the antagonist a first time, measuring reduction in tumor size in the subject, using imaging techniques such as radiography and/or MRI after administration of the antagonist a second time, comparing imaging findings in the subject at the first time and at the second time, and if the score is less at the second time than at the first time, continuing administration of the antagonist.

In a still further embodiment, a step is included in the treatment method to test the subject’s response to treatment after the administration step to determine that the level of response is effective to treat the angiogenic disorder. For example, a step is included to test the imaging (radiographic and/or MRI) score after administration and compare it to baseline imaging results obtained before administration to determine if treatment is effective by measuring if, and by how much, it has been changed. This test may be repeated at various scheduled or unscheduled time intervals after the administration to determine maintenance of any partial or complete remission.

In one embodiment of the invention, no other medicament than VEGF antagonist such as anti-VEGF antibody is administered to the subject to treat cancer.

In any of the methods herein, the anti-cancer agent may be administered in combination with an effective amount of an additional agent(s). Suitable additional agent(s) include, for example, an anti-lymphangiogenic agent, an anti-angiogenic agent, an anti-neoplastic agent, a chemotherapeutic agent, a growth inhibitory agent, a cytotoxic agent, or combinations thereof.

All these additional agent(s) may be used in combination with each other or by themselves with the first medicament, so that the expression "additional agent" as used herein does not mean it is the only medicament in addition to the VEGF antagonist. Thus, the additional agent need not be a single agent, but may constitute or comprise more than one such drug.

These additional agent(s) as set forth herein are generally used in the same dosages and with administration routes as used hereinbefore or about from 1 to 99% of the heretofore-employed dosages. If such additional agent(s) are used at all, preferably, they are used in lower amounts than if the first medicament were not present, especially in subsequent dosings beyond the initial dosing with the first medicament, so as to eliminate or reduce side effects caused thereby.

For the re-treatment methods described herein, where an additional agent(s) is administered in an effective amount with an antagonist exposure, it may be administered with any exposure, for example, only with one exposure, or with more than one exposure. In one embodiment, the additional agent(s) is administered with the initial exposure. In another embodiment, the additional agent(s) is administered with the initial and second exposures. In a still further embodiment, the additional agent(s) is administered with all exposures. It is preferred that after the initial exposure, such as of steroid, the amount of such additional agent(s) is reduced or eliminated so as to reduce the exposure of the subject to an agent with side effects such as prednisone, prednisolone, methylprednisolone, and cyclophosphamide.

The combined administration of an additional agent(s) includes coadministration (concurrent administration), using separate formulations or a single pharmaceutical formulation, and consecutive administration in either order, wherein preferably there is a time period while both (or all) active agents (medicaments) simultaneously exert their biological activities.

The anti-cancer therapy is administered by any suitable means, including parenteral, topical, subcutaneous, intraperitoneal, intrapulmonary, intranasal, and/or intralesional administration. Parenteral infusions include intramuscular, intravenous (i.v.), intraarterial, intraperitoneal, or subcutaneous administration. Intrathecal administration is also contemplated. In addition, the anti-cancer agent may suitably be administered by pulse infusion, *e.g*., with declining doses of the anti-cancer agent. Preferably, the dosing is given intravenously or subcutaneously, and more preferably by intravenous infusion(s).

If multiple exposures of anti-cancer agents are provided, each exposure may be provided using the same or a different administration means. In one embodiment, each exposure is by intravenous administration. In another embodiment, each exposure is given by subcutaneous administration. In yet another embodiment, the exposures are given by both intravenous and subcutaneous administration.

In one embodiment, the anti-cancer agent such as an anti-VEGF antibody is administered as a slow intravenous infusion rather than an intravenous push or bolus. For example, a steroid such as prednisolone or methylprednisolone (*e.g.*, about 80-120 mg i.v., more specifically about 100 mg i.v.) is administered about 30 minutes prior to any infusion of the anti-VEGF antibody. The anti-VEGF antibody is, for example, infused through a dedicated line.

For the initial dose of a multi-dose exposure to anti-VEGF antibody, or for the single dose if the exposure involves only one dose, such infusion is preferably commenced at a rate of about 50 mg/hour. This may be escalated, *e.g.*, at a rate of about 50 mg/hour increments every about 30 minutes to a maximum of about 400 mg/hour. However, if the subject is experiencing an infusion-related reaction, the infusion rate is preferably reduced, *e.g.*, to half the current rate, *e.g.,* from 100 mg/hour to 50 mg/hour. Preferably, the infusion of such dose of anti-VEGF antibody (*e.g.*, an about 1000-mg total dose) is completed at about 255 minutes (4 hours 15 min.). Optionally, the subjects receive a prophylactic treatment of acetaminophen/paracetamol (*e.g.*, about 1 g) and diphenhydramine HCl (*e.g.*, about 50 mg or equivalent dose of similar agent) by mouth about 30 to 60 minutes prior to the start of an infusion.

If more than one infusion (dose) of anti-VEGF antibody is given to achieve the total exposure, the second or subsequent anti-VEGF antibody infusions in this infusion embodiment are preferably commenced at a higher rate than the initial infusion, *e.g.,* at about 100 mg/hour. This rate may be escalated, *e.g.,* at a rate of about 100 mg/hour increments every about 30 minutes to a maximum of about 400 mg/hour. Subjects who experience an infusion-related reaction preferably have the infusion rate reduced to half that rate, *e.g.,* from 100 mg/hour to 50 mg/hour. Preferably, the infusion of such second or subsequent dose of anti-VEGF antibody (*e.g.*, an about 1000-mg total dose) is completed by about 195 minutes (3 hours 15 minutes).

In a preferred embodiment, the anti-cancer agent is an anti-VEGF antibody and is administered in a dose of about 0.4 to 4 grams, and more preferably the antibody is administered in a dose of about 0.4 to 1.3 grams at a frequency of one to four doses within a period of about one month. Still more preferably, the dose is about 500 mg to 1.2 grams, and in other embodiments is about 750 mg to 1.1 grams. In such aspects, the antagonist is preferably administered in two to three doses, and/or is administered within a period of about 2 to 3 weeks.

In one embodiment, the subject has never been previously administered any drug(s) to treat the cancer. In another embodiment, the subject or patient has been previously administered one or more medicaments(s) to treat the cancer. In a further embodiment, the subject or patient was not responsive to one or more of the medicaments that had been previously administered. Such drugs to which the subject may be non-responsive include, for example, anti-neoplastic agents, chemotherapeutic agents, cytotosic agents, and/or growth inhibitory agents. More particularly, the drugs to which the subject may be non-responsive include VEGF antagonists such as anti-VEGF antibodies. In a further aspect, such anti-cancer agentinclude an antibody or immunoadhesin, such that re-treatment is contemplated with one or more antibodies or immunoadhesins of this invention to which the subject was formerly non-responsive.

## IV. Treatment with the Anti-Cancer Agent

Once the patient population most responsive or sensitive to treatment with the VEGF antagonist has been identified, treatment with the VEGF antagonist, alone or in combination with other medicaments, results in a therapeutic benefit to the patient with cancer. For instance, such treatment may result in a reduction in tumor size or progression free survival. Moreover, treatment with the combination of an anti-cancer agent and at least one additional agent(s) preferably results in an additive, more preferably synergistic (or greater than additive) therapeutic benefit to the patient. Preferably, in this combination method the timing between at least one administration of the additional agent(s) and at least one administration of the anti-cancer agent is about one month or less, more preferably, about two weeks or less.

It will be appreciated by one of skill in the medical arts that the exact manner of administering to said patient a therapeutically effective amount of an anti-cancer agent following a diagnosis of a patient’s likely responsiveness to the anti-cancer agent will be at the discretion of the attending physician. The mode of administration, including dosage, combination with other agents, timing and frequency of administration, and the like, may be affected by the diagnosis of a patient’s likely responsiveness to such anti-cancer agent, as well as the patient’s condition and history. Thus, even patients diagnosed with a disorder who are predicted to be relatively insensitive to the anti-cancer agent may still benefit from treatment therewith, particularly in combination with other agents, including agents that may alter a patient’s responsiveness to the anti-cancer agent.

The composition comprising an anti-cancer agent will be formulated, dosed, and administered in a fashion consistent with good medical practice. Factors for consideration in this context include the particular type of disorder being treated, the particular mammal being treated, the clinical condition of the individual patient, the cause of the angiogenic disorder, the site of delivery of the agent, possible side-effects, the type of antagonist, the method of administration, the scheduling of administration, and other factors known to medical practitioners. The effective amount of the anti-cancer agent to be administered will be governed by such considerations.

As a general proposition, the effective amount of the anti-cancer agent administered parenterally per dose will be in the range of about 20 mg to about 5000 mg, by one or more dosages. Exemplary dosage regimens for antibodies such as anti-VEGF antibodies include 100 or 400 mg every 1, 2, 3, or 4 weeks or is administered a dose of about 1, 3, 5, 7.5, 10, 15, or 20 mg/kg every 1, 2, 3, or 4 weeks. The dose may be administered as a single dose or as multiple doses (e.g., 2 or 3 doses), such as infusions.

As noted above, however, these suggested amounts of anti-cancer agent are subject to a great deal of therapeutic discretion. The key factor in selecting an appropriate dose and scheduling is the result obtained, as indicated above. In some embodiments, the anti-cancer agentis administered as close to the first sign, diagnosis, appearance, or occurrence of the disorder as possible.

The anti-cancer agent is administered by any suitable means, including parenteral, topical, subcutaneous, intraperitoneal, intrapulmonary, intranasal, and/or intralesional administration. Parenteral infusions include intramuscular, intravenous, intraarterial, intraperitoneal, or subcutaneous administration. Intrathecal administration is also contemplated. In addition, the antagonist may suitably be administered by pulse infusion, *e.g.*, with declining doses of the antagonist. Most preferably, the dosing is given by intravenous injections.

One may administer an additional agent(s), as noted above, with the anti-cancer agents herein. The combined administration includes coadministration, using separate formulations or a single pharmaceutical formulation, and consecutive administration in either order, wherein preferably there is a time period while both (or all) active agents simultaneously exert their biological activities.

Aside from administration of anti-cancer agents to the patient by traditional routes as noted above, the present invention includes administration by gene therapy. Such administration of nucleic acids encoding the anti-cancer agent is encompassed by the expression "administering an effective amount of an anti-cancer agent". See, for example, WO 1996/07321 concerning the use of gene therapy to generate intracellular antibodies.

There are two major approaches to getting the nucleic acid (optionally contained in a vector) into the patient's cells; *in vivo* and *ex vivo.* For *in* vivo delivery the nucleic acid is injected directly into the patient, usually at the site where the antagonist is required. For *ex vivo* treatment, the patient's cells are removed, the nucleic acid is introduced into these isolated cells and the modified cells are administered to the patient either directly or, for example, encapsulated within porous membranes which are implanted into the patient (see, *e.g.* U.S. Patent Nos. 4,892,538 and 5,283,187). There are a variety of techniques available for introducing nucleic acids into viable cells. The techniques vary depending upon whether the nucleic acid is transferred into cultured cells *in vitro* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, *etc.* A commonly used vector for *ex vivo* delivery of the gene is a retrovirus.

The currently preferred *in vivo* nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). In some situations it is desirable to provide the nucleic acid source with an agent specific for the target cells, such as an antibody specific for a cell-surface membrane protein on the target cell, a ligand for a receptor on the target cell, *etc.* Where liposomes are employed, proteins that bind to a cell-surface membrane protein associated with endocytosis may be used for targeting and/or to facilitate uptake, *e.g.* capsid proteins or fragments thereof tropic for a particular cell type, antibodies for proteins that undergo internalization in cycling, and proteins that target intracellular localization and enhance intracellular half-life. The technique of receptor-mediated endocytosis is described, for example, by Wu et al., J. Biol. Chem. 262:4429-4432 (1987); and Wagner et al., PNAS USA 87:3410-3414 (1990). Gene-marking and gene-therapy protocols are described, for example, in Anderson et al., Science 256:808-813 (1992) and WO 1993/25673.

An anti-cancer agent may be combined in a pharmaceutical combination formulation, or dosing regimen as combination therapy, with at least one additional compound having anti-cancer properties. The at least one additional compound of the pharmaceutical combination formulation or dosing regimen preferably has complementary activities to the VEGF antagonist composition such that they do not adversely affect each other.

The at least one additional compound may be a chemotherapeutic agent, a cytotoxic agent, a cytokine, a growth inhibitory agent, an anti-hormonal agent, an anti-angiogenic agent, an anti-lymphangiogenic agent, and combinations thereof. Such molecules are suitably present in combination in amounts that are effective for the purpose intended. A pharmaceutical composition containing a VEGF antagonist (e.g., an anti-VEGF antibody) may also comprise a therapeutically effective amount of an anti-neoplastic agent, a chemotherapeutic agent a growth inhibitory agent, a cytotoxic agent, or combinations thereof.

In one aspect, the first compound is an anti-VEGF antibody and the at least one additional compound is a therapeutic antibody other than an anti-VEGF antibody. In one embodiment, the at least one additional compound is an antibody that binds a cancer cell surface marker. In one embodiment the at least one additional compound is an anti-HER2 antibody, trastuzumab (*e.g*., Herceptin®, Genentech, Inc., South San Francisco, CA). In one embodiment the at least one additional compound is an anti-HER2 antibody, pertuzumab (Omnitarg™, Genentech, Inc., South San Francisco, CA, see US6949245). In an embodiment, the at least one additional compound is an antibody (either a naked antibody or an ADC), and the additional antibody is a second, third, fourth, fifth, sixth antibody or more, such that a combination of such second, third, fourth, fifth, sixth, or more antibodies (either naked or as an ADC) is efficacious in treating an angiogenic disorder.

Other therapeutic regimens in accordance with this invention may include administration of a VEGF-antagonist anti-cancer agent and, including without limitation radiation therapy and/or bone marrow and peripheral blood transplants, and/or a cytotoxic agent, a chemotherapeutic agent, or a growth inhibitory agent. In one of such embodiments, a chemotherapeutic agent is an agent or a combination of agents such as, for example, cyclophosphamide, hydroxydaunorubicin, adriamycin, doxorubincin, vincristine (ONCOVIN™), prednisolone, CHOP, CVP, or COP, or immunotherapeutics such as anti-PSCA, anti-HER2 (*e.g*., HERCEPTIN®, OMNITARG™). The combination therapy may be administered as a simultaneous or sequential regimen. When administered sequentially, the combination may be administered in two or more administrations. The combined administration includes coadministration, using separate formulations or a single pharmaceutical formulation, and consecutive administration in either order, wherein preferably there is a time period while both (or all) active agents simultaneously exert their biological activities.

In one embodiment, treatment with an anti-VEGF antibody involves the combined administration of an anticancer agent identified herein, and one, two, or more chemotherapeutic agents or growth inhibitory agents, including coadministration of cocktails of different chemotherapeutic agents. Chemotherapeutic agents include taxanes (such as paclitaxel and docetaxel) and/or anthracycline antibiotics. Preparation and dosing schedules for such chemotherapeutic agents may be used according to manufacturer's instructions or as determined empirically by the skilled practitioner. Preparation and dosing schedules for such chemotherapy are also described in "Chemotherapy Service", (1992) Ed., M.C. Perry, Williams & Wilkins, Baltimore, Md.

Suitable dosages for any of the above coadministered agents are those presently used and may be lowered due to the combined action (synergy) of the newly identified agent and other chemotherapeutic agents or treatments.

The combination therapy may provide "synergy" and prove "synergistic", *i.e.* the effect achieved when the active ingredients used together is greater than the sum of the effects that results from using the compounds separately. A synergistic effect may be attained when the active ingredients are: (1) co-formulated and administered or delivered simultaneously in a combined, unit dosage formulation; (2) delivered by alternation or in parallel as separate formulations; or (3) by some other regimen. When delivered in alternation therapy, a synergistic effect may be attained when the compounds are administered or delivered sequentially, *e.g.* by different injections in separate syringes. In general, during alternation therapy, an effective dosage of each active ingredient is administered sequentially, *i.e.* serially, whereas in combination therapy, effective dosages of two or more active ingredients are administered together.

For the prevention or treatment of disease, the appropriate dosage of the additional therapeutic agent will depend on the type of disease to be treated, the type of antibody, the severity and course of the disease, whether the VEGF antagonist and additional agent are administered for preventive or therapeutic purposes, previous therapy, the patient's clinical history and response to the VEGF antagonist and additional agent, and the discretion of the attending physician. The VEGF antagonist and additional agent are suitably administered to the patient at one time or over a series of treatments. The VEGF antagonist is typically administered as set forth above. Depending on the type and severity of the disease, about 20 mg/m$^2$ to 600 mg/m$^2$ of the additional agent is an initial candidate dosage for administration to the patient, whether, for example, by one or more separate administrations, or by continuous infusion. One typical daily dosage might range from about or about 20 mg/m$^2$, 85 mg/m$^2$, 90 mg/m$^2$, 125 mg/m$^2$, 200 mg/m$^2$, 400 mg/m$^2$, 500 mg/m$^2$ or more, depending on the factors mentioned above. For repeated administrations over several days or longer, depending on the condition, the treatment is sustained until a desired suppression of disease symptoms occurs. Thus, one or more doses of about 20 mg/m$^2$, 85 mg/m$^2$, 90 mg/m$^2$, 125 mg/m$^2$, 200 mg/m$^2$, 400 mg/m$^2$, 500 mg/m$^2$, 600 mg/m$^2$ (or any combination thereof) may be administered to the patient. Such doses may be administered intermittently, *e.g.* every week or every two, three weeks, four, five, or six (*e.g.* such that the patient receives from about two to about twenty, *e.g.* about six doses of the additional agent). An initial higher loading dose, followed by one or more lower doses may be administered. However, other dosage regimens may be useful. The progress of this therapy is easily monitored by conventional techniques and assays.

### V. Pharmaceutical Formulations

Therapeutic formulations of the antagonists used in accordance with the present invention are prepared for storage by mixing the antagonist having the desired degree of purity with optional pharmaceutically acceptable carriers, excipients, or stabilizers in the form of lyophilized formulations or aqueous solutions. For general information concerning formulations, see, *e.g.,* Gilman et al., (eds.) (1990), The Pharmacological Bases of Therapeutics, 8th Ed., Pergamon Press; A. Gennaro (ed.), Remington's Pharmaceutical Sciences, 18th Edition, (1990), Mack Publishing Co., Eastori, Pennsylvania.; Avis et al., (eds.) (1993) Pharmaceutical Dosage Forms: Parenteral Medications Dekker, New York; Lieberman et al., (eds.) (1990) Pharmaceutical Dosage Forms: Tablets Dekker, New York; and Lieberman et al., (eds.) (1990), Pharmaceutical Dosage Forms: Disperse Systems Dekker, New York, Kenneth A. Walters (ed.) (2002) Dermatological and Transdermal Formulations (Drugs and the Pharmaceutical Sciences), Vol 119, Marcel Dekker.

Acceptable carriers, excipients, or stabilizers are non-toxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.* Zn-protein complexes); and/or non-ionic surfactants such as TWEEN™, PLURONICS™, or polyethylene glycol (PEG).

Exemplary anti-VEGF antibody formulations are described in U.S. Patent Nos. 6,884,879. In certain embodiments anti-VEGF antibodies are formulated at 25 mg/mL in single use vials. In certain embodiments, 100 mg of the anti-VEGF antibodies are formulated in 240 mg $\alpha,\alpha$-trehalose dihydrate, 23.2 mg sodium phosphate (monobasic, monohydrate), 4.8 mg sodium phosphate (dibasic anhydrous), 1.6 mg polysorbate 20, and water for injection, USP. In certain embodiments, 400 mg of the anti-VEGF antibodies are formulated in 960 mg $\alpha,\alpha$-trehalose dihydrate, 92.8 mg sodium phosphate (monobasic, monohydrate), 19.2 mg sodium phosphate (dibasic anhydrous), 6.4 mg polysorbate 20, and water for injection, USP.

Lyophilized formulations adapted for subcutaneous administration are described, for example, in US Pat No. 6,267,958 (Andya et al.). Such lyophilized formulations may be reconstituted with a suitable diluent to a high protein concentration and the reconstituted formulation may be administered subcutaneously to the mammal to be treated herein.

Crystallized forms of the antagonist are also contemplated. See, for example, US 2002/0136719A1 (Shenoy et al.).

The formulation herein may also contain more than one active compound (an additional agent(s) as noted above), preferably those with complementary activities that do not adversely affect each other. The type and effective amounts of such medicaments depend, for example, on the amount and type of VEGF antagonist present in the formulation, and clinical parameters of the subjects. The preferred such additional agent(s) are noted above.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semi-permeable matrices of solid hydrophobic polymers containing the antagonist, which matrices are in the form of shaped articles, *e.g.* films, or microcapsules. Examples of sustained-release matrices include polyesters, hydrogels (for example, poly(2-hydroxyethylmethacrylate), or poly(vinylalcohol)), polylactides (U.S. Pat. No. 3,773,919), copolymers of L-glutamic acid and $\gamma$ ethyl-L-glutamate, non-degradable ethylenevinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for *in vivo* administration must be sterile. This is readily accomplished by filtration through sterile filtration membranes.

## VI. Kits

For use in detection of $VEGF_{110}$, kits or articles of manufacture are also provided by the invention. Such kits can be used to determine if a subject with cancer will be effectively responsive to an anti-cancer agent therapy comprising a VEGF antagonist (including, e.g., an anti-VEGF antibody such as bevacizumab). These kits may comprise a carrier means being compartmentalized to receive in close confinement one or more container means such as vials, tubes, and the like, each of the container means comprising one of the separate elements to be used in the method. For example, one of the container means may comprise a compound that specifically binds $VEGF_{110}$.

Such kit will typically comprise the container described above and one or more other containers comprising materials desirable from a commercial and user standpoint, including buffers, diluents, filters, needles, syringes, and package inserts with instructions for use. A label may be present on the container to indicate that the composition is used for a specific application, and may also indicate directions for either *in vivo* or *in vitro* use, such as those described above.

The kits of the invention have a number of embodiments. A typical embodiment is a kit comprising a container, a label on said container, and a composition contained within said container, wherein the composition includes compound(s) that specifically bind(s) $VEGF_{110}$, and the label on said container indicates that the composition can be used to detect of $VEGF_{110}$, and wherein the kit includes instructions for using the compound(s) for detecting $VEGF_{110}$. The kit can further comprise a set of instructions and materials for preparing and using the compound(s).

Other optional components of the kit include one or more buffers (*e.g.,* dilution buffer, *etc.*), other reagents such as carrier (*e.g.,* dextran, albumin). Kits can also include instructions for interpreting the results obtained using the kit.

## EXAMPLES

The following examples are provided to illustrate, but not to limit the presently claimed invention.

### Example 1:

In the AVADO trial (BO17708) patients with untreated locally advanced, recurrent or metastatic HER-2 negative breast cancer were randomized to docetaxel 100 mg/$m^2$ plus bevacizumab 7.5 mg/kg every 3 weeks (n=248), bevacizumab 15 mg/kg (n=247) every three weeks or placebo (n=241) *see,* Miles, J. Clin. Oncol., 24 May 2010 (e-published)).

Blood plasma baseline samples were available from 396 patients in this trial.

An investigation of the status of biomarkers related to angiogenesis and tumorigenesis revealed that the expression levels of three biomarkers relative to control levels determined in the entire biomarker patient population correlated with an improved treatment parameter. In particular, patients exhibiting a higher expression level of VEGFA relative to control levels determined in the entire biomarker patient population, demonstrated a prolonged progression free survival in response to the addition of bevacizumab to docetaxel therapy. Patients exhibiting a higher expression level of VEGFR2 relative to control levels determined in the entire biomarker patient population, demonstrated a prolonged progression free survival in response to the addition of bevacizumab to docetaxel therapy. Also patients exhibiting higher combined expression level of VEGFA and VEGFR2 relative to control levels determined in the entire biomarker patient population, demonstrated a prolonged progression free survival in response to the addition of bevacizumab to docetaxel therapy. In addition, patients exhibiting higher combined expression level of VEGFA and PLGF relative to control levels determined in the entire patient population, demonstrated a prolonged prolonged progression free survival in response to the addition of bevacizumab to docetaxel therapy.

### Patients and Immunochemical Methods

A total of 736 patients participated in the BO17708 study, and blood plasma samples from 396 of the participants were available for biomarker analysis. The baseline characteristics of the 396 patients in the biomarker analysis and the remaining patients for which no biomarker analysis was possible are provided in Table 1A and Table 1B.

*Table 1A. Baseline characteristics*

| | biomarker evaluable N=396 | biomarker unevaluable N=334 |
|---|---|---|
| Sex | | |
| Female | 396 (100%) | 334 (100%) |
| n | 396 | 334 |
| | | |
| Randomized treatment | | |
| placebo + docetaxel | 129 (33%) | 109 (33%) |
| bevacizumab (7.5 mg/kg) + docetaxel | 129 (33%) | 118 (35%) |
| bevacizumab (15 mg/kg) + docetaxel | 138 (35%) | 107 (32%) |
| n | 396 | 334 |
| | | |
| Age (years) | | |
| Mean | 54.4 | 52.8 |

(continued)

| | biomarker evaluable N=396 | biomarker unevaluable N=334 |
|---|---|---|
| SD | 10.72 | 10.46 |
| SEM | 0.54 | 0.57 |
| Median | 55.0 | 53.0 |
| Min-Max | 29 - 83 | 26 - 77 |
| n | 396 | 334 |
| | | |
| Age Category (years) | | |
| <65 | 316 (80%) | 288 (86%) |
| >=65 | 80 (20%) | 46 (14%) |
| n | 396 | 334 |
| | | |
| Race | | |
| White | 375 (95%) | 234 (70%) |
| Black | 4(1%) | 3 (<1%) |
| Other | 17 (4%) | 97 (29%) |
| n | 396 | 334 |
| | | |
| Weight (kg) | | |
| Mean | 68.79 | 67.23 |
| SD | 14.097 | 14.185 |
| SEM | 0.708 | 0.780 |
| Median | 67.00 | 66.70 |
| Min-Max | 42.8 - 135.6 | 37.5 - 121.2 |
| n | 396 | 331 |
| | | |
| Height (cm) | | |
| Mean | 161.79 | 160.41 |
| SD | 7.158 | 7.351 |
| SEM | 0.360 | 0.402 |
| Median | 162.00 | 160.0 |
| Min-Max | 137.0 - 189.0 | 140.0 - 184.0 |
| n | 396 | 334 |
| | | |
| Smoking Status | | |
| Never smoked | 252 (64%) | 232 (70%) |
| Past smoker | 99 (25%) | 61 (18%) |
| Current smoker | 44 (11%) | 38 (11%) |

(continued)

| | biomarker evaluable N=396 | biomarker unevaluable N=334 |
|---|---|---|
| n | 395 | 331 |
| | | |
| Smoking - Pack Years | | |
| Mean | 24.06 | 33.63 |
| SD | 79.907 | 81.309 |
| SEM | 7.294 | 8.925 |
| Median | 10.00 | 15.00 |
| Min-Max | 0.3 - 860.0 | 0.5 - 720.0 |
| n | 120 | 83 |

*Table 1B. Baseline characteristics*

| | biomarker evaluable N=396 | biomarker unevaluable N=334 |
|---|---|---|
| Body Surface Area (sqm) | | |
| Mean | 1.726 | 1.698 |
| SD | 0.1725 | 0.1796 |
| SEM | 0.0087 | 0.0099 |
| Median | 1.710 | 1.700 |
| Min-Max | 1.35 - 2.42 | 1.29 - 2.33 |
| n | 396 | 331 |
| | | |
| Performance Status (ECOG) | | |
| 0 | 247 (63%) | 196 (60%) |
| 1 | 143 (37%) | 133 (40%) |
| 2 | 1 (<1%) | - |
| n | 391 | 329 |
| | | |
| LVEF | | |
| < = median (64) | 181 (35%) | 172 (55%) |
| > median (64) | 177 (49%) | 138 (45%) |
| n | 358 | 310 |
| | | |
| Disease Free Interval | | |
| < = 24 months | 138 (35%) | 118 (35%) |
| > 24 months | 255 (65%) | 216 (65%) |
| n | 393 | 334 |
| | | |

(continued)

| | biomarker evaluable N=396 | biomarker unevaluable N=334 |
|---|---|---|
| Hormone Receptor ER Status | | |
| Negative | 104 (26%) | 103 (31%) |
| Positive | 290 (73%) | 229 (69%) |
| Unknown | 2 (<1%) | 2 (<1%) |
| n | 396 | 334 |
| | | |
| ER/PgR Combined Status | | |
| Negative | 81 (21%) | 82 (25%) |
| Positive | 314 (79%) | 250 (75%) |
| n | 395 | 332 |
| Number of Metastatic Sites | | |
| < 3 | 209 (53%) | 175 (53%) |
| > = 3 | 183 (47%) | 156 (47%) |
| n | 392 | 331 |

**Blood Plasma Analysis**

Plasma samples were collected after randomization and before any study treatment was administered. All samples were obtained from patients that were thereafter treated with docetaxel 100mg/m2 plus either bevacitumab 7.5mg/kg every three weeks, bevacizumab 15mg/kg every three weeks or placebo until disease progression.

A total of 4.9 mLs of blood were drawn into a S-monovette® (EDTA) tube (or a citrated plasma tube for the 16 patients on anticoagulant therapy). They were mixed immediately thereafter by gentle invertion of the tube and were centrifuged within 30 minutes at approximately 1500g in centrifuge (room temperature for 10 minutes). Immediately hereafter, supernatant plasma was aliquoted in a clear polypropylene 5mL transfer tube. Thereafter, plasma was aliquoted into 2 plastic storage tubes (approximately 1.25 ml each). Samples were stored in an upright position at -70°C. In some cases, samples were stored at -20°C for up to one month and then transferred to -70°C.

Samples were used for measurement of levels of VEGFA, VEGF receptor-1 (VEGFR1), VEGFR2, PLGF and E-SELECTIN using an Immunological MultiParameter Chip Technology (IMPACT) from Roche Diagnostics GmbH.

**IMPACT Multiplex Assay Technology**

Roche Professional Diagnostics (Roche Diagnostics GmbH) is developing a multimarker platform under the working name IMPACT (Immunological MultiParameter Chip Technology). This technology was used for the measurement of the protein markers mentioned above in the "blood plasma analysis" section. The technology is based on a small polystyrene chip manufactured by procedures as disclosed in EP 0939319 and EP 1610129. The chip surface was coated with a streptavidin layer, onto which the biotinylated antibodies were then spotted for every assay. For each marker, spots of antibodies were loaded in a vertical line onto the chip. During the assay, the array was probed with specimen samples containing the specific analytes.

The plasma volume required per specimen for measuring all markers on one chip was 8 μL, which was applied together with 32 μL of an incubation buffer (50 mM HEPES pH 7.2, 150 mM NaCl, 0.1% Thesit, 0.5% bovine serum albumin and 0.1% Oxypyrion as a preservative agent). After incubation for 12 minutes and washing of the chip using a washing buffer (5 mM Tris pH 7.9, 0.01% Thesit and 0.001% Oxypyrion) the digoxigenylated monoclonal antibody mix was added (40 1μL of incubation buffer including a mix of the analyte-specific antibodies labeled with Digoxigenin) and was incubated for an additional 6 minutes to bind onto the captured analytes. The second antibody was finally detected with 40 μL of a reagent buffer (62.5 mM TAPS pH 8.7, 1.25 M NaCl, 0.5% bovine serum albumin, 0.063% Tween 20 and 0.1% Oxypyrion) including an anti-digoxigenin antibody conjugate coupled with fluorescent latex. Using this label, 10 individual binding events in a single spot could be detected, resulting in very high sensitivity down to the fmol/L concentration. Chips were transported into the detection unit, and a charge coupled device (CCD) camera generated an image that was transformed into signal intensities using dedicated software. Individual spots were automatically located at predefined positions and quantified by image analysis. For each marker, lines of 10-12 spots were loaded on the chips, and a minimum of 5 spots was required to determine the mean concentration of samples. The advantages of the technology are the ability of multiplexing up to 10 parameters in a sandwich or competitive format. The calibrators and patient samples were measured in duplicate. One run was designed to contain a total of 100 determinations, including 2 multi-controls as a run control. Since some of the selected analytes react with each other (i.e VEGFA and PLGF with VEGFR1 or VEGRF2 or VEGFA forms heterodimers with PLGF), the 5 analytes were divided on three different chips as follows:

Chip 1: VEGFA
Chip 2: VEGFR1, VEGFR2, E-Selectin
Chip 3: PLGF

The following antibodies were used for the different assays:

| Analyte | Capture antibody | Manufacturer | Detection antibody | Manufacturer |
|---|---|---|---|---|
| VEGFA | <VEGF-A>M-3C5 | Bender RELIATech | <VEGF>M-26503 | R&D Systems |
| VEGFR1 | <VEGF-R1>M-49560 | Roche Diagnostics | <VEGF-R1>M-49543 | Roche Diagnostics |
| VEGFR2 | <VEGF-R2>M-89115 | R&D Systems | <VEGF-R2>M-89109 | R&D Systems |
| E-Selectin | <E-Selectin>M-BBIG-E5 | R&D Systems | <E-Selectin>M-5D11 | R&D Systems |
| PLGF | <PLGF>M-2D6D5 | Roche Diagnostics | <PLGF>M-6A11D2 | Roche Diagnostics |

**Statistical Analysis**

Sample median was used to dichotomize biomarker values as low (below median) or high (at or above median).

Hazard Ratio of treatment effect in sub-group of patients with high or low biomarker levels were estimated with proportional hazard cox regression analysis.

In addition, proportional hazard cox regressions was used to evaluate the association between biomarker level and treatment effect. The model included the following covariates: trial treatment, biomarker level, binary stratification factors (ER/PgR status, measurable disease at baseline, prior adjuvant taxane therapy), interaction term of treatment by biomarker level. Wald test for the interaction term was used to determine the association between biomarker level and treatment effect. P-value below 0.05 was considered significant.

**Results**

<u>Blood Plasma Markers</u>

The baseline descriptive statistics of the biomarkers are presented in Table 2.

*Table 2: Descriptive Statistics of Biomaker Values (Baseline)*

| | ***VEGFA*** *(pg/mL)* | ***VEGER2*** *(ng/mL)* | ***PLGF*** *(pg/mL)* |
|---|---|---|---|
| ***min*** | 20.0 | 0.1 | 5.8 |
| ***qu 25%*** | 64.5 | 9.1 | 17.04 |
| ***median*** | 125.0 | 11.0 | 21.31 |
| ***qu 75%*** | 240.5 | 13.4 | 27.02 |
| ***max*** | 3831.1 | 72.4 | 282.10 |
| ***mean*** | 216.5 | 11.6 | 24.58 |
| ***sd*** | 322.63 | 4.58 | 20.38 |

Table 3 presents the results of the analysis of the association of VEGFA or VEGFR2 with treatment effect on progression free survival.

*Table 3:*

| | Low dose | | High dose | |
|---|---|---|---|---|
| | HR (95%CI) | Interaction p-value | HR (95%CI) | Interaction p-value |
| VEGFA low | 0.96 (0.62 - 1.48) | P=0.0136 | 0.86 (0.56 - 1.32) | P=0.0808 |
| VEGFA high | 0.52 (0.33 - 0.81) | | 0.49 (0.31 - 0.76) | |
| VEGFR2 low | 1.10 (0.73 - 1.67) | P=0.0342 | 0.75 (0.49 - 1.16) | P=0.2545 |
| VEGFR2 high | 0.46 (0.28 - 0.74) | | 0.54 (0.35 - 0.85) | |

In this analysis, for VEGFA, Low VEGFA (<125 pg/ml) and High VEGFA (≥125 pg/ml), and for VEGFR2, Low VEGFR2 (<11 ng/ml) and High VEGFR2 (≥11 ng/ml) were used.

These results show that the Hazard Ratio for treatment effect is significantly better in the subset of patients with high VEGFA compared to patients with low VEGFA. These results also show that the Hazard Ratio for treatment effect is significantly better in the subset of patients with high VEGFR2 compared to patients with low VEGFR2. The same trend is observed when comparing low and high dose bevacizumab to placebo, the statistical evidence of difference between high and low biomarker sub-group is stronger in the patients treated with low dose bevacizumab. Therefore, VEGFA and VEGFR2 are each independent predictive biomarkers for bevacizumab treatment effect on Progression Free Survival.

Table 4 presents the analysis of biomarker combinations association with treatment effect on progression free survival for low dose (7.5 mg/kg every 3 weeks) bevacizumab and for high dose (15 mg/kg every 3 weeks) bevacizumab. For this analysis

$$\text{Formula 1: norm(VEGFA)+1.3*norm(VEGFR2)}$$

$$\text{Equivalent formula: } 0.71*\log2(\text{VEGFA})+3.16*\log2(\text{VEGFR2})-15.6$$

and

$$\text{Formula 2: } 0.25*\text{norm(VEGFA)}+0.21*\text{norm(PLGF)}$$

$$\text{Equivalent formula: } 0.18*\log2(\text{VEGFA})+0.42*\log2(\text{PLGF})-3.1$$

Where we use log2 transformation and

$$x_i \rightarrow norm(x_i) = \frac{\log 2(x_i) - median(\log 2(x))}{mad(\log 2(x))}$$

Where mad is the median absolute deviation adjusted by a factor of 1.4826.

*Table 4: Association with treatment effect on Progression Free Survival (bimarker analysis) for low dose (7.5 mg/kg every 3 weeks) bevacizumab and for high dose (15 mg/kg every 3 weeks) bevacizumab*

| | **Low Dose (7.5 mg/kg) versus Placebo** | | **High Dose (15 mg/kg) versus Placebo** | |
|---|---|---|---|---|
| | HR (95%CI) | Interaction p-value | HR (95%CI) | Interaction p-value |
| VEGFA & VEGFR2 low | 1.1 (0.72,1.69) | 0.0077 | 0.84 (0.54, 1.3) | 0.0580 |
| VEGFA & VEGFR2 high | 0.474 (0.3,0.75) | | 0.483 (0.31,0.76) | |
| VEGFA & PLGF low | 1.01 (0.65,1.58) | 0.037 | 0.845 (0.53,1.34) | 0.12 |
| VEGFA & PLGF high | 0.518 (0.33,0.81) | | 0.507 (0.33,0.78) | |

In this analysis, a high combined expression level of VEGFA and VEGFR2 is Formula 1 $\geq$ -0.132 and a low combined expression level of VEGFA and VEGFR2 is Formula 1 < -0.132, and a high combined expression level of VEGFA and PLGF is Formula 2 $\geq$ -0.006 and a low combined expression level of VEGFA and PLGF is Formula 2 < -0.006.

These results show that the Hazard Ratio for treatment effect is significantly better in the subset of patients with high VEGFA & VEGFR2 combination compared to patients with low VEGFA & VEGFR2 combination. These results also show that the Hazard Ratio for treatment effect is significantly better in the subset of patients with high VEGA & PLGF combination compared to patients with low VEGFA & PLGF combination. The same trend is observed when comparing low and high dose bevacizumab to placebo, the statistical evidence of difference between high and low biomarker sub-group is stronger in patients treated with low dose bevacizumab. Therefore, VEGFA & VEGFR2 combination and VEGFA & PLGF combination are each independent predictive biomarkers for bevacizumab treatment effect on Progression Free Survival.

The predictive value of VEGF-A in the bevacizumab 15 mg/kg arm was explored further by subdividing the cohort into quartiles according to VEGF-A levels. The 95% confidence intervals for all quartiles overlapped. In the first quartile (< 64 pg/ml), a very limited treatment effect was observed (hazard ratio 0.86). In the highest quartile (>240 pg/ml), the hazard ratio for PFS was 0.39 (95% CI:0.19-0.77) and the difference in median PFS was more pronounced than in the other groups. Overall, the point estimates of the quartiles show a consistent improvement in the hazard ratio with increasing VEGF-A levels. These results are shown in Table 5 below.

*Table 5: PFS According to VEGF-A Quartile*

| Median PFS Months | | | | | |
|---|---|---|---|---|---|
| VEGF-A Quartile | No. of patients | No. of Events | Bevacizumab 15 mg/kg + docetaxel | Placebo + docetaxel | HR (95% CI) |
| 1$^{st}$ | 71 | 43 | 8.6 | 8.3 | 0.86 (0.47-1.59) |
| 2$^{nd}$ | 68 | 43 | 8.5 | 7.2 | 0.75 (0.42-1.44) |
| 3$^{rd}$ | 65 | 43 | 8.4 | 6.5 | 0.55 (0.30-1.01) |
| 4$^{th}$ | 61 | 36 | 10.3 | 7.5 | 0.39 (0.19-0.77) |

**Example 2: Detection of shorter isoforms of VEGF-A using the IMPACT Assay**

This example demonstrates that, based on the antibodies used for detection of VEGF-A on the IMPACT platform, the shorter isoforms of VEGF-A are preferentially measured as compared to the longer isoforms of VEGF-A.

The assay was performed as described above under the section relating to the IMPACT technology using the antibodies listed in the table before the "statistical analysis" section.

Four different VEGF-A forms, i.e. $VEGF_{111}$, $VEGF_{121}$, $VEGF_{165}$ and $VEGF_{189}$ were available and used in the analysis. $VEGF_{111}$, $VEGF_{121,}$ and $VEGF_{165}$ was purchased from R&D Systems, Minneapolis, USA and $VEGF_{189}$ was obtained from Reliatech, Wolfenbüttel, Germany. As shown in **Figure 11** the shorter isoforms having 111 or 121 amino acids, respectively, are detected better as compared to the longer isoforms with 165 and 189 amino acids, respectively. The biologically interesting plasmin cleavage product $VEGF_{110}$ was not available for testing at this point in time, but is has to be expected that detection of this isoform will be comparable to what is seen for the VEGF-molecule with 111 amino acids.

Without being bound by theory, it is assumed that the preferential binding of short VEGF isoforms like $VEGF_{110}$ and $VEGF_{121}$, respectively, of this assay, could explain why in the studies described herein a statistically significant predictive value was observed, while previous measurements of VEGF-A had lead to conflicting results in that respect.

**Example 3: Detection of short VEGF isoforms using the Elecsys®**

**Analyzer**

This example describes experiments demonstrating that an assay using the Elecsys® Analyzer can be used to detect short VEGF isoforms in human plasma.

The VEGF-A assay was transferred from IMPACT to the automated in-vitro diagnostics system Elecsys® (Roche Diagnostics GmbH, Mannheim). The same capture antibody as in the IMPACT Assay, <hVEGF-A>-m3C5 (Reliatech, Wolfenbüttel) was used, while the capture antibody <hVEGF-A>-m25603 (R&D Systems, Minneapolis) used on the IMPACT system was replaced by <hVEGF-A>-mA4.6.1 (Genentech, South San Francisco).

The immunoassays running on the automated Elecsys® system are immuno assays using electrochemiluminescense (ECLIA) as the signal generating technology. In the present sandwich assay the biotinylated capture antibody binds to streptavidin coated, magnetic microparticles and the ruthenylated detection antibody allows for signal generation. 75 µl of biotinylated <VEGF-A>-m3C5 at 1.5 µg/ml and 75 µl of ruthenylated <VEGF-A>M-A.4.6.1 at 2 µg/ml both in reaction buffer (50 mM Tris (pH 7.4), 2 m M EDTA, 0.1 % thesit, 0.2 % bovine IgG, 1.0 % bovine serum albumin) were incubated for 9 minutes with 20 µl of sample. 30 µl of a microparticle suspension was added after the first 9 minutes of incubation and the whole mixture then incubated for an additional 9 minutes. During these incubation steps an antibody analyte antibody sandwich is formed that is bound to the microparticles. Finally the microparticles were transferred to the detection chamber of the Elecsys system for signal generation and readout.

The cleavage product/isoform preference of the Elecsys® VEGF-A assay was assessed with purified recombinant proteins: $VEGF_{110}$ (produced by plasmin cleavage at Genentech, South San Francisco), VEGF 121 and VEGF 165

(both supplied by R&D Systems, Minneapolis). The preferential binding of short VEGF isoforms that had been seen with the IMPACT® Assay was confirmed in the Elecsys assay. As shown in **Figure 12,** in the Elecsys® assay the isoforms VEGF 121 and the plasmin cleavage product $VEGF_{110}$, respectively, both were detected with an approximately 5-fold higher sensitivity than VEGF 165.

**Examples 4: A phase III randomized trial in first-line unresectable, locally advanced, metastatic gastric cancer of bevacizumab in combination with chemotherapies**

This example concerns analysis of results obtained from patients with first-line metastatic gastric cancer treated in the AVAGAST clinical trial. The primary aim of the study was to determine the clinical benefit of adding bevacizumab to chemotherapy for treating gastric cancer, as measured by overall survival (OS). Secondary endpoints included progression-free survival (PFS), overall response rate and duration of response. The chemotherapy used in this trial was a combination of capecitabine or 5-flurouracil (5-FU) and cisplatin.

***Study Design***

774 patients were enrolled in the AVAGAST trial and were randomized 1:1 to the following two arms:

Arm A (387):

- Oral capecitabine 1,000 mg/m2 twice daily for 2 weeks followed by one week rest, every 3 weeks until disease progression or unmanageable toxicity or, for patients not deemed appropriate to take oral capecitabine (because of, e.g., difficulty swallowing, malabsorption or other conditions that could affect intake of oral capecitabine medication), 5-fluorouracil may be administered instead, at a dose of 800 mg/m2/day as a continuous iv infusion over 5 days (days 1 to 5 of each cycle) every 3 weeks; and
- cisplatin 80 mg/m2 as a 2 hr iv infusion with hyperhydration and pre-medication (steroids and anti-emetics), every 3 weeks for a maximum of 6 cycles, until disease progression or unmanageable toxicity.

Arm B (387):

- Oral capecitabine 1,000 mg/m2 twice daily for 2 weeks followed by one week rest, every 3 weeks until disease progression or unmanageable toxicity or, for patients not deemed appropriate to take oral capecitabine (because of, e.g., difficulty swallowing, malabsorption or other conditions that could affect intake of oral capecitabine medication), 5-fluorouracil may be administered instead, at a dose of 800 mg/m2/day as a continuous iv infusion over 5 days (days 1 to 5 of each cycle) every 3 weeks; and
- cisplatin 80 mg/m2 as a 2 hr iv infusion with hyperhydration and pre-medication (steroids and anti-emetics), every 3 weeks until disease progression or unmanageable toxicity for a maximum of 6 cycles; and
- bevacizumab (7.5 mg/kg) every 3 weeks until disease progression or unmanageable toxicity.

Treatment arms were balanced for stratification variables with the exception of advanced disease (2% vs 5%). Approx 95% of patients were metastatic, two-thirds male, 49% from Asia/Pacific, 32% from Europe and 19% from the Americas.

Bevacizumab (AVASTIN®) was supplied as a clear to slightly opalescent, sterile liquid ready for parenteral administration in two vial sizes: each 100 mg (25 mg/ml - 4 ml fill) glass vial contained bevacizumab with phosphate, trehalose, polysorbate 20 and Sterile Water for Injection, USP and each 400 mg (25 mg/ml - 16 ml fill) glass vial contained bevacizumab with phosphate, trehalose, polysorbate 20, and Sterile Water for Injection, USP. AVASTIN® was administered by withdrawing the necessary amount for a dose of 5 mg/kg and diluted in a total volume of 100 ml of 0.9% Sodium Chloride Injection, USP before intravenous administration.

**Methods**

Eligible Subjects/Patients had the following key eligibility criteria: Age > 18 years, ECOG 0, 1 or 2 (ECOG Performance Status Scale). All subjects had histologically confirmed adenocarcinoma of the stomach or gastro-oesophageal junction with inoperable, locally advanced or metastatic disease, not amenable to curative therapy. Subjects may have had either measurable or non-measurable but evaluable disease (per the Response Evaluation Criteria in Solid Tumors (RECIST)). Subjects not receiving anticoagulant medication had an INR less than or equal to 1.5 and a PTT less than or equal to 1.5 x ULN within 7 days prior to randomization.

Exclusion criteria included the following: previous chemotherapy for locally advanced or metastatic gastric cancer (patients may have received prior neoadjuvant or adjuvant chemotherapy as long as it was completed at least 6 months prior to randomisation); previous platinum or anti-angiogenic therapy (i.e. anti-VEGF or VEGFR tyrosine kinase inhibitor etc); patients with locally advanced disease who were candidates for curative therapy (including operation and/or chemotherapy and/or radiotherapy); radiotherapy within 28 days of randomisation; major surgical procedure, open biopsy or significant traumatic injury within 28 days prior to randomisation, or anticipation of the need for major surgery during the course of the study treatment (planned elective surgery); minor surgical procedures within 2 days prior to randomisation; evidence of CNS metastasis at baseline; history or evidence upon physical/neurological examination of CNS disease unrelated to cancer unless adequately treated with standard medical therapy, e.g. uncontrolled seizures; inadequate bone marrow function, liver function or renal function; uncontrolled hypertension or clinically significant (i.e. active) cardiovascular disease; active infection requiring intravenous antibiotics at randomisation; history or evidence of inherited bleeding diathesis or coagulopathy with the risk of bleeding; serious or non-healing wound, peptic ulcer, or (incompletely healed) bone fracture; active gastrointestinal bleeding; history of abdominal fistula, gastrointestinal perforation, or intra-abdominal abscess within 6 months of randomisation; neuropathy (e.g. impairment of hearing and balance) $\geq$ grade II according to CTCAE v3.0; chronic daily treatment with aspirin or clopidogrel; chronic daily treatment with oral corticosteroids (inhaled steroids and short courses of oral steroids for anti-emesis or as an appetite stimulant were allowed); known dihydropyrimidine dehydrogenase (DPD) deficiency; and known acute or chronic-active infection with HBV or HCV.

The primary endpoint of the study was overall survival (OS), defined as the time from randomization until death from any cause. Time-to-event data are compared between treatment arms using a stratified log-rank test. The Kaplan-Meier method was used to estimate duration of time-to-event data. The 95% confidence intervals for median time-to-event were computed using the Brookmeyer-Crowley method. The HR for time-to-event data was estimated using a stratified Cox regression model.

The secondary endpoints included progression free survival (PFS), objective response rate (RR), duration of response, and safety. Progression free survival (PFS) is defined as the time from randomization to disease progression or to death, based on investigator assessment. Kaplan-Meier methodology was used to estimate median PFS for each treatment arm. In certain embodiments, the hazard ratio for PFS was estimated using a stratified Cox regression model with the same stratification factors used in the stratified log-rank test. Analyses of PFS in each cohort was performed at the two-sided $\alpha$=0.05 level. Time-to-event data were compared between treatment arms using a stratified log-rank test. The Kaplan-Meier method was used to estimate duration of time-to-event data. The 95% confidence intervals for median time-to-event was computed using the Brookmeyer-Crowley method. The HR for time-to-event data was estimated using a stratified Cox regression model. RR is defined as the percentage of patients who achieved a complete or partial response confirmed ≥28 days after initial documentation of response. RR in patients with measurable disease at baseline was compared using the stratified Mantel-Haenszel $\chi 2$ test. Randomization stratification factors were included in all stratified analyses.

Blood plasma samples were collected from patients participating in a randomized phase-III study comparing the results of adding bevacizumab to capecitabine/cisplatin therapy for the treatment of inoperable locally advanced/metastatic gastric/gastro-oesophageal adenocarcinoma (the BO20904 study, *see,* Kang et al, J. Clin. Oncol. 2010; 28 (18S): LBA4007).

An investigation of the status of biomarkers related to angiogenesis and tumorigenesis revealed that the expression levels of one plasma biomarker relative to control levels determined in the entire biomarker patient population correlated with an improved treatment parameter. In particular, patients exhibiting a higher expression level of VEGFA relative to control levels determined in the entire biomarker patient population, demonstrated a prolonged overall survival and a prolonged progression free survival in response to the addition of bevacizumab to capecitabine/cisplatin therapy.

**Patients, samples and Immunochemical Methods**

A total of 774 patients participated in the BO20904 study, and blood plasma sampling for analysis of plasma VEGF-A was pre-specified in the protocol.

All samples were obtained from patients treated with capecitabine/cisplatin plus bevacizumab or placebo. A total of 4.9 ml was collected in a 4.9 mL EDTA S-Monovette® blood collection tube.

Within 30-60 minutes of blood collection, blood tubes were placed into the centrifuge and spun 1500 g at 4°C for 15 minutes, until cells and plasma were separated. Immediately after centrifugation, the plasma was carefully transferred.into a propylene transfer tube using a plastic pipette, being careful not to aspirate the interface containing the platelets at the bottom of the tube. The plasma was then aliquotted equally into 2 storage tubes (half volume each approximately 1.25 mL) using a pipette.

Once the plasma was separated, the samples were stored in an upright position at -70°C. Samples that could only be stored at -20°C, were shipped to Roche central sample office (CSO) within one month after the blood draw. All samples were analysed at Roche Diagnostics GmbH, Penzberg, Germany.

All samples were thawed and distributed in 40 µl aliquots into 384-well REMP micro tube plates in batches of 72. The tubes were sealed with an aluminum septum and stored in freezers set to maintain a temperature of -85 to -55°C until analysis. The samples were analyzed for VEGFA concentrations.

Plasma samples from 712 of the participants were available for biomarker analysis. The baseline characteristics of the 712 patients in the biomarker analysis are provided in Table 6.

*Table 6. Vaseline characteristics: biomarker population (n=712)*

| | Pl+CapC<br>N = 357 | Bv7.5+CapC<br>N = 355 |
|---|---|---|
| Sex | | |
| MALE | 239 (67%) | 238 (67%) |
| FEMALE | 118 (33%) | 117 (33%) |
| n | 357 | 355 |
| Ethnicity | | |
| CAUCASIAN | 142 (40%) | 144 (41%) |
| BLACK | 8 (2%) | 5 (1%) |
| ORIENTAL | 188 (53%) | 184 (52%) |
| OTHER | 19 (5%) | 22 (6%) |
| n | 357 | 355 |
| Age (years) | | |
| Mean | 57.6 | 56.5 |
| SD | 11.29 | 11.50 |
| SEM | 0.60 | 0.61 |
| Median | 59.0 | 58.0 |
| Min-Max | 22 - 82 | 22 - 81 |
| n | 357 | 355 |

(continued)

| | Pl+CapC N = 357 | Bv7.5+CapC N = 355 |
|---|---|---|
| Weight in kg | | |
| Mean | 60.75 | 62.46 |
| SD | 13.328 | 13.805 |
| SEM | 0.706 | 0.734 |
| Median | 59.00 | 60.90 |
| Min-Max | 36.0 - 145.4 | 35.7 - 149.5 |
| n | 356 | 354 |
| Height in cm | | |
| Mean | 164.5 | 165.3 |
| SD | 9.32 | 8.79 |
| SEM | 0.49 | 0.47 |
| Median | 164.0 | 165.0 |
| Min-Max | 138 - 192 | 140 - 188 |
| n | 356 | 354 |
| Age Category <65 ; >=65 | | |
| <65 | 253 (71%) | 256 (72%) |
| >=65 | 104 (29%) | 99 (28%) |
| n | 357 | 355 |
| Age Category <40;40-65;>=65 | | |
| <40 | 26 (7%) | 33 (9%) |
| 40-65 | 227 (64%) | 223 (63%) |
| >=65 | 104 (29%) | 99 (28%) |
| n | 357 | 355 |
| ECOG Category at Baseline | | |
| 0 | 157 (44%) | 164 (46%) |
| >=1 | 200 (56%) | 191 (54%) |
| n | 357 | 355 |

n represents number of patients contributing to summary statistics.
Percentages are based on n (number of valid values). Percentages not calculated if n < 10.

**Blood Plasma Analysis**

Plasma samples were collected after randomization and before any study treatment was given to the patients. VEGFA was measured using the multiplex IMPACT ELISA assay described in Example 1.

**Statistical Analysis**

Sample median was used to dichotomize biomarker values as low (below median) or high (above median).

Hazard Ratio of treatment effect in sub-group of patients with high or low biomarker levels were estimated with proportional hazard COX regression analysis.

In addition, proportional hazard COX regressions was used to evaluate the association between biomarker level and treatment effect. The model included the following covariates: trial treatment, biomarker level, interaction term of treatment by biomarker level. Wald test for the interaction term was used to determined the association between biomarker level and treatment effect. P-value below 0.05 was considered significant.

Values reported as below the limit of quantification (BLQ) or above the limit of quantification (ALQ) were imputed at the lower limit of quantification (LLQ) and at the upper limit of quantification (ULQ) values. A logarithmic transformation of the concentration levels was used in the analysis. If a cut-off was needed for the analysis, the sample median was used.

**Results**

Blood Plasma Markers

The baseline descriptive statistics of the biomarkers are presented in Table 7.

*Table 7: Descriptive Statistics of Biomarker Values (Baseline)*

| **Biomarker** | | **pl+CapC** | **Bv7.5+CapC** | **All Patients** |
|---|---|---|---|---|
| Plasma VEGF at BL (pg/mL) | n | 357 | 355 | 712 |
| | Geometric Mean | 114 | 115 | 114 |
| | Arithmetic Mean | 170 | 181 | 175 |
| | SE | 9.3 | 12.0 | 7.6 |
| | SD | 175.6 | 226.6 | 202.6 |
| | Min - Max | 20 - 1747 | 20 - 1868 | 20 - 1868 |
| | $25^{th}$ percentile | 59 | 61 | 59 |
| | Median | 119 | 108 | 111 |
| | $75^{th}$ percentile | 220 | 203 | 208 |
| | CV (%) | 103 | 126 | 116 |
| | Number of Min/BLQ | 20 | 12 | 32 |
| | Number of Max/ALQ | 1 | 1 | 1 |

Table 8 presents the univariate analysis of the association of the selected biomarkers with treatment effect on overall survival.

*Table 8: Association with treatment effect on Overall Survival - (uni-variate analysis)*

| | | HR (95% CI) | P-value for interaction |
|---|---|---|---|
| Overall | VEGFA low | 1.01 [0.77;1.31] | P=0.07 |
| | VEGFA high | 0.72 [0.57;0.93] | |
| Non-Asia | VEGFA low | 1.01 [0.68;1.51] | p=0.04 |
| | VEGFA high | 0.59 [0.43;0.82] | |
| Asia | VEGFA low | 0.99 [0.70;1.40] | P=0.76 |
| | VEGFA high | 0.92 [0.63;1.34] | |

In this analysis, for VEGFA, Low VEGFA $\leq$ 111 pg/ml and High VEGFA > 111 $\mu$g/ml was used.

For VEGFA the cut-off level was determined as sample data median value, such that 50% of patients have high expression and 50% of patients have low expression, as per pre-determined analysis plan.

This result table shows that the Hazard Ratio for treatment effect is significantly better in the subset of patients with high VEGFA compared to patients with low VEGFA. Therefore, VEGFA is an independent predictive biomarkers for Bevacizumab treatment effect on overall survival.

Table 9 presents the univariate analysis of the association of the selected biomarkers with treatment effect on progression free survival.

*Table 9: Association with treatment effect on Progression Free Survival (univariate analysis)*

| | | HR (95% CI) | P-value for interaction |
|---|---|---|---|
| overall | VEGFA low | 0.86 [0.67;1.10] | P=0.11 |
| | VEGFA high | 0.66 [0.52;0.85] | |
| Non-Asia | VEGFA low | 0.85 [0.57;1.26] | P=0.06 |
| | VEGFA high | 0.54 [0.39;0.76] | |
| Asia | VEGFA low | 0.86 [0.63;1.18] | P=0.99 |
| | VEGFA high | 0.87 [0.61;1.25] | |

In this analysis, for VEGFA, Low VEGFA ≤ 111pg/ml and High VEGFA > 111 μg/ml was used.

For VEGFA the cut-off level was determined as sample data median value, such that 50% of patients have high expression and 50% of patients have low expression, as per pre-determined analysis plan.

This result table shows that the Hazard Ratio for treatment effect is significantly better in the subset of patients with high VEGFA compared to patients with low VEGFA. Therefore, VEGFA is an independent predictive biomarker for bevacizumab treatment effect on progression free survival.

**Example 5: A phase III randomized trial in metastatic pancreatic cancer of bevacizumab in combination with chemotherapies**

Patients with metastatic pancreatic adenocarcinoma were randomized to gemicitamibe-erlotinib plus bevacizumab (n=306) or placebo (n=301).

Blood plasma samples were collected from patients participating in a randomized phase-III study comparing the results of adding bevacizumab to gemicitamibe-erlotinib therapy for the treatment of metastatic pancreatic cancer (the BO17706 study, *see,* Van Cutsem, J. Clin. Oncol. 2009 27:2231-2237). Patients with metastatic pancreatic adenocarcinoma were randomized to gemicitamibe-erlotinib plus bevacizumab (n=306) or placebo (n=301). P Patients with metastatic pancreatic adenocarcinoma were randomly assigned to receive gemcitabine (1,000 mg/$m^2$/week), erlotinib (100 mg/day), and bevacizumab (5 mg/kg every 2 weeks) or gemcitabine, erlotinib, and placebo.

An investigation of the status of biomarkers related to angiogenesis and tumorigenesis revealed that the expression levels of three biomarkers relative to control levels determined in the entire biomarker patient population correlated with an improved treatment parameter. In particular, patients exhibiting a higher expression level of VEGFA relative to control levels determined in the entire biomarker patient population, demonstrated a prolonged overall survival and a prolonged progression free survival in response to the addition of bevacizumab to gemicitamibe-erlotinib therapy. Patients exhibiting a higher expression level of VEGFR2 relative to control levels determined in the entire biomarker patient population, demonstrated a prolonged overall survival in response to the addition of bevacizumab to gemicitamibe-erlotinib therapy. Patient exhibiting a higher expression level of PLGF relative to control levels determined in the entire biomarker patient population, demonstrated a prolonged progression free survival in response to the addition of bevacizumab to gemicitamibe-erlotinib therapy. Also patients exhibiting higher combined expression level of VEGFA and VEGFR2 relative to control levels determined in the entire biomarker patient population, demonstrated a prolonged overall survival and a prolonged progression free survival in response to the addition of bevacizumab to gemicitamibe-erlotinib therapy. In addition, patients exhibiting higher combined expression level of VEGFA and PLGF relative to control levels determined in the entire patient population, demonstrated a prolonged overall survival and a prolonged progression free survival in response to the addition of bevacizumab to gemcitamibe-erlotinib therapy. Patients exhibiting higher combined expression level of VEGFA, VEGFR2 and PLGF relative to control levels determined in the entire patient population, demonstrated a prolonged overall survival and a prolonged progression free survival in response to the addition of bevacizumab to gemcitamibe-erlotinib therapy.

**Patients and Immunochemical Methods**

A total of 607 patients participated in the BO17706 study, and blood plasma samples from 224 of the participants were available for biomarker analysis. The baseline characteristics of the 224 patients in the biomarker analysis are provided in Table 10.

*Table 10. Baseline characteristics: biomarker population (n=224)*

| | bevacizumab | | placebo | |
|---|---|---|---|---|
| | N | (%) | N | (%) |
| Sex | | | | |
| Female | 45 | 38.46 | 32 | 29.91 |
| Male | 72 | 61.54 | 75 | 70.09 |
| | | | | |
| Age Category (years) | | | | |
| <65 | 73 | 62.39 | 71 | 66.36 |
| >=65 | 44 | 37.61 | 36 | 33.64 |
| | | | | |
| KPS (%) Category at Baseline | | | | |
| <80% | 15 | 12.82 | 13 | 12.15 |
| >=80% | 102 | 87.18 | 94 | 87.85 |
| | | | | |
| VAS Category at Baseline | | | | |
| below baseline (not available) | 10 | 8.55 | 16 | 14.95 |
| <20 | 68 | 58.12 | 56 | 52.34 |
| >=20 | 39 | 33.33 | 35 | 32.71 |
| | | | | |
| CRP Category (median value) at Baseline (mg/dL) | | | | |
| below baseline (not available) | 13 | 11.11 | 9 | 8.41 |
| <=1.4 | 52 | 44.44 | 49 | 45.79 |
| >1.4 | 52 | 44.44 | 49 | 45.79 |
| VAS: Visual Analogue Scale of Pain<br>KPS: Karnofsky Performance Score | | | | |

**Blood Plasma Analysis**

Plasma samples were collected after randomization and before any study treatment was given to the patients and VEGFA, vascular endothelial growth factor receptor 1 (VEGFR1), VEGFR2, PLGF and E-SELECTIN were measured using the IMPACT Assay described in Example 1 above.

**Statistical Analysis**

Sample median was used to dichotomize biomarker values as low (below median) or high (above median).

Hazard Ratio of treatment effect in sub-group of patients with high or low biomarker levels were estimated with proportional hazard cox regression analysis.

In addition, proportional hazard cox regressions was used to evaluate the association between biomarker level and treatment effect. The model included the following covariates: trial treatment, biomarker level, interaction term of treatment by biomarker level. Wald test for the interaction term was used to determined the association between biomarker level and treatment effect. P-value below 0.05 was considered significant.

**Results**

Blood Plasma Markers

The baseline descriptive statistics of the biomarkers are presented in Table 11.

*Table 11: Descriptive Statistics of Biomarker Values (Baseline)*

| | ***VEGFA*** *(pg/mL) at baseline* | ***VEGFR2*** *(ng/mL) at baseline* | ***PIGF*** *(pg/ml) at baseline* |
|---|---|---|---|
| ***min*** | *3.06* | *0.23* | *0* |
| ***qu 25%*** | *80.08* | *7.9* | *32.9* |
| ***median*** | *152.80* | *9.9* | *37.8* |
| ***qu 75%*** | *275.90* | *12.6* | *43.6* |
| ***max*** | *2127.00* | *58.1* | *142.3* |
| ***mean*** | *215.30* | *10.4* | *39.4* |
| ***sd*** | *254.8* | *4.7* | *12.5* |

Table 12 presents the univariate analysis of the association of the selected biomarkers with treatment effect on overall survival.

*Table 12: Association with treatment effect on Overall Survival - (uni-variate analysis)*

| | HR (95% CI) | P-value for interaction |
|---|---|---|
| VEGFA low | 1.018 (0.69, 1.5) | 0.0308 |
| VEGFA high | 0.558 (0.37,0.83) | |
| VEGFR2 low | 1.057 (0.72,1.55) | 0.0461 |
| VEGFR2 high | 0.583 (0.39,0.87) | |
| PLGF low | 1.048 (0.67, 1.63) | 0.089 |
| PLGF high | 0.659 (0.46, 0.95) | |

In this analysis, for VEGFA, Low VEGFA <152.9 pg/ml and High VEGFA ≥ 152.9 pg/ml, for VEGFR2, Low VEGFR2 < 9.9 ng/ml and High VEGFRA ≥ 9.9 ng/ml, and for PLGF, Low PLGF < 36.5 pg/ml and High PLGF ≥ 36.5 pg/ml, were used.

For VEGFA and VEGFR2 the cut-off levels were determined as sample data median value, such that 50% of patients have high expression and 50% of patients have low expression, as per pre-determined analysis plan. The PLGF cut-off levels were determined as $42^{nd}$ percentile of the data. Accordingly, 58% of patients have high expression of PLGF and 42% have low expression. The cut-off was determined in order to increase the statistical difference between treatment effect in high and low level subgroup.

This result table shows that the Hazard Ratio for treatment effect is significantly better in the subset of patients with high VEGFA compared to patients with low VEGFA. This result table also shows that the Hazard Ratio for treatment effect is significantly better in the subset of patients with high VEGFR2 compared to patients with low VEGFR2. Therefore, VEGFA and VEGFR2 are each independent predictive biomarkers for Bevacizumab treatment effect on overall survival. Table 13 presents the univariate analysis of the association of the selected biomarkers with treatment effect on progression free survival.

*Table 13: Association with treatment effect on Progression Free Survival (univariate analysis)*

| | HR (95% CI) | P-value for interaction |
|---|---|---|
| VEGFA low | 0.771 (0.53,1.13) | 0.0603 |
| VEGFA high | 0.522 (0.35,0.78) | |

(continued)

| | HR (95% CI) | P-value for interaction |
|---|---|---|
| VEGFR2 low | 0.773 (0.53,1.12) | 0.4012 |
| VEGFR2 high | 0.541 (0.36,0.81) | |
| PLGF low | 0.957 (0.63,1.46) | 0.0136 |
| PLGF high | 0.505 (0.35,0.73) | |

In this analysis, for VEGFA, Low VEGFA < 152.9 pg/ml and High VEGFA ≥ 152.9 pg/ml, for VEGFR2, Low VEGFR2 < 9.9 ng/ml and High VEGFRA ≥ 9.9 ng/ml, and for PLGF, Low PLGF < 36.5 pg/ml and High PLGF ≥ 36.5pg/ml, were used. For VEGFA and VEGFR2 the cut-off levels were determined as sample data median value, such that 50% of patients have high expression and 50% of patients have low expression, as per pre-determined analysis plan. The PLGF cut-off levels were determined as 42$^{nd}$ percentile of the data. Accordingly, 58% of patients have high expression of PLGF and 42% have low expression. The cut-off was determined in order to increase the statistical difference between treatment effect in high and low level subgroup.

This result table shows that the Hazard Ratio for treatment effect is significantly better in the subset of patients with high VEGFA compared to patients with low VEGFA. This result table also shows that the Hazard Ratio for treatment effect is significantly better in the subset of patients with high PLGF compared to patients with low PLGF. Therefore, VEGFA and PLGF are each independent predictive biomarkers for bevacizumab treatment effect on progression free survival.

Table 14 presents the analysis of biomarker combinations association with treatment effect on overall survival. For this analysis the following equations were used:

Formula 1: norm(VEGFA)+1.3*norm(VEGFR2). Cut-point= median or 0

Equivalent formula: VEGFA+3.3*VEGFR2. Cut-point= median or 0

and

Formula 2: 0.25*norm(VEGFA)+0.21 *norm(PLGF), cut-point=median or 0 Equivalent formula: 0.19*VEGFA+0.67*PLGF, cut-point= median or 4.8

Where we use log2 transformation and

$$x_i \rightarrow norm(x_i) = \frac{\log 2(x_i) - median(\log 2(x))}{mad(\log 2(x))}$$

*Table 14: Association with treatment effect on Overall Survival (bi-marker analysis)*

| | HR (95% CI) | P-value for interaction |
|---|---|---|
| VEGFA & VEGFR2 low | 1.317 (0.89,1.94) | 0.0002 |
| VEGFA & VEGFR2 high | 0.42 (0.28,0.64) | |
| VEGFA & PLGF low | 1.101 (0.74,1.64) | 0.0096 |
| VEGFA & PLGF high | 0.546 (0.37,0.81) | |

In this analysis, a high combined expression level of VEGFA and VEGFR2 is (Formula 1 ≥ -0.10) and a low combined expression of VEGFA and VEGFR2 is (Formula 1 < -0.10), and a high combined expression level of VEGFA and PLGF is (Formula 2 ≥ -0.042) and a low combined expression of VEGFA and PLGF is (Formula 2 < -0.042).

This results table shows that the Hazard Ratio for treatment effect is significantly better in the subset of patients with high VEGFA & VEGFR2 combination compared to patients with low VEGFA & VEGFR2 combination. This result table also shows that the Hazard Ratio for treatment effect is significantly better in the subset of patients with high VEGA & PLGF combination compared to patients with low VEGFA & PLGF combination. Therefore, VEGFA & VEGFR2 combination and VEGFA & PLGF combination are each independent predictive biomarkers for bevacizumab treatment effect on overall survival.

Table 15 presents the analysis of biomarker combinations association with treatment effect on progression free survival. For this analysis the following equations were used:

Formula 1: norm(VEGFA)+1.3*norm(VEGFR2). Cut-point= median or 0 Equivalent formula: VEGFA+3.3*VEGFR2. Cut-point= median or 0
and
Formula 2: 0.25*norm(VEGFA)+0.21*norm(PLGF), cut-point=median or 0 Equivalent formula: 0.19*VEGFA+0.67*PLGF, cut-point= median or 4.8
Where we use log2 transformation and

$$x_i \rightarrow norm(x_i) = \frac{\log 2(x_i) - median(\log 2(x))}{mad(\log 2(x))}$$

*Table 15: Association with treatment effect on Progression Free Survival (bi-marker analysis)*

| | HR (95% CI) | P-value for interaction |
|---|---|---|
| VEGFA & VEGFR2 low | 0.984 (0.68,1.43) | 0.0040 |
| VEGFA & VEGFR2 high | 0.411 (0.26,0.64) | |
| VEGFA & PLGF low | 0.936 (0.64,1.37) | 0.0011 |
| VEGFA & PLGF high | 0.426 (0.28,0.64) | |

In this analysis, a high combined expression level of VEGFA and VEGFR2 is (Formula 1 ≥ -0.10) and a low combined expression of VEGFA and VEGFR2 is (Formula 1 < -0.10), and a high combined expression level of VEGFA and PLGF is (Formula 2 ≥ -0.042) and a low combined expression of VEGFA and PLGF is (Formula 2 < -0.042).

This results table shows that the Hazard Ratio for treatment effect is significantly better in the subset of patients with high VEGFA & VEGFR2 combination compared to patients with low VEGFA & VEGFR2 combination. This result table also shows that the Hazard Ratio for treatment effect is significantly better in the subset of patients with high VEGA & PLGF combination compared to patients with low VEGFA & PLGF combination. Therefore, VEGFA & VEGFR2 combination and VEGFA & PLGF combination are each independent predictive biomarkers for bevacizumab treatment effect on progression free survival.
Tables 16 and Table 17 present the analysis of biomarker combinations of VEGFA, VEGFR2 and PLGF association with treatment effect on overall survival and progression free survival, respectively.
In this analysis, the following equation was used:

$$\text{Formula 3: } 0.0127 * \ln(\text{PLGF}+1) + 0.144 * \ln(\text{VEGFR2}+1) + 0.0949 * \ln(\text{VEGFA}+1)$$

Where ln = log basis e

*Table 16: Association with treatment effect on Overall Survival (tri-marker analysis)*

| Overall Survival | HR (95% CI) | P-value for interaction |
|---|---|---|
| VEGFA & VEGFR2 & PLGF low | 1.051 (0.71,1.55) | 0.0033 |
| VEGFA & VEGFR2 & PLGF high | 0.554 (0.38;0.8) | |

*Table 17: Association with treatment effect on Progression Free Survival (try-marker analysis)*

| Progression Free Survival | HR (95% CI) | P-value for interaction |
|---|---|---|
| VEGFA & VEGFR2 & PLGF low | 0.974 (0.64,1.48) | 0.0096 |
| VEGFA & VEGFR2 & PLGF high | 0.488 (0.34,0.71) | |

In this analysis, for overall survival, a high combined expression level of VEGFA, VEGFR2 and PLGF is (Formula 3 ≥ 0.837) and a low combined expression of VEGFA, VEGFR2 and PLGF is (Formula 3 < 0.837), and for progression free survival, a high combined expression level of VEGFA, VEGFR2 and PLGF is (Formula 3 ≥ 0.837) and a low combined expression of VEGFA, VEGFR2 and PLGF is (Formula 3 < 0.837).

This results table shows that the Hazard Ratio for treatment effect is significantly better in the subset of patients with high VEGFA & VEGFR2 & PLGF combination compared to patients with a low VEGFA & VEGFR2 & PLGF combination. Therefore, the VEGFA & VEGFR2 & PLGF combination is a predictive biomarkers for bevacizumab treatment effect on progression free survival.

This results table also shows that for overall survival the Hazard Ratio for treatment effect is significantly better in the subset of patients with high VEGFA & VEGFR2 & PLGF combination compared to patients with low VEGFA & VEGFR2 & PLGF combination. Therefore, the VEGFA & VEGFR2 & PLGF combination is a predictive biomarkers for Bevacizumab treatment effect on overall survival.

**Example 6: Analyses of Additional Plasma Samples for VEGFA**

Baseline samples from the AVF2107g (a phase III, multicenter, randomized, active controlled clinical trial to evaluate the efficacy and safety of rhumab (bevacizumab) in combination with standard chemotherapy in subjects with metastatic colorectal cancer), AVAiL (a randomized, double-blind, multicenter phase III study of bevacizumab in combination with cisplatin and gemcitabine versus placebo, cisplatin and gemcitabine in patients with advanced or recurrent non-squamous non-small cell lung cancer who have not received prior chemotherapy), and AVOREN (a randomized, double-blind, phase III study to evaluate the efficacy and safety of bevacizumab in combination with interferon alfa-2a (roferon) versus interferon alfa-2a and placebo as first line treatment administered to nephrectomised patients with metastatic clear cell renal cell carcinoma) were also analyzed with the IMPACT Assay described in Example 1.

In Study AVF2107g, 380 samples from (47%) were available for retesting. The new VEGF-A data confirmed a prognostic value for VEGF-A but did not show a potential predictive value. Patients with metastatic colorectal cancer with high levels of VEGF-A, as measured by the IMPACT ELISA, had similar hazard ratios for PFS (0.52 for VEGF-A high vs. 0.64 for VEGF-A low) and OS (0.68 for VEGF A high vs. 0.70 for VEGF-A low).

A similar prognostic value was seen in the results of retesting of the 852 AVAIL samples (52%). However, patients with non-small cell lung cancer with high levels of VEGF-A also showed a similar hazard ratio compared with patients with low levels of VEGF-A in the 7.5-mg/kg group (0.75 vs. 0.77, respectively, for PFS and 0.89 vs. 0.92 for OS). The 15-mg/kg dose group also showed similar hazard ratios for patients with high levels of VEGF-A (0.76 for PFS and 0.98 for OS) compared with patients with low levels (0.96 for PFS and 0.97 for OS).

Also in the AVOREN trial, retesting of 400 baseline plasma samples (62%) did not reveal a potential predictive value for VEGF-A in patients with renal cell carcinoma, although the prognostic value of the biomarker was seen. Patients with high levels of VEGF-A showed a hazard ratio of 0.67 for PFS compared with 0.49 for patients with low levels of VEGF-A.

The VEGF-A biomarker correlation with clinical outcomes from the six different Avastin studies are presented in Table 18.

**Table 18**

VEGF-A Biomarker Correlation with Clinical Outcome in Bevacizumab Studies

| Study (Indication): VEGF-A Level by Bev Dose Cohort | Median (mo) | | PFS Hazard Ratio (VEGF-A Low vs. High) | Interaction p-value |
|---|---|---|---|---|
| | Chemo | Chemo + Bev | | |
| AVADO (MBC) | | | | |
| VEGF-A at 15 mg/kg Bev | | | 0.86 vs. 0.49 | 0.08 |
| Low | 8 | 8.5 | | |
| High | 6.6 | 8.8 | | |
| VEGF-A at 7.5 mg/kg Bev | | | 0.96 vs. 0.52 | 0.01 |
| Low | 8 | 8.8 | | |
| High | 6.6 | 8.5 | | |
| AVITA (pancreatic cancer): at 7.5 mg/kg Bev | | | | |

(continued)

**Table 18**
VEGF-A Biomarker Correlation with Clinical Outcome in Bevacizumab Studies

| Study (Indication): VEGF-A Level by Bev Dose Cohort | Median (mo) | | PFS Hazard Ratio (VEGF-A Low vs. High) | Interaction p-value |
|---|---|---|---|---|
| | Chemo | Chemo + Bev | | |
| VEGF-A | | | 0.76 vs. 0.56 | 0.06 |
| Low | 4.6 | 5.3 | | |
| High | 3.3 | 5.1 | | |
| AVAGAST (gastric cancer): at 7.5 mg/kg Bev | | | | |
| VEGF-A | | | 0.86 vs. 0.67 | 0.14 |
| Low | 5.7 | 7 | | |
| High | 4.8 | 6.9 | | |
| VEGF-A, excluding Asia-Pacific region | | | 0.80 vs. 0.56 | 0.14 |
| Low | 5.5 | 7 | | |
| High | 4.4 | 7.2 | | |
| AVF2107g (mCRC): at 7.5 mg/kg Bev | | | | |
| VEGF-A | | | 0.64 vs. 0.52 | 0.61 |
| Low | 6.9 | 9.8 | | |
| High | 5.6 | 10.6 | | |
| Bev = bevacizumab; Chemo = chemotherapy; MBC = metastatic breast cancer; mCRC = metastatic colorectal cancer; PFS = progression-free survival; mRCC = metastatic renal cell carcinoma; VEGF-A = vascular endothelial growth factor A. | | | | |
| AVOREN (RCC): at 15 mg/kg Bev | | | | |
| VEGF-A | | | 0.49 vs. 0.67 | 0.42 |
| Low | 7.2 | 12.9 | | |
| High | 3.7 | 7.7 | | |
| AVAiL | | | | |
| VEGF-A: at 15 mg/kg Bev | | | 0.96 vs. 0.76 | 0.13 |
| Low | 6.6 | 6.9 | | |
| High | 6.0 | 6.5 | | |
| VEGF-A: at 7.5 mg/kg Bev | | | 0.77 vs. 0.75 | 0.77 |
| Low | 6.6 | 7.1 | | |
| High | 6.0 | 6.6 | | |
| Bev = bevacizumab; Chemo = chemotherapy; MBC = metastatic breast cancer; mCRC = metastatic colorectal cancer; PFS = progression-free survival; RCC = renal cell carcinoma; VEGF-A = vascular endothelial growth factor A. | | | | |

All three studies confirmed the prognostic value of plasma VEGF-A, as previously shown with previous VEGF-A assays. However, the potential predictive value, as shown in AVADO, AVITA, and AVAGAST could not be confirmed in the additional trials in three different indications. It should be noted that the samples collected in the AVF2107g, AVAiL, and AVOREN trials were handled somewhat differently compared with the samples collected in AVADO, AVAGAST, and AVITA (i.e., citrate vs. EDTA, more freeze/thaw cycles in 12%-16% of the samples, longer storage time). As demonstrated in Example 7 below, collection of plasma into citrate or EDTA can affect the sensitivity of VEGF-A assays. The exact differences between the samples are shown in Table 19 below.

*Table 19: Sample Storage Conditions*

| Study | Samples NR | Sample Type | Percent Freeze/Thaw Cycle > 2 | Mean Time in Storage (mo) | Comments |
|---|---|---|---|---|---|
| AVF2107g | 380 (47%) | Citrate | UNK | UNK | 41 clotted samples |
| AVOREN | 400 (62%) | Citrate | 16% | 63.4 | |
| AVAiL | 852 (52%) | Citrate | 12% | 56.9 | 8 different tubes |
| AVITA | 225 (32%) | EDTA | 0 | 38.1 | |
| AVAGAST | 712 (92%) | EDTA | 0 | TBD | |
| AVADO | 396 (54%) | EDTA | 0 | 45.3 | |
| EDTA = ethylenediaminetetra-acetic acid; NR = not reported; TBD = to be determined; UNK = unknown. | | | | | |

Therefore, the lack of predictive value in these three studies does not negate the possibility that VEGF-A may act as a predictive marker in MBC. It also cannot be ruled out that the finding is indication specific. The differences between the two assays have revealed a difference in sensitivity of the second-generation assay for the shorter VEGF isoforms ($VEGF_{110}$ and $VEGF_{121}$). Without being bound by theory, there may be a different biologic effect and abundance of different isoforms in different indications. For example, $VEGF_{121}$ has showed a stronger induction of tumorigenesis than the other commonly expressed 165 and 189 isoforms in transfected xenograft models (Zhang, H.T. et al., Brit. J. Cancer 83 (2000) 63-68). The 121-amino acid isoform of vascular endothelial growth factor is more strongly tumorigenic than other splice variants in vivo (Zhang, H.T. et al., Brit. J. Cancer 83 (2000) 63-68). This is particularly significant in view of the fact that the 121 isoform has been shown to be the predominant isoform in primary human breast carcinomas (Relf, M. et al., Cancer Res. 57 (1997) 963-969).

In summary, the IMPACT Assay has shown a consistent prognostic value for plasma VEGF-A across the tested indications. In addition, this test generated consistent results in AVADO, AVITA, and AVAGAST, demonstrating a predictive value for bevacizumab in these indications, while retesting of the plasma samples in the AVF2107g, AVAiL, and AVOREN trials did not show such correlation, suggesting no potential predictive value of plasma VEGF-A expression in these three other indications. However, it should be noted that differences in samples might have affected these differences in results; one such difference is demonstrated in Example 7 below. Without being bound by theory, there may also be a different biologic effect and abundance of different isoforms in different indications, given that the IMPACT Assay favors the shorter isoforms $VEGF_{110}$ and VEGF121.

**Example 7: Detection of short VEGF isoforms in plasma collected in Na citrate and EDTA**

Paired plasma samples were collected from patients with HER2+ locally recurrent or metastatic breast cancer in both an EDTA monovette (5mL)-and Citrate Monovette collection tube (5mL). Within 30 minutes of blood collection, blood tubes were placed into the centrifuge and spun 1500 g at room temperature for 10 minutes, until cells and plasma were separated. Immediately after centrifugation, the plasma was carefully transferred into a propylene transfer tube and then aliquotted equally into 2 storage tubes (half volume each approximately 1.25 mL) using a pipette. The levels of VEGF-A in the samples were measured using the IMPACT Assay described above. As shown in Figure 21, the VEGFA concentration is about 40% higher for plasma samples collected and stored in EDTA compared to plasma samples collected and stored in citrate with a Spearman correlation for the EDTA-Citrate method comparison of about 0.8 for baseline samples collected prior to treatment.

Although the foregoing invention has been described in some detail by way of illustration and example for purposes of clarity of understanding, the descriptions and examples should not be construed as limiting the scope of the invention. The disclosures of all patents, patent applications, scientific references, and Genbank Accession Nos. cited herein are expressly incorporated by reference in their entirety for all purposes as if each patent, patent application, scientific reference, and Genbank Accession No. were specifically and individually incorporated by reference.

## Claims

1. A method of identifying a patient who may benefit from treatment with an anti-cancer therapy comprising a VEGF antagonist, the method comprising:

   determining an expression level of $VEGF_{110}$ in a sample obtained from the patient, wherein a level of $VEGF_{110}$ in the sample obtained from the patient at or above a reference level indicates that the patient may benefit from treatment with the anti-cancer therapy.

2. A method of predicting responsiveness of a patient suffering from cancer to treatment with an anti-cancer therapy comprising a VEGF-A antagonist, the method comprising:

   determining an expression level of $VEGF_{110}$ in a sample obtained from the patient, wherein a level of $VEGF_{110}$ in the sample obtained from the patient at or above a reference level indicates that the patient is more likely to be responsive to treatment with the anti-cancer therapy.

3. A method for determining the likelihood that a patient with cancer will exhibit benefit from anti-cancer therapy comprising a VEGF-A antagonist, the method comprising:

   determining an expression level of $VEGF_{110}$ in a sample obtained from the patient, wherein a level of $VEGF_{110}$ in the sample obtained from the patient at or above a reference level indicates that the patient has increased likelihood of benefit from the anti-cancer therapy.

4. A method for optimizing the therapeutic efficacy of an anti-cancer therapy comprising a VEGF-A antagonist, the method comprising:

   determining an expression level of $VEGF_{110}$ in a sample obtained from the patient, wherein a level of $VEGF_{110}$ in the sample obtained from the patient at or above a reference level indicates that the patient has increased likelihood of benefit from the anti-cancer therapy.

5. The method of any one of claims 1 to 4, wherein the cancer is selected from the group consisting of: colorectal cancer, glioblastoma, renal cancer, ovarian cancer, breast cancer, pancreatic cancer, gastric cancer, and lung cancer.

6. The method of any one of claims 1 to 4, wherein the sample obtained from the patient is a member selected from the group consisting of: whole blood, plasma, serum, and combinations thereof.

7. The method of any one of claims 1 to 4, wherein the $VEGF_{110}$ level is a protein level.

8. The method of claim 9, wherein the protein level is determined by measuring plasma protein level.

9. The method of claim 8, wherein a plasma level of $VEGF_{110}$ in the sample obtained from the patient that is at or above the level of $VEGF_{110}$ in a reference sample, indicates that the patient may benefit from the anti-cancer therapy, is more likely to be responsive to the anti-cancer therapy, or has increased likelihood of benefit from the anti-cancer therapy.

10. A kit for determining whether a patient may benefit from treatment with an anti-cancer therapy comprising a VEGF-A antagonist, the kit comprising a set of compounds capable of specifically binding to $VEGF_{110}$, and instructions for using said compounds to determine the level of $VEGF_{110}$ to predict responsiveness of a patient to treatment with an anti-cancer therapy comprising a VEGF-A antagonist, wherein a level of $VEGF_{110}$ at or above the level of $VEGF_{110}$ in a reference sample indicates that the patient may benefit from treatment with an anti-cancer therapy comprising a VEGF-A antagonist.

11. The kit of claim 10, wherein the compounds are proteins.

12. The kit of claim 11, wherein the proteins are antibodies.

13. A set of compounds for detecting levels of $VEGF_{110}$, the set comprising at least one compound capable of specifically

binding to $VEGF_{110}$.

**14.** The set of compounds of claim 13, wherein the compounds are proteins.

**15.** The set of compounds of claim 14, wherein the proteins are antibodies.

**Figure 1**

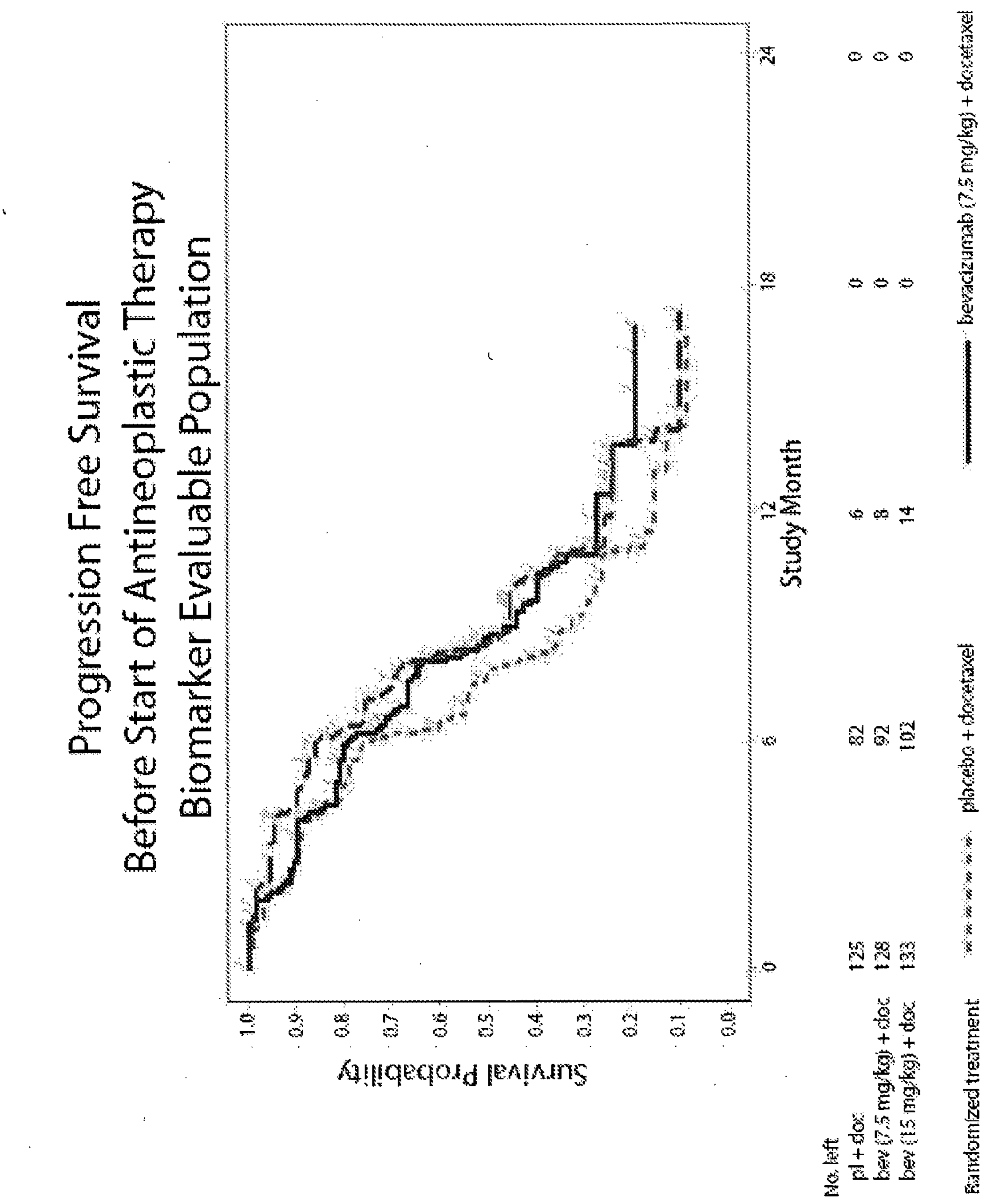
Progression Free Survival
Before Start of Antineoplastic Therapy
Biomarker Evaluable Population
Survival Probability
1.0
0.9
0.8
0.7
0.6
0.5
0.4
0.3
0.2
0.1
0.0
0
6
12
18
24
Study Month
No. left
pl + doc 125 82 6 0 0
bev (7.5 mg/kg) + doc 128 92 8 0 0
bev (15 mg/kg) + doc 133 102 14 0 0
Randomized treatment
placebo + docetaxel
bevacizumab (15 mg/kg) + docetaxel
bevacizumab (7.5 mg/kg) + docetaxel

Figure 2

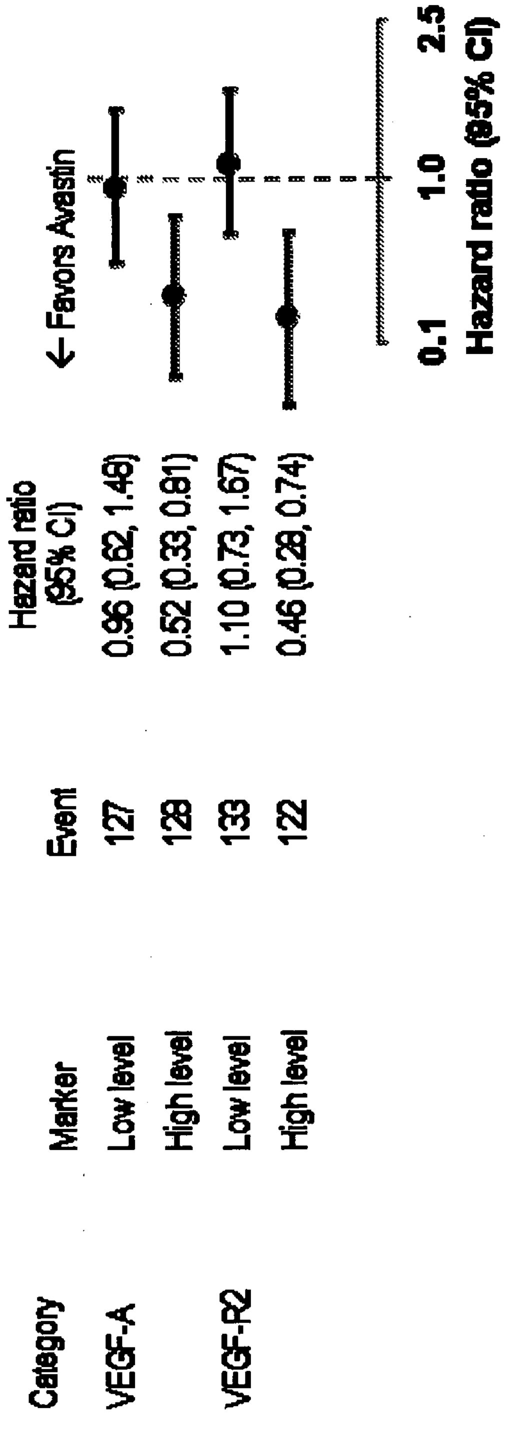


Antineoplastic Therapy by Biomarker (Placebo and Low Dose Avastin)

Figure 3

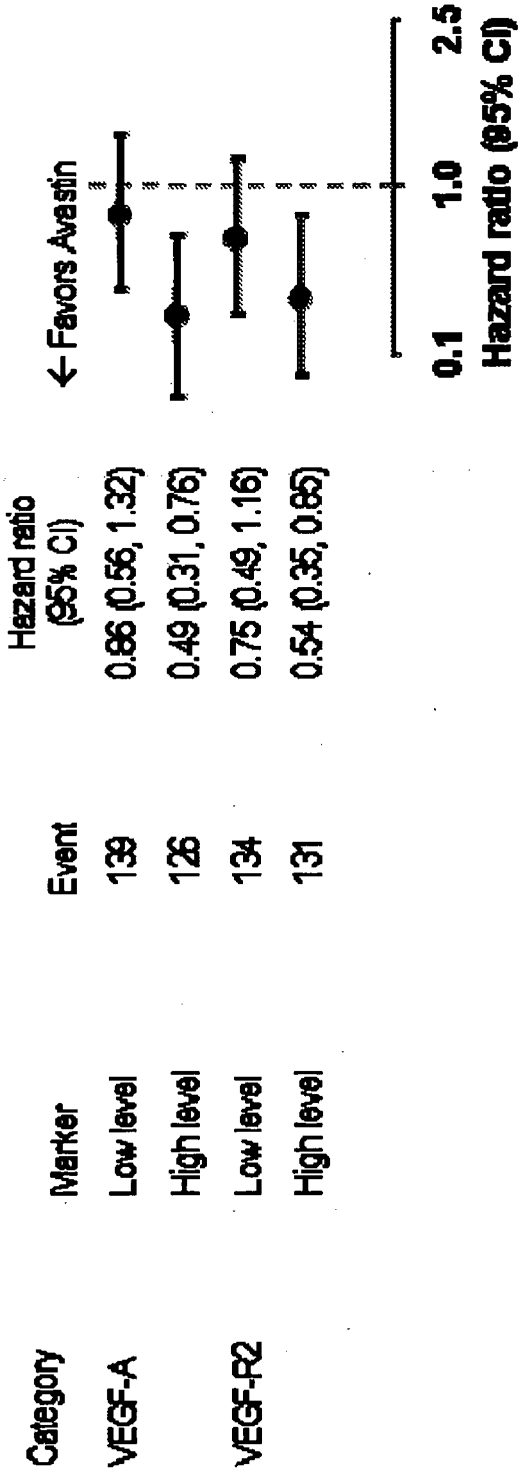


Antineoplastic Therapy by Biomarker (Placebo and High Dose Avastin)

**Figure 4A**

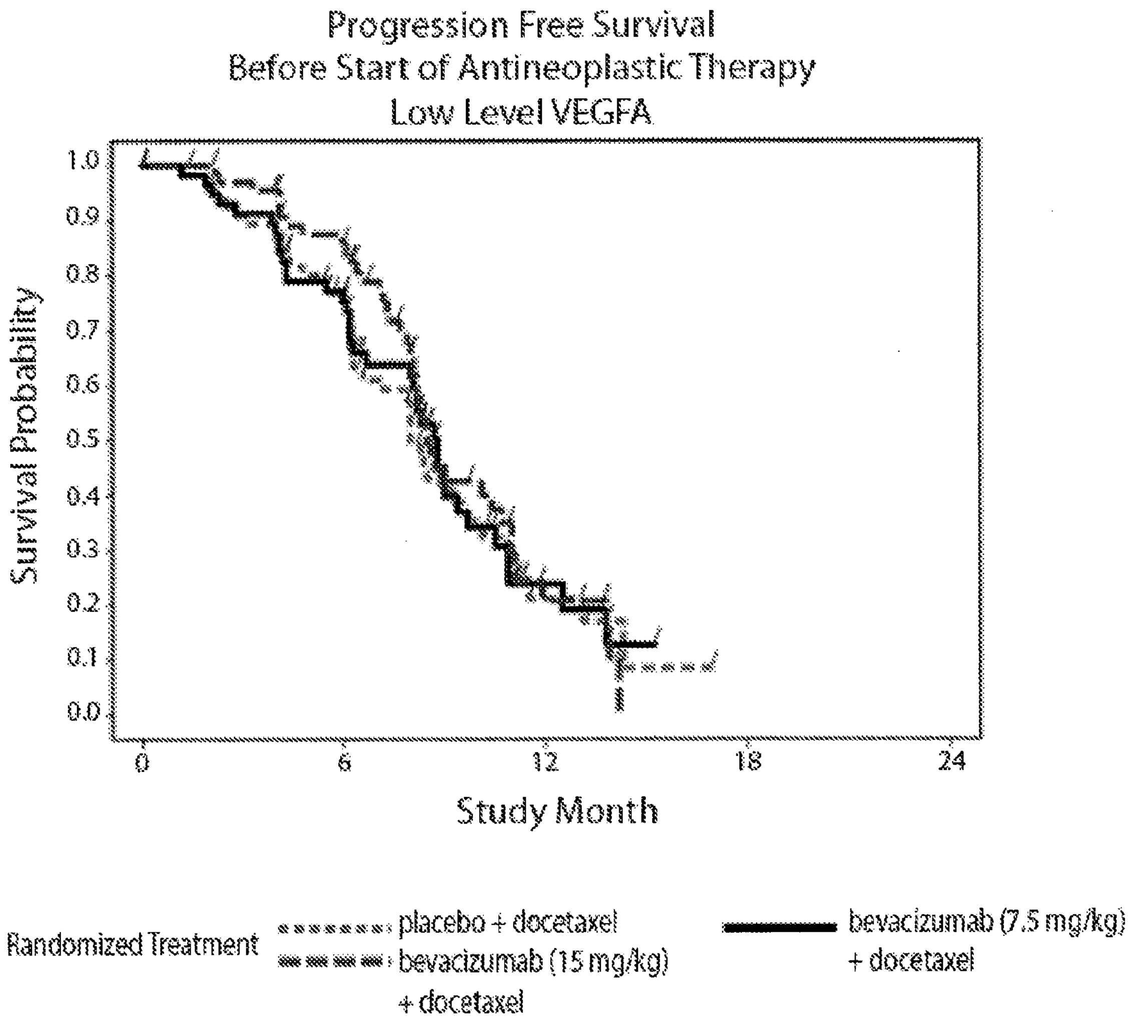
Progression Free Survival
Before Start of Antineoplastic Therapy
Low Level VEGFA
Survival Probability
1.0
0.9
0.8
0.7
0.6
0.5
0.4
0.3
0.2
0.1
0.0
0
6
12
18
24
Study Month
Randomized Treatment
placebo + docetaxel
bevacizumab (15 mg/kg) + docetaxel
bevacizumab (7.5 mg/kg) + docetaxel

Figure 4B

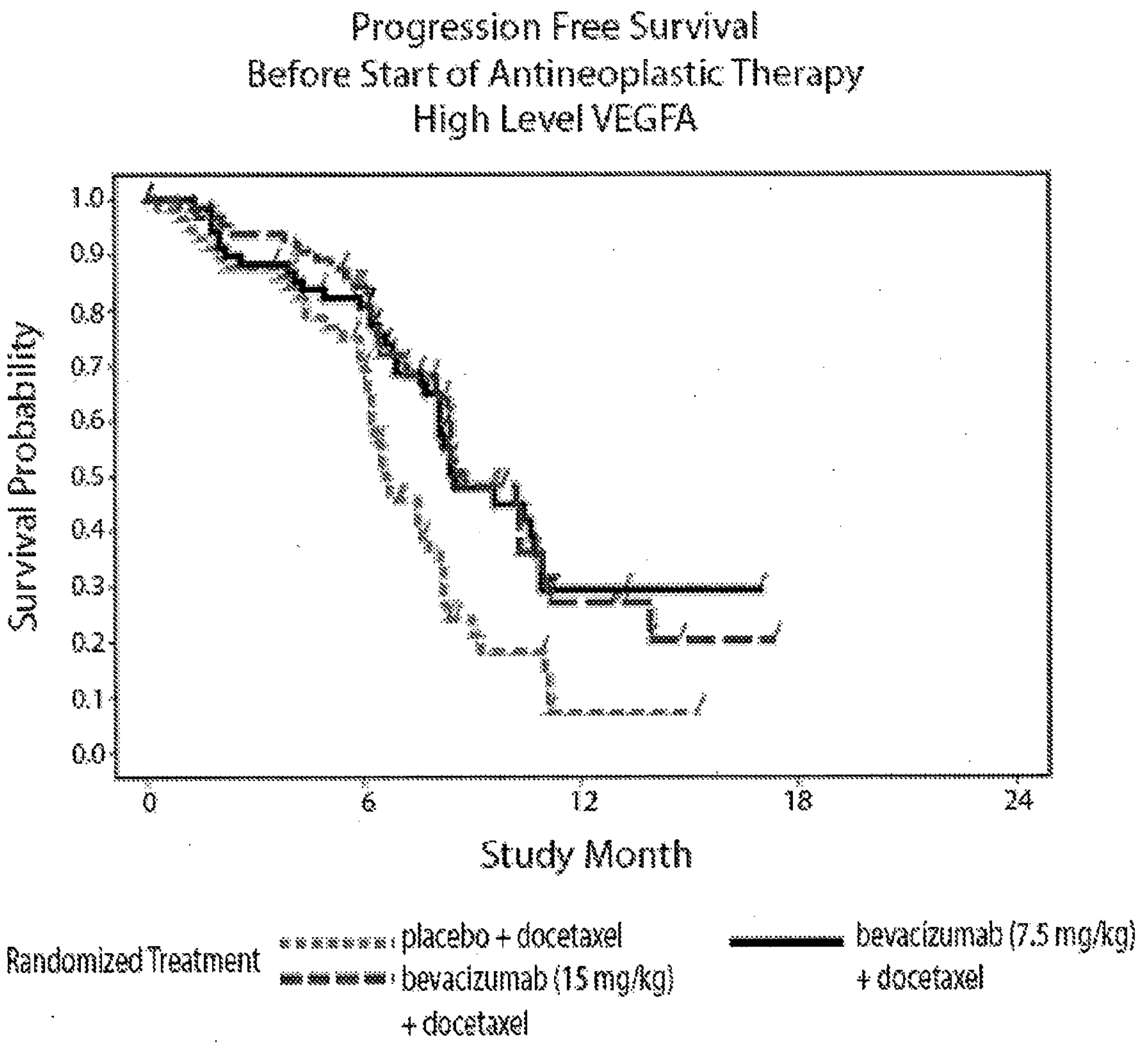
Progression Free Survival
Before Start of Antineoplastic Therapy
High Level VEGFA
Survival Probability
1.0
0.9
0.8
0.7
0.6
0.5
0.4
0.3
0.2
0.1
0.0
0
6
12
18
24
Study Month
Randomized Treatment
placebo + docetaxel
bevacizumab (15 mg/kg) + docetaxel
bevacizumab (7.5 mg/kg) + docetaxel

Figure 5A

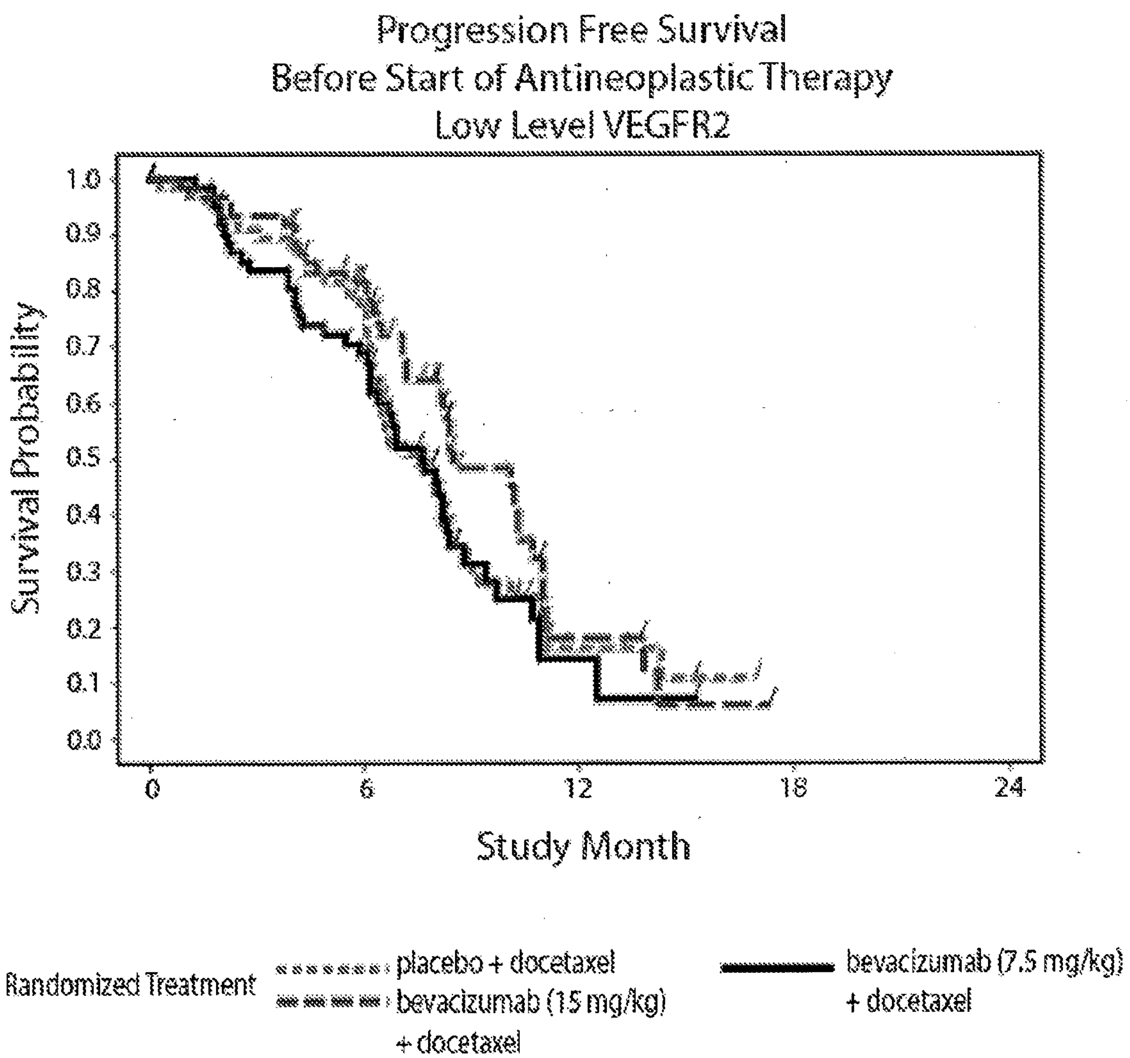
Progression Free Survival
Before Start of Antineoplastic Therapy
Low Level VEGFR2
Survival Probability
1.0
0.9
0.8
0.7
0.6
0.5
0.4
0.3
0.2
0.1
0.0
0
6
12
18
24
Study Month
Randomized Treatment
placebo + docetaxel
bevacizumab (15 mg/kg) + docetaxel
bevacizumab (7.5 mg/kg) + docetaxel

**Figure 5B**

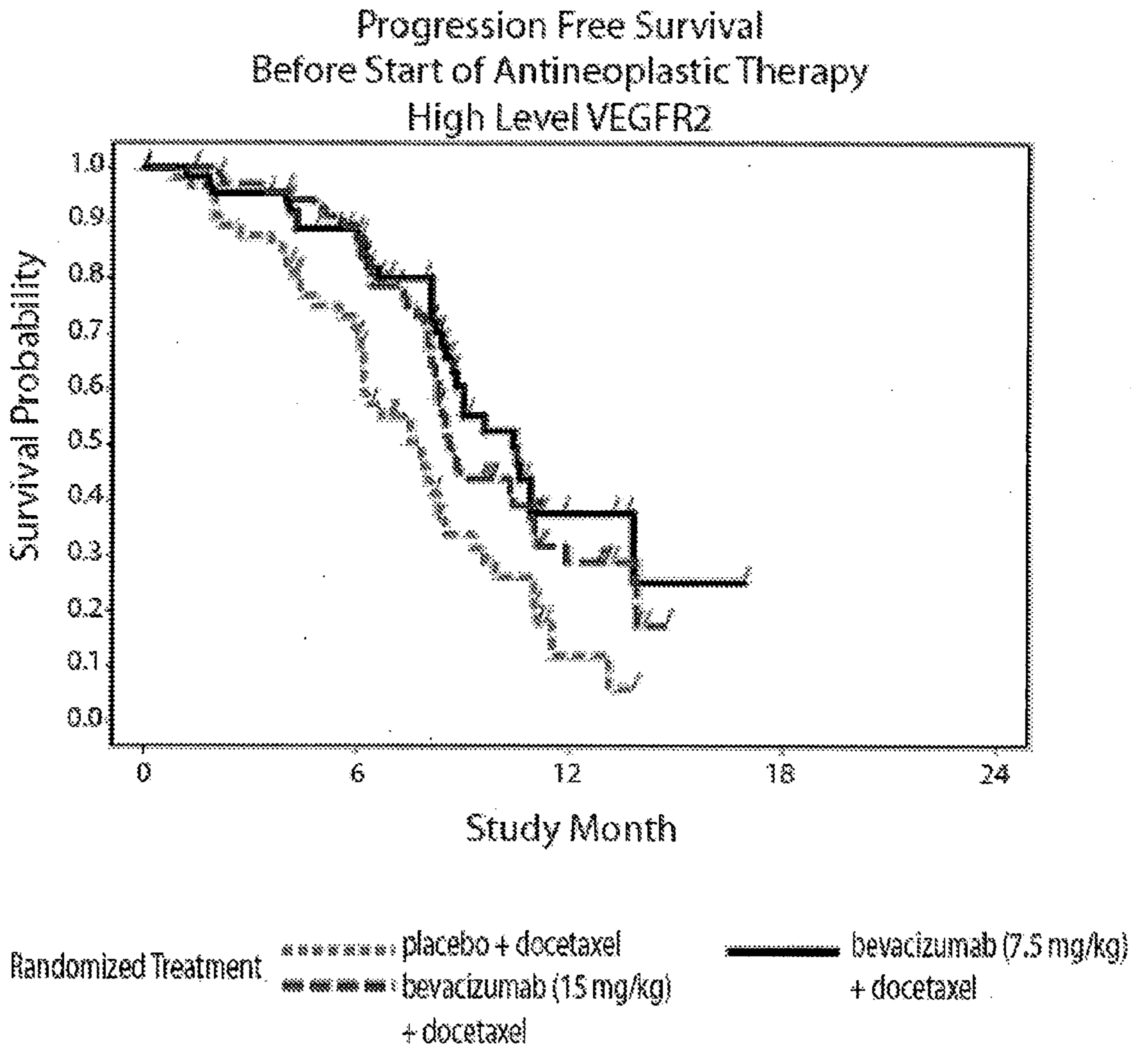
Progression Free Survival
Before Start of Antineoplastic Therapy
High Level VEGFR2
Survival Probability
1.0
0.9
0.8
0.7
0.6
0.5
0.4
0.3
0.2
0.1
0.0
0
6
12
18
24
Study Month
Randomized Treatment
placebo + docetaxel
bevacizumab (15 mg/kg) + docetaxel
bevacizumab (7.5 mg/kg) + docetaxel

**Figure 6**

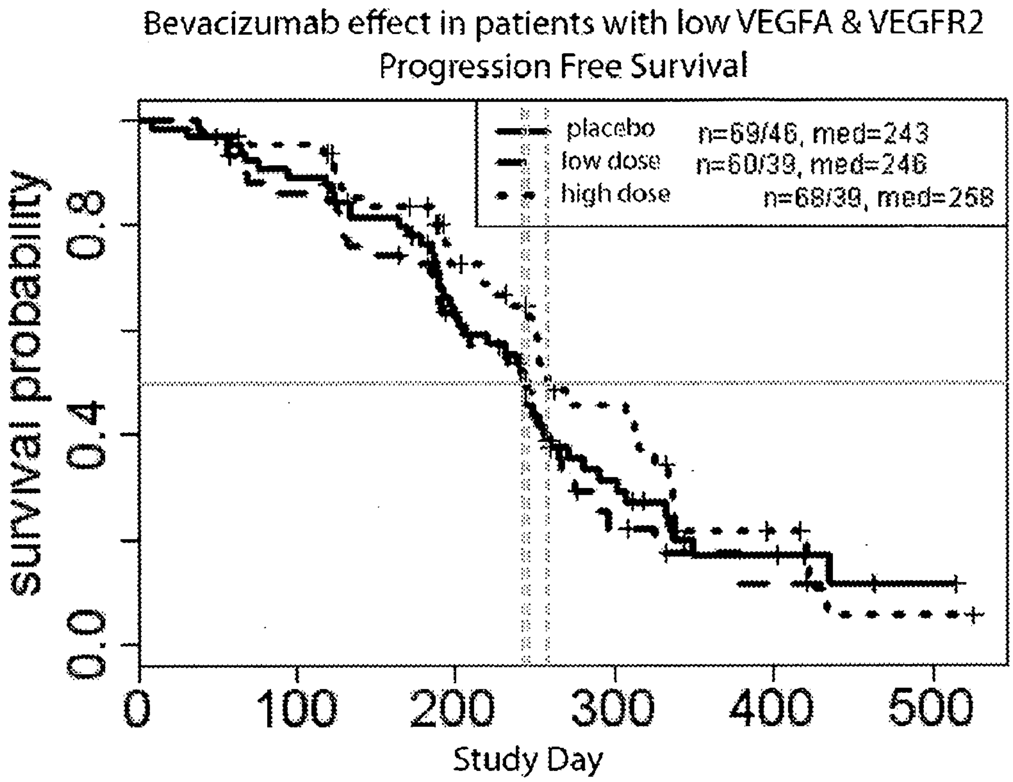
Bevacizumab effect in patients with low VEGFA & VEGFR2
Progression Free Survival
placebo n=69/46, med=243
low dose n=60/39, med=246
high dose n=68/39, med=258
survival probability
0.0
0.4
0.8
0
100
200
300
400
500
Study Day

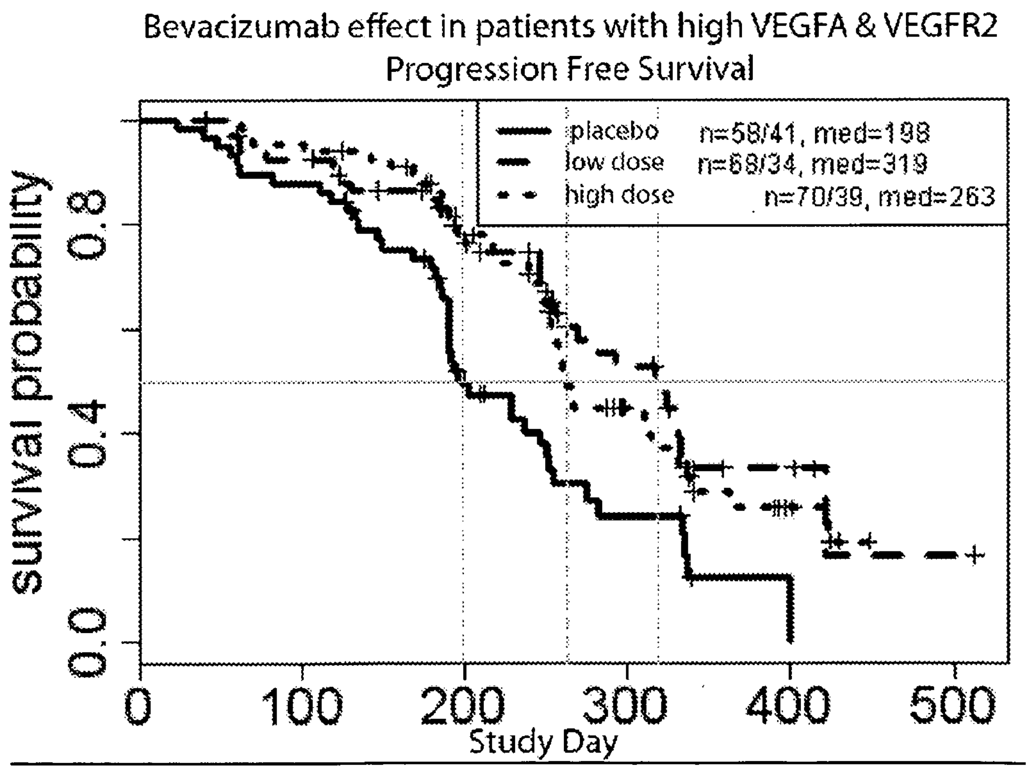
Bevacizumab effect in patients with high VEGFA & VEGFR2
Progression Free Survival
placebo n=58/41, med=198
low dose n=68/34, med=319
high dose n=70/39, med=263
survival probability
0.0
0.4
0.8
0
100
200
300
400
500
Study Day

**Figure 7**

Bevacizumab effect in patients with low VEGFA & PLGF
Progression Free Survival

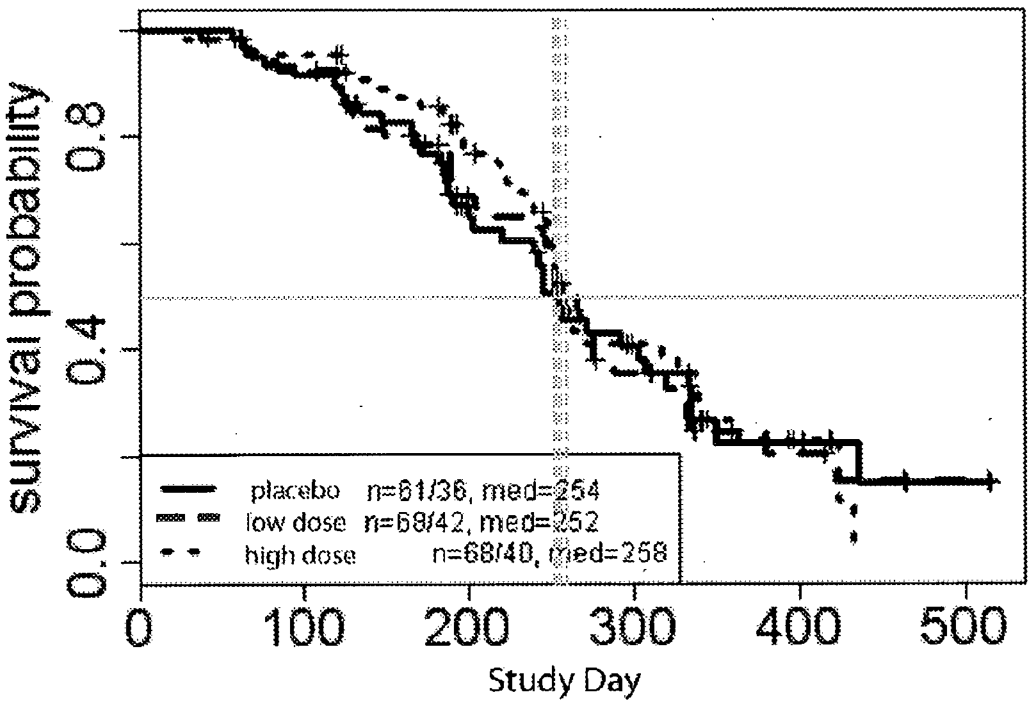


Bevacizumab effect in patients with high VEGFA & PLGF
Progression Free Survival

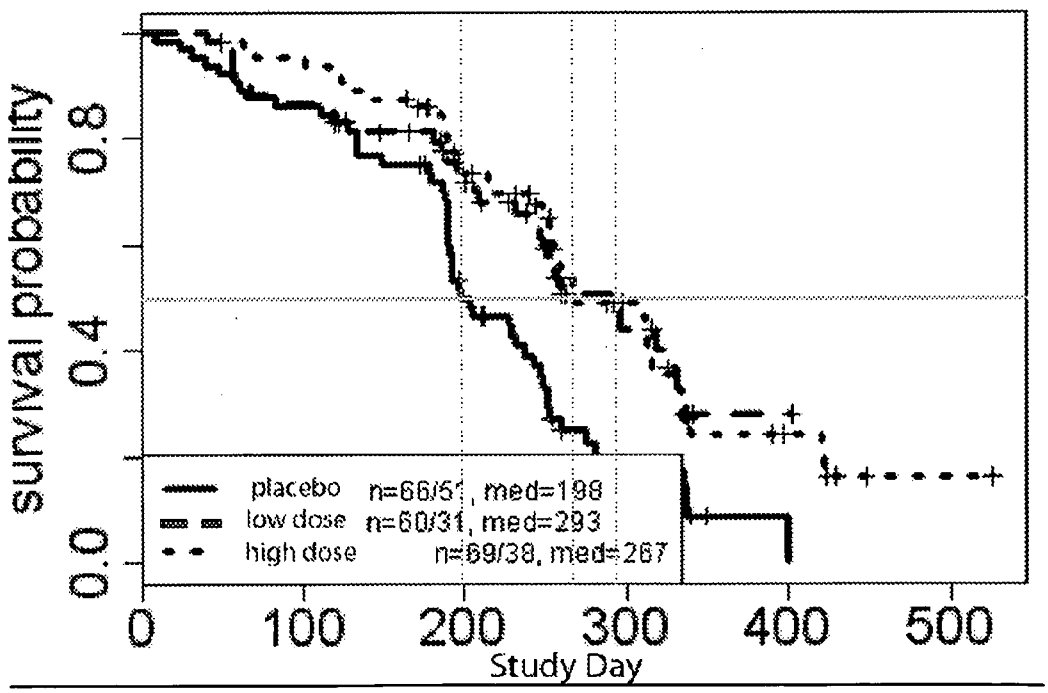

**Figure 8:** SEQ ID NO:1, Exemplary amino acid sequence of VEGFA.

```
        10         20         30         40         50         60
MNFLLSWVHW SLALLLYLHH AKWSQAAPMA EGGGQNHHEV VKFMDVYQRS YCHPIETLVD

        70         80         90        100        110        120
IFQEYPDEIE YIFKPSCVPL MRCGGCCNDE GLECVPTEES NITMQIMRIK PHQGQHIGEM

       130        140        150        160        170        180
SFLQHNKCEC RPKKDRARQE KKSVRGKGKG QKRKRKKSRY KSWSVYVGAR CCLMPWSLPG

       190        200        210        220        230
PHPCGPCSER RKHLFVQDPQ TCKCSCKNTD SRCKARQLEL NERTCRCDKP RR
```

**Figure 9**: SEQ ID NO:2, Exemplary amino acid sequence of VEGFR2.

```
        10         20         30         40         50         60
MQSKVLLAVA LWLCVETRAA SVGLPSVSLD LPRLSIQKDI LTIKANTTLQ ITCRGQRDLD

        70         80         90        100        110        120
WLWPNNQSGS EQRVEVTECS DGLFCKTLTI PKVIGNDTGA YKCFYRETDL ASVIYVYVQD

       130        140        150        160        170        180
YRSPFIASVS DQHGVVYITE NKNKTVVIPC LGSISNLNVS LCARYPEKRF VPDGNRISWD

       190        200        210        220        230        240
SKKGFTIPSY MISYAGMVFC EAKINDESYQ SIMYIVVVVG YRIYDVVLSP SHGIELSVGE

       250        260        270        280        290        300
KLVLNCTART ELNVGIDFNW EYPSSKHQHK KLVNRDLKTQ SGSEMKKFLS TLTIDGVTRS

       310        320        330        340        350        360
DQGLYTCAAS SGLMTKKNST FVRVHEKPFV AFGSGMESLV EATVGERVRI PAKYLGYPPP

       370        380        390        400        410        420
EIKWYKNGIP LESNHTIKAG HVLTIMEVSE RDTGNYTVIL TNPISKEKQS HVVSLVVYVP

       430        440        450        460        470        480
PQIGEKSLIS PVDSYQYGTT QTLTCTVYAI PPPHHIHWYW QLEEECANEP SQAVSVTNPY

       490        500        510        520        530        540
PCEEWRSVED FQGGNKIEVN KNQFALIEGK NKTVSTLVIQ AANVSALYKC EAVNKVGRGE

       550        560        570        580        590        600
RVISFHVTRG PEITLQPDMQ PTEQESVSLW CTADRSTFEN LTWYKLGPQP LPIHVGELPT

       610        620        630        640        650        660
PVCKNLDTLW KLNATMFSNS TNDILIMELK NASLQDQGDY VCLAQDRKTK KRHCVVRQLT

       670        680        690        700        710        720
VLERVAPTIT GNLENQTTSI GESIEVSCTA SGNPPPQIMW FKDNETLVED SGIVLKDGNR
```

**Figure 9 (continued)**: SEQ ID NO:2, Exemplary amino acid sequence of VEGFR2.

```
       730        740        750        760        770        780
NLTIRRVRKE DEGLYTCQAC SVLGCAKVEA FFIIEGAQEK TNLEIIILVG TAVIAMFFWL

       790        800        810        820        830        840
LLVIILRTVK RANGGELKTG YLSIVMDPDE LPLDEHCERL PYDASKWEFP RDRLKLGKPL

       850        860        870        880        890        900
GRGAFGQVIE ADAFGIDKTA TCRTVAVKML KEGATHSEHR ALMSELKILI HIGHHLNVVN

       910        920        930        940        950        960
LLGACTKPGG PLMVIVEFCK FGNLSTYLRS KRNEFVPYKT KGARFRQGKD YVGAIPVDLK

       970        980        990       1000       1010       1020
RRLDSITSSQ SSASSGFVEE KSLSDVEEEE APEDLYKDFL TLEHLICYSF QVAKGMEFLA

      1030       1040       1050       1060       1070       1080
SRKCIHRDLA ARNILLSEKN VVKICDFGLA RDIYKDPDYV RKGDARLPLK WMAPETIFDR

      1090       1100       1110       1120       1130       1140
VYTIQSDVWS FGVLLWEIFS LGASPYPGVK IDEEFCRRLK EGTRMRAPDY TTPEMYQTML

      1150       1160       1170       1180       1190       1200
DCWHGEPSQR PTFSELVEHL GNLLQANAQQ DGKDYIVLPI SETLSMEEDS GLSLPTSPVS

      1210       1220       1230       1240       1250       1260
CMEEEEVCDP KFHYDNTAGI SQYLQNSKRK SRPVSVKTFE DIPLEEPEVK VIPDDNQTDS

      1270       1280       1290       1300       1310       1320
GMVLASEELK TLEDRTKLSP SFGGMVPSKS RESVASEGSN QTSGYQSGYH SDDTDTTVYS

      1330       1340       1350
SEEAELLKLI EIGVQTGSTA QILQPDSGTT LSSPPV
```

**Figure 10**: SEQ ID NO:3, Exemplary amino acid sequence of PLGF.

```
        10         20         30         40         50         60
MPVMRLFPCF LQLLAGLALP AVPPQQWALS AGNGSSEVEV VPFQEVWGRS YCRALERLVD

        70         80         90        100        110        120
VVSEYPSEVE HMFSPSCVSL LRCTGCCGDE NLHCVPVETA NVTMQLLKIR SGDRPSYVEL

       130        140        150        160        170        180
TFSQHVRCEC RHSPGRQSPD MPGDFRADAP SFLPPRRSLP MLFRMEWGCA LTGSQSAVWP

       190        200        210        220
SSPVPEEIPR MHPGRNGKKQ QRKPLREKMK PERCGDAVPR R
```

**Figure 11**

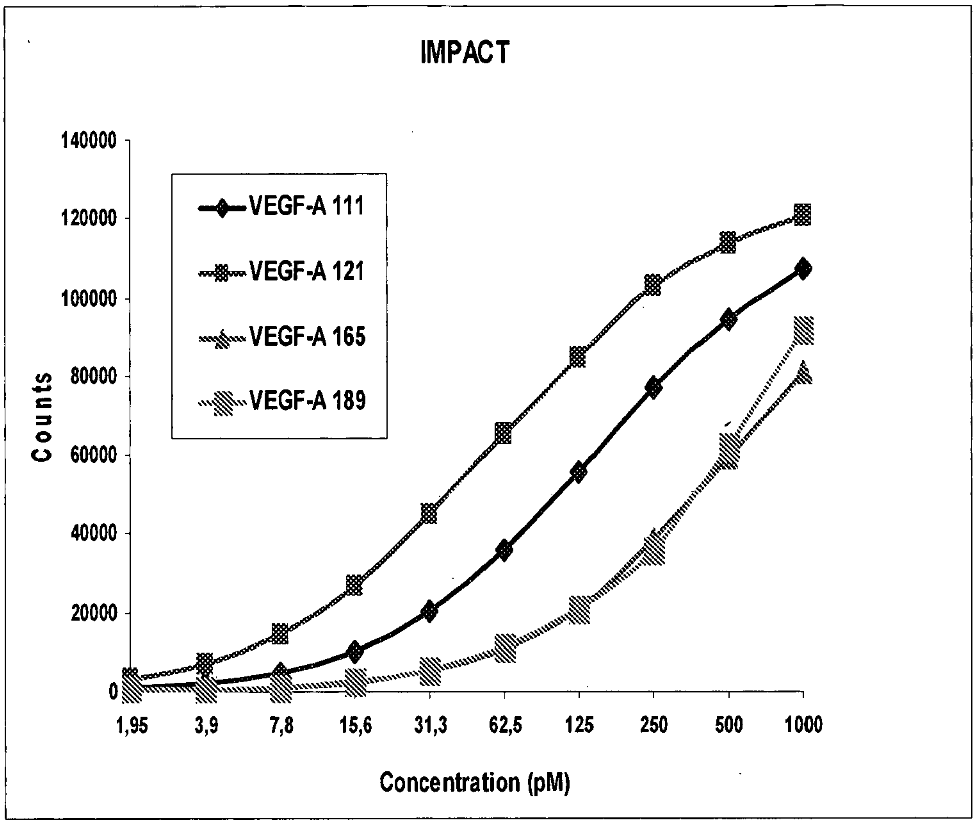
IMPACT
140000
120000
100000
80000
60000
40000
20000
0
Counts
VEGF-A 111
VEGF-A 121
VEGF-A 165
VEGF-A 189
1,95
3,9
7,8
15,6
31,3
62,5
125
250
500
1000
Concentration (pM)

Figure 12

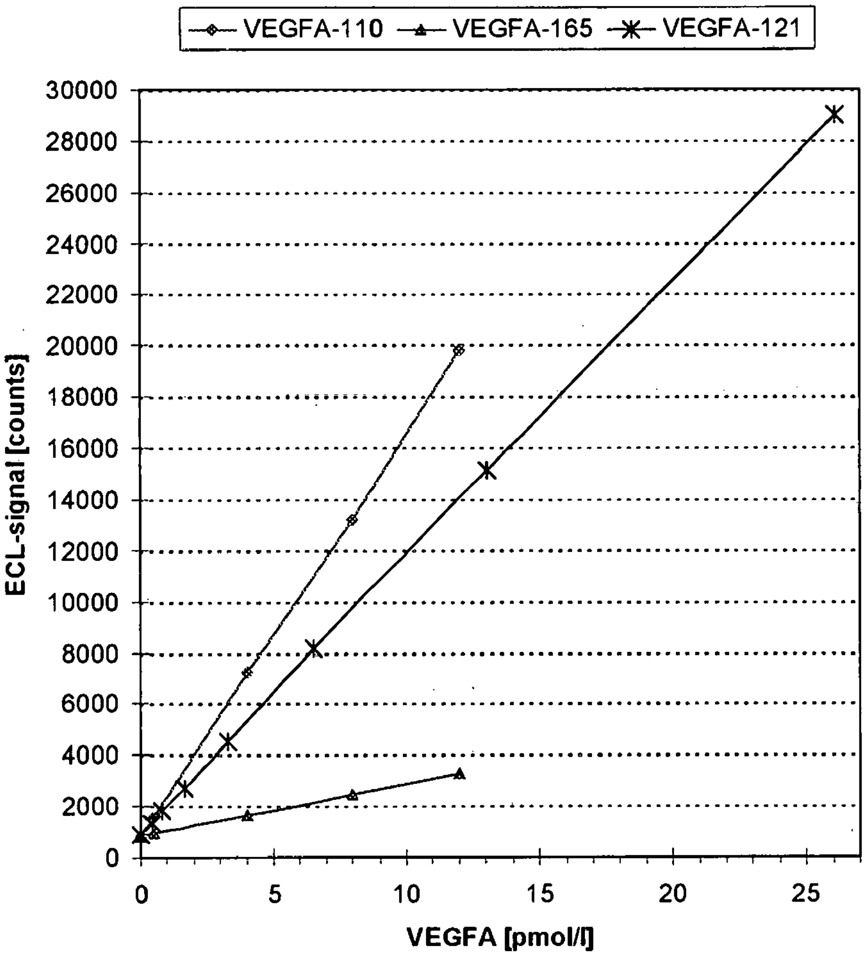
VEGFA-110
VEGFA-165
VEGFA-121
30000
28000
26000
24000
22000
20000
18000
16000
14000
12000
10000
8000
6000
4000
2000
0
ECL-signal [counts]
0
5
10
15
20
25
VEGFA [pmol/l]

**Figure 13A**

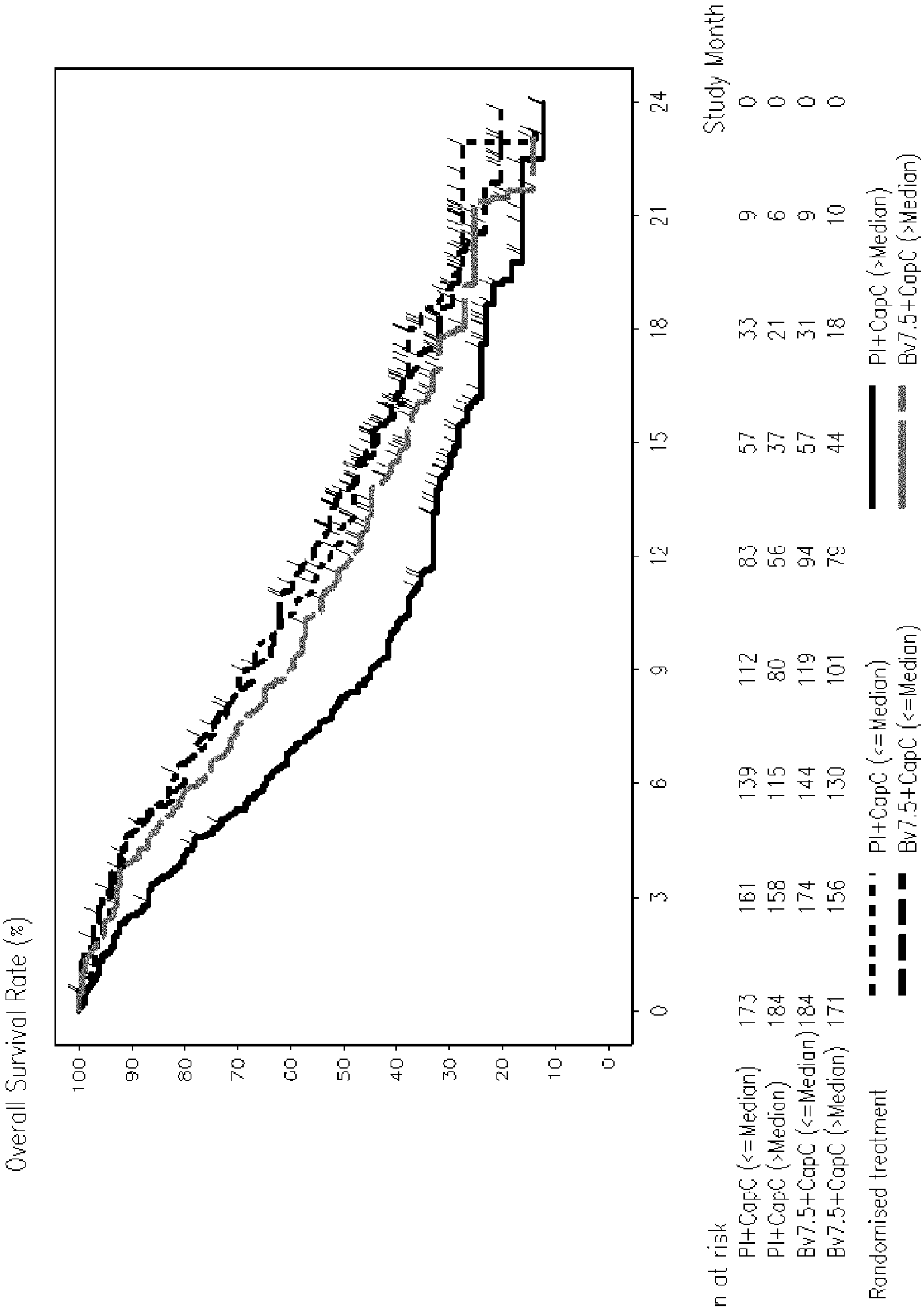

Overall Survival Rate (%)
100
90
80
70
60
50
40
30
20
10
0
0
3
6
9
12
15
18
21
24
Study Month
n at risk
PI+CapC (<=Median) 173 161 139 112 83 57 33 9 0
PI+CapC (>Median) 184 158 115 80 56 37 21 6 0
Bv7.5+CapC (<=Median) 184 174 144 119 94 57 31 9 0
Bv7.5+CapC (>Median) 171 156 130 101 79 44 18 10 0
Randomised treatment
PI+CapC (<=Median)
Bv7.5+CapC (<=Median)
PI+CapC (>Median)
Bv7.5+CapC (>Median)

**Figure 13B**

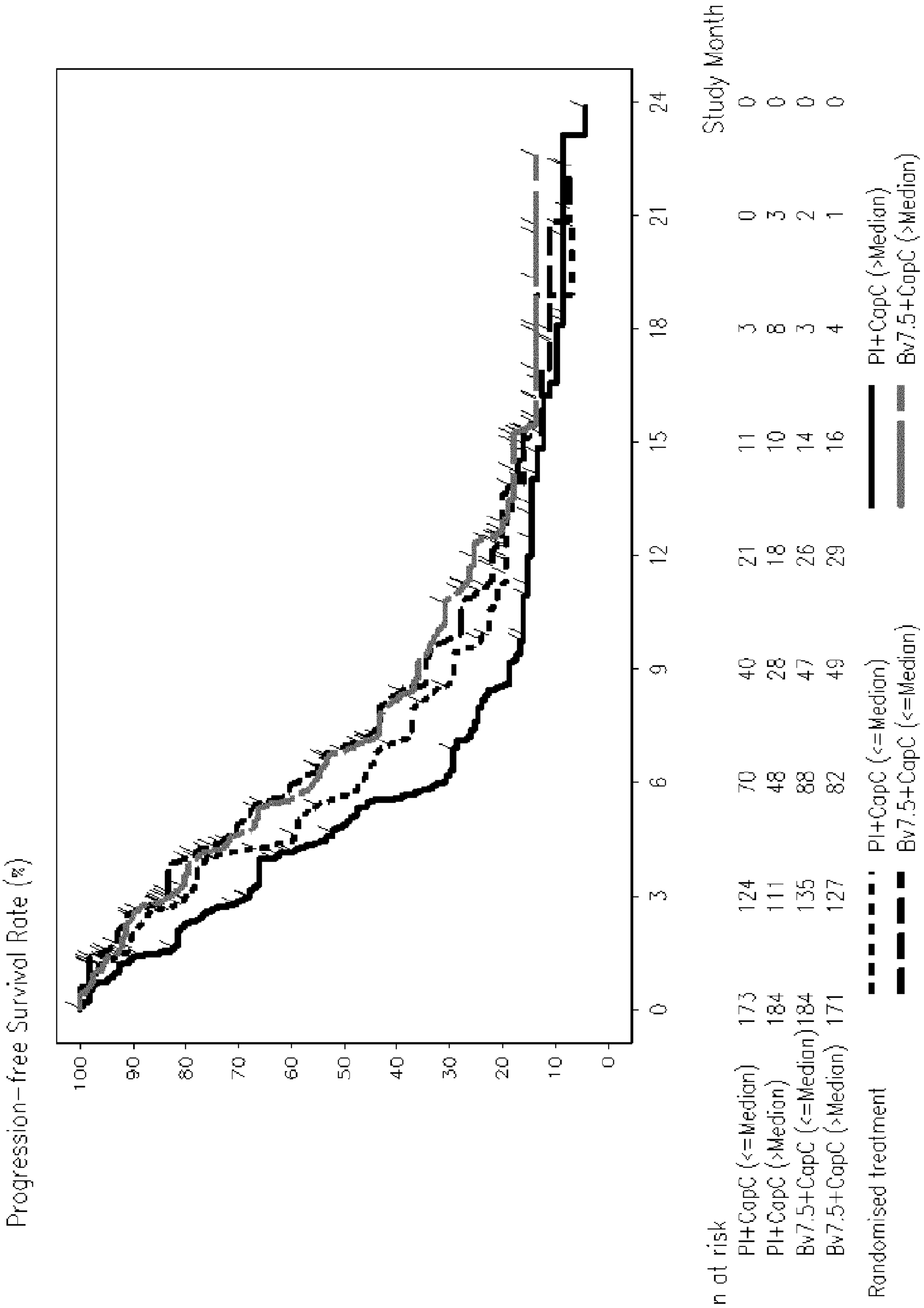

Progression-free Survival Rate (%)
100
90
80
70
60
50
40
30
20
10
0
0
3
6
9
12
15
18
21
24
Study Month
n at risk
PI+CapC (<=Median) 173 124 70 40 21 11 3 0 0
PI+CapC (>Median) 184 111 48 28 18 10 8 3 0
Bv7.5+CapC (<=Median) 184 135 88 47 26 14 3 2 0
Bv7.5+CapC (>Median) 171 127 82 49 29 16 4 1 0
Randomised treatment
PI+CapC (<=Median)
Bv7.5+CapC (<=Median)
PI+CapC (>Median)
Bv7.5+CapC (>Median)

**Figure 14A**

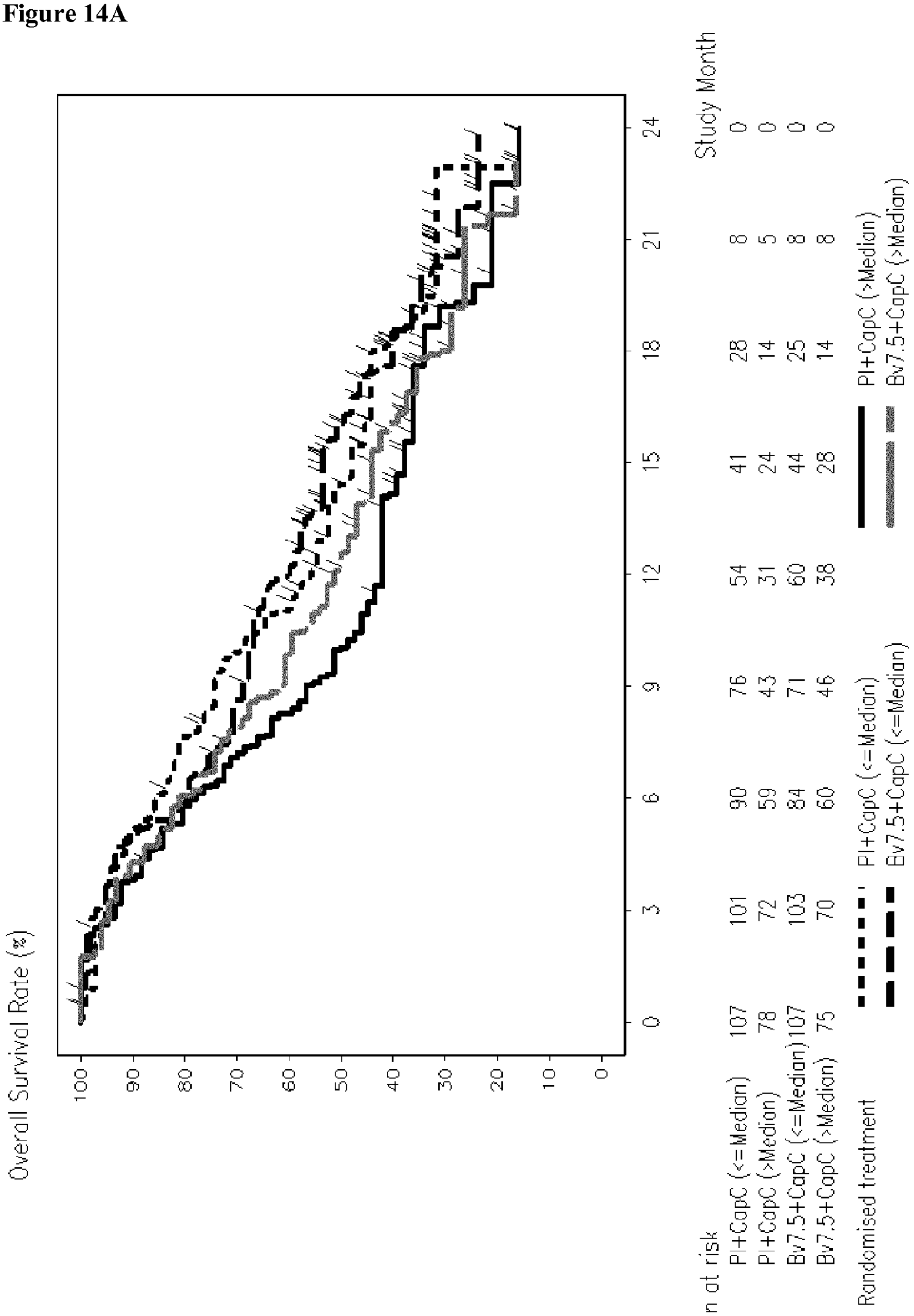

Overall Survival Rate (%)
100
90
80
70
60
50
40
30
20
10
0
0
3
6
9
12
15
18
21
24
Study Month
n at risk
Pl+CapC (<=Median) 107 101 90 76 54 41 28 8 0
Pl+CapC (>Median) 78 72 59 43 31 24 14 5 0
Bv7.5+CapC (<=Median) 107 103 84 71 60 44 25 8 0
Bv7.5+CapC (>Median) 75 70 60 46 38 28 14 8 0
Randomised treatment
Pl+CapC (<=Median)
Pl+CapC (>Median)
Bv7.5+CapC (<=Median)
Bv7.5+CapC (>Median)

**Figure 14B**

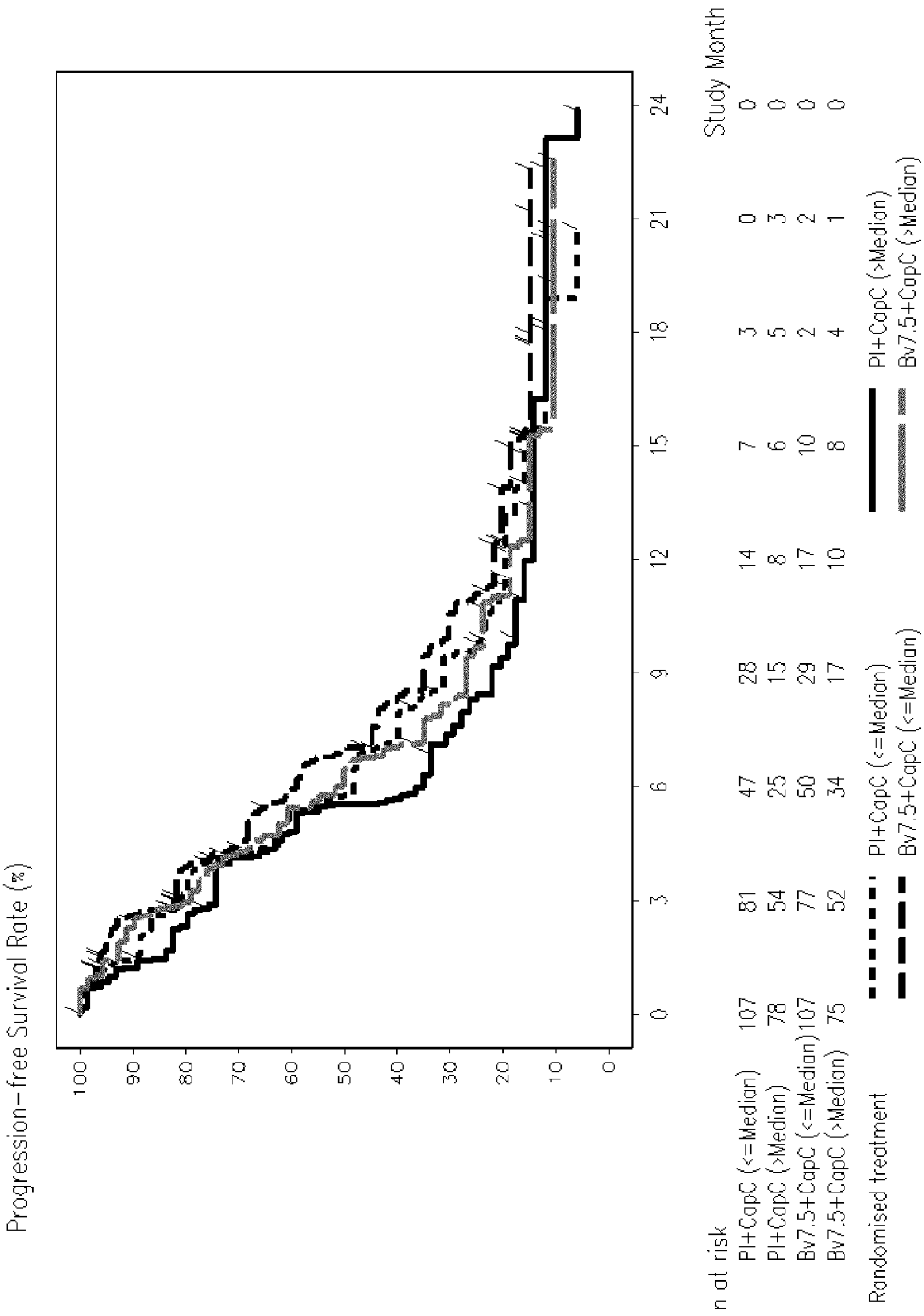
Progression-free Survival Rate (%)
100
90
80
70
60
50
40
30
20
10
0
0
3
6
9
12
15
18
21
24
Study Month
n at risk
PI+CapC (<=Median) 107 81 47 28 14 7 3 0 0
PI+CapC (>Median) 78 54 25 15 8 6 5 3 0
Bv7.5+CapC (<=Median) 107 77 50 29 17 10 2 2 0
Bv7.5+CapC (>Median) 75 52 34 17 10 8 4 1 0
Randomised treatment
PI+CapC (<=Median)
Bv7.5+CapC (<=Median)
PI+CapC (>Median)
Bv7.5+CapC (>Median)

**Figure 15A**

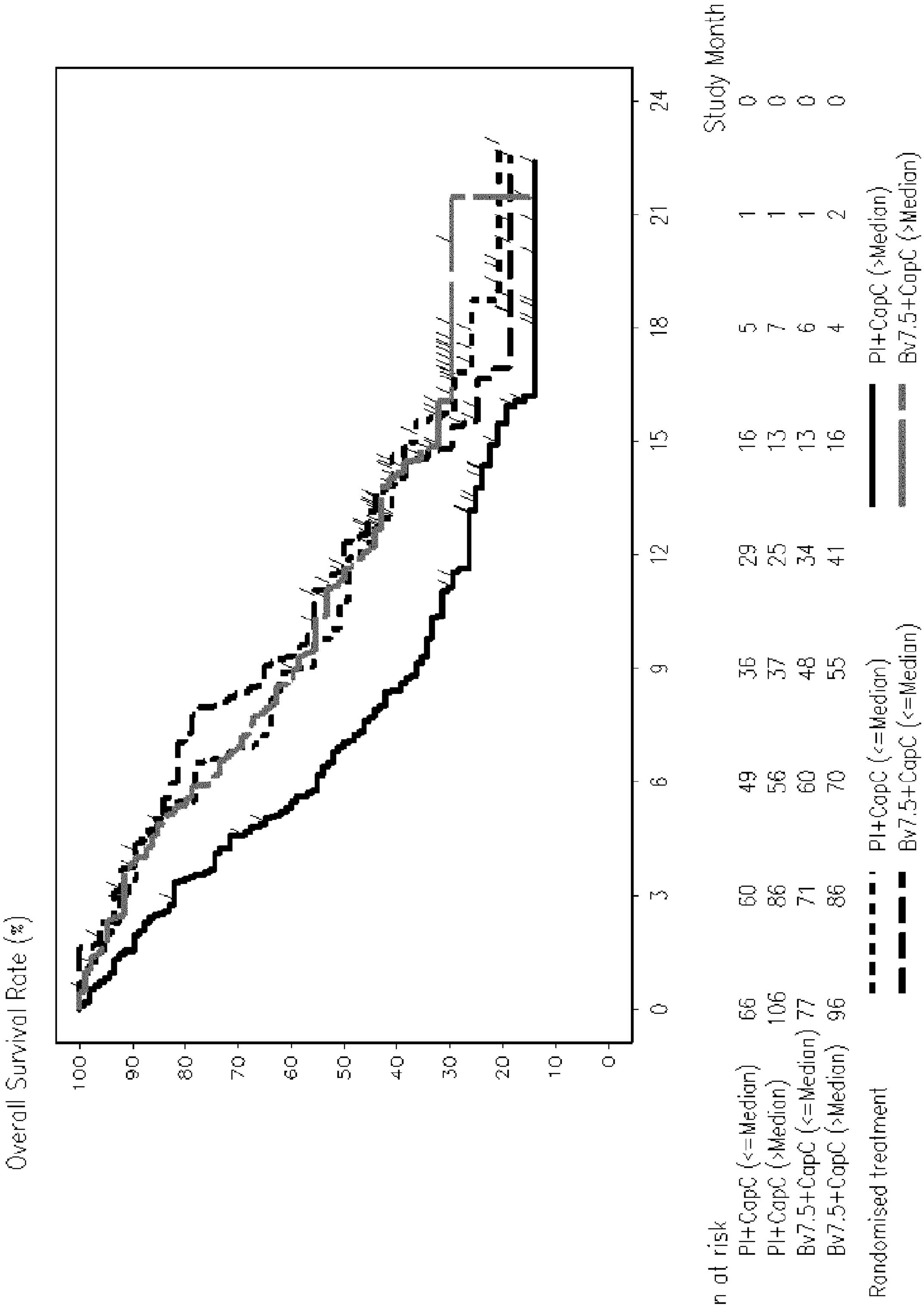
Overall Survival Rate (%)
100
90
80
70
60
50
40
30
20
10
0
0
3
6
9
12
15
18
21
24
Study Month
n at risk
PI+CapC (<=Median) 66 60 49 36 29 16 5 1 0
PI+CapC (>Median) 106 86 56 37 25 13 7 1 0
Bv7.5+CapC (<=Median) 77 71 60 48 34 13 6 1 0
Bv7.5+CapC (>Median) 96 86 70 55 41 16 4 2 0
Randomised treatment
PI+CapC (<=Median)
Bv7.5+CapC (<=Median)
PI+CapC (>Median)
Bv7.5+CapC (>Median)

**Figure 15B**

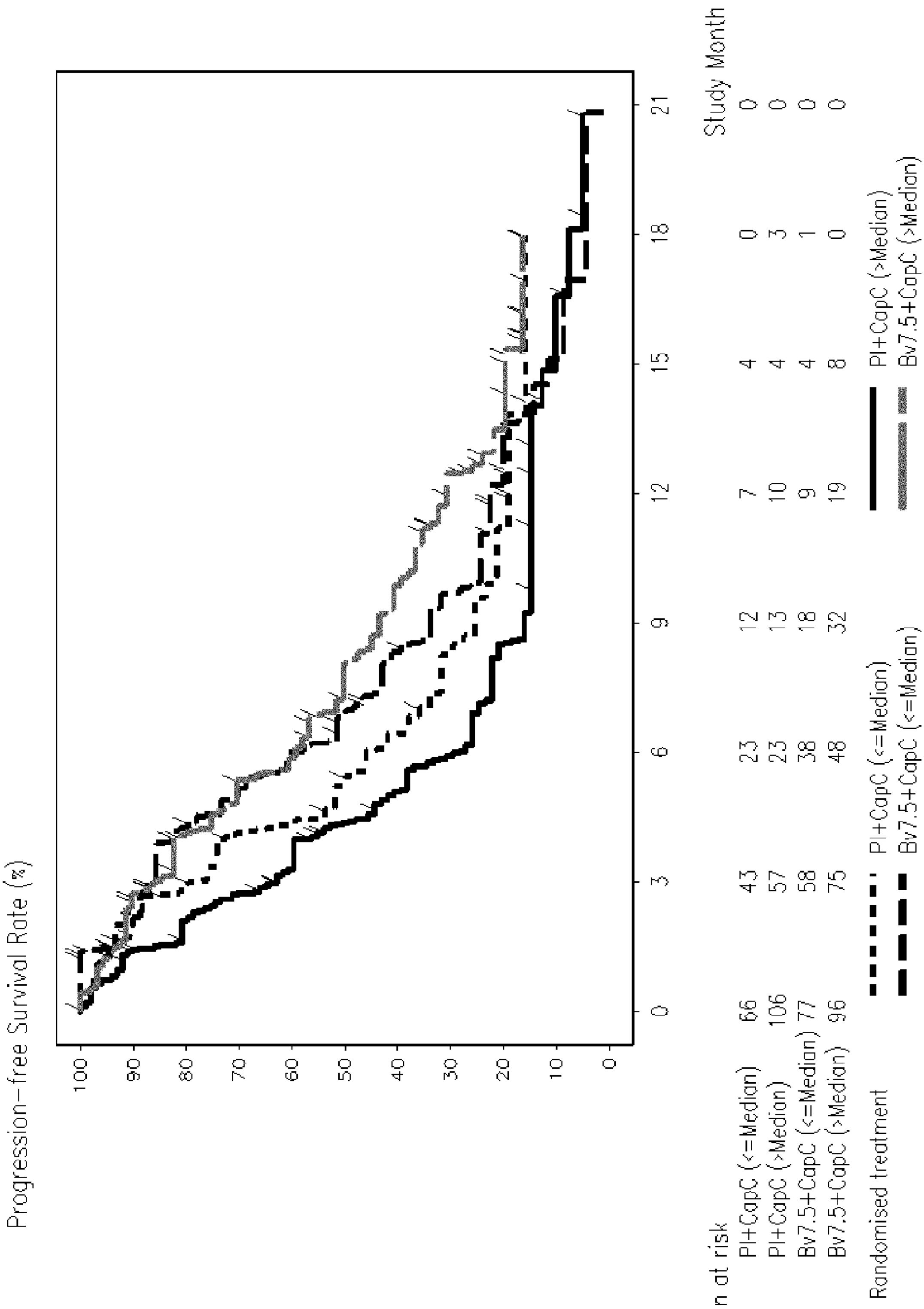
Progression-free Survival Rate (%)
100
90
80
70
60
50
40
30
20
10
0
0
3
6
9
12
15
18
21
Study Month
n at risk
Pl+CapC (<=Median) 66 43 23 12 7 4 0 0
Pl+CapC (>Median) 106 57 23 13 10 4 3 0
Bv7.5+CapC (<=Median) 77 58 38 18 9 4 1 0
Bv7.5+CapC (>Median) 96 75 48 32 19 8 0 0
Randomised treatment
Pl+CapC (<=Median)
Bv7.5+CapC (<=Median)
Pl+CapC (>Median)
Bv7.5+CapC (>Median)

**Figure 16A**

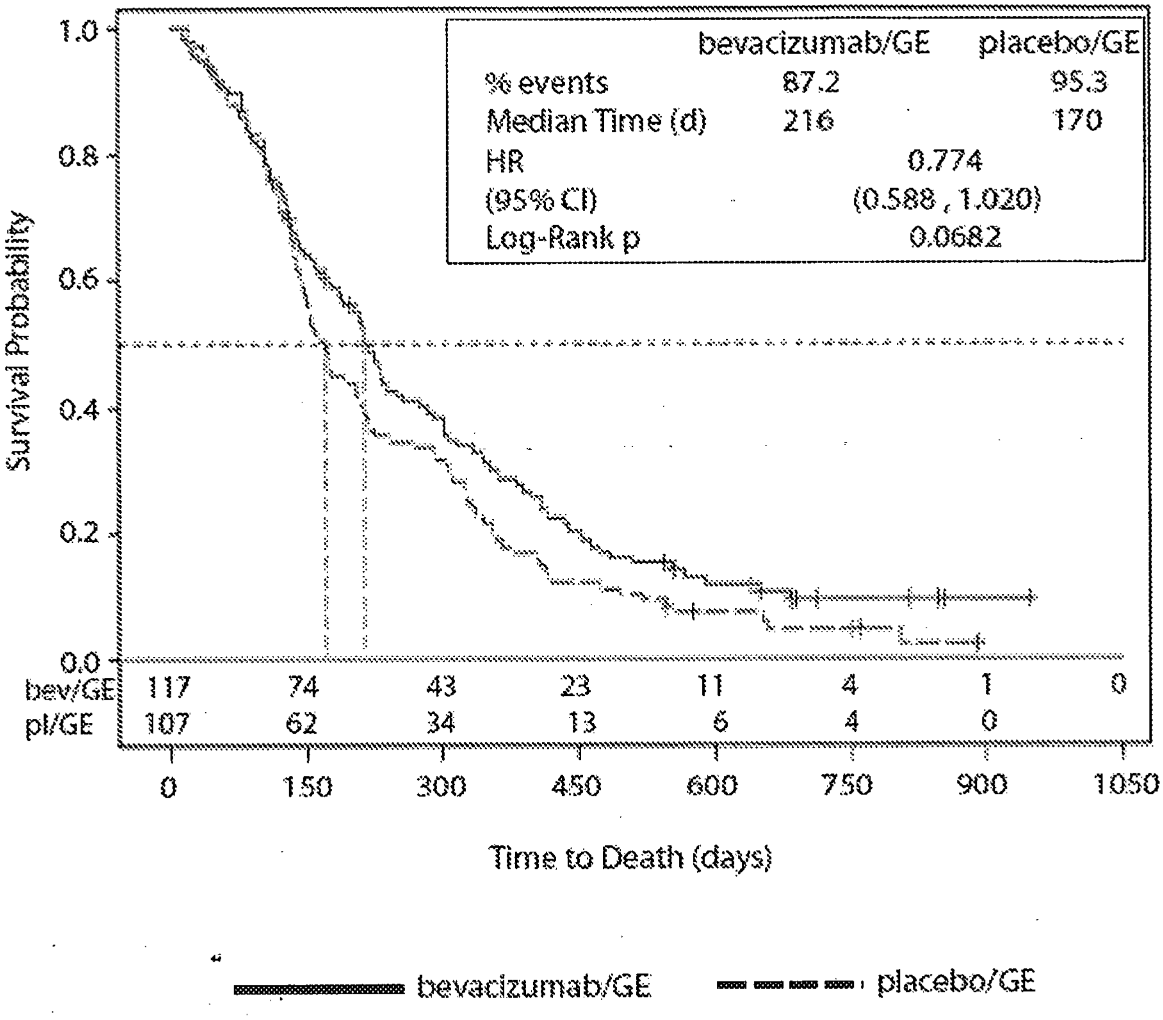
bevacizumab/GE
placebo/GE
% events 87.2 95.3
Median Time (d) 216 170
HR 0.774
(95% CI) (0.588, 1.020)
Log-Rank p 0.0682
Survival Probability
1.0
0.8
0.6
0.4
0.2
0.0
bev/GE 117 74 43 23 11 4 1 0
pl/GE 107 62 34 13 6 4 0
0 150 300 450 600 750 900 1050
Time to Death (days)
bevacizumab/GE
placebo/GE

**Figure 16B**

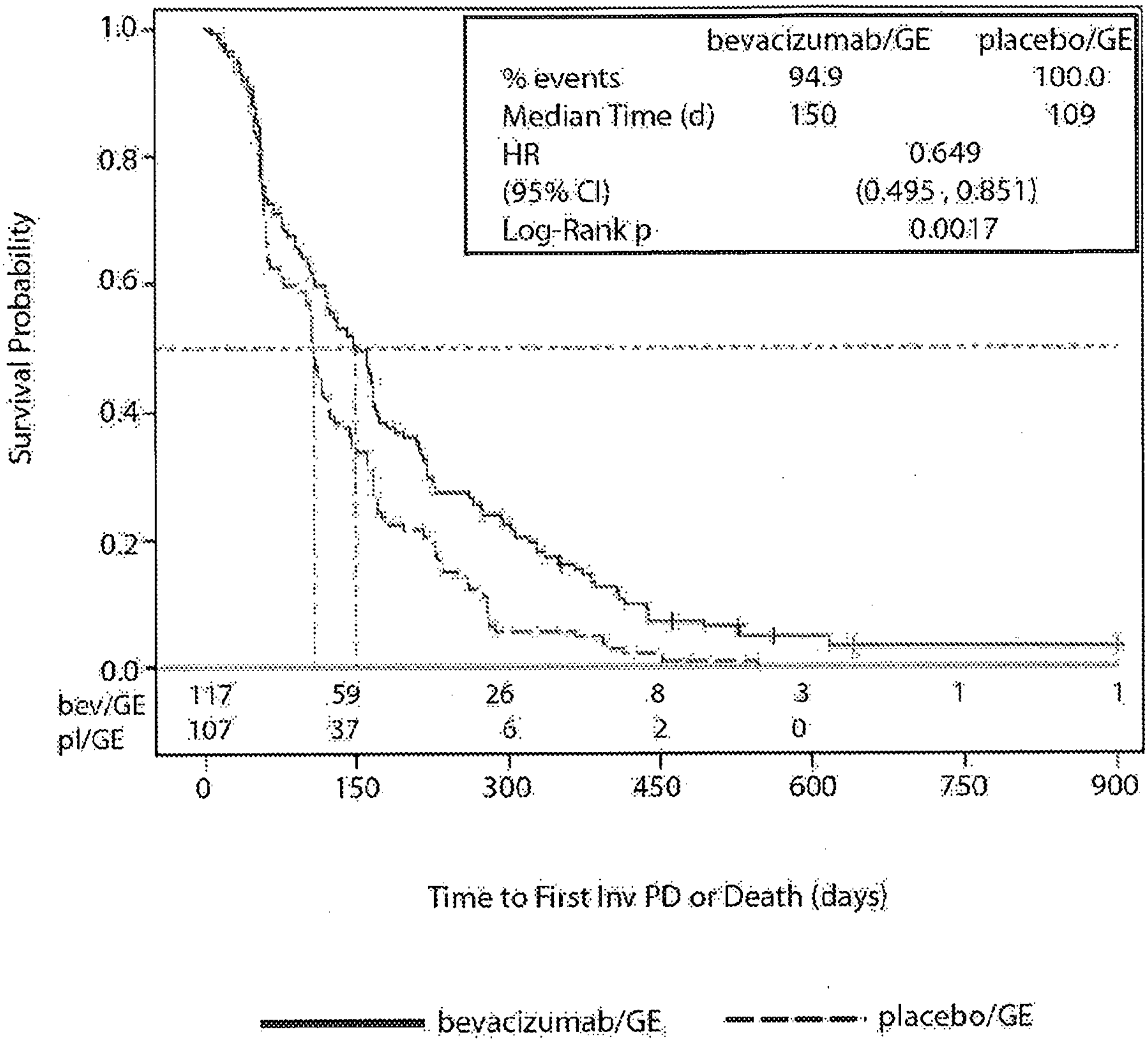
bevacizumab/GE placebo/GE
% events 94.9 100.0
Median Time (d) 150 109
HR 0.649
(95% CI) (0.495 , 0.851)
Log-Rank p 0.0017
Survival Probability
1.0
0.8
0.6
0.4
0.2
0.0
bev/GE 117 59 26 8 3 1 1
pl/GE 107 37 6 2 0
0 150 300 450 600 750 900
Time to First Inv PD or Death (days)
bevacizumab/GE
placebo/GE

Figure 17A

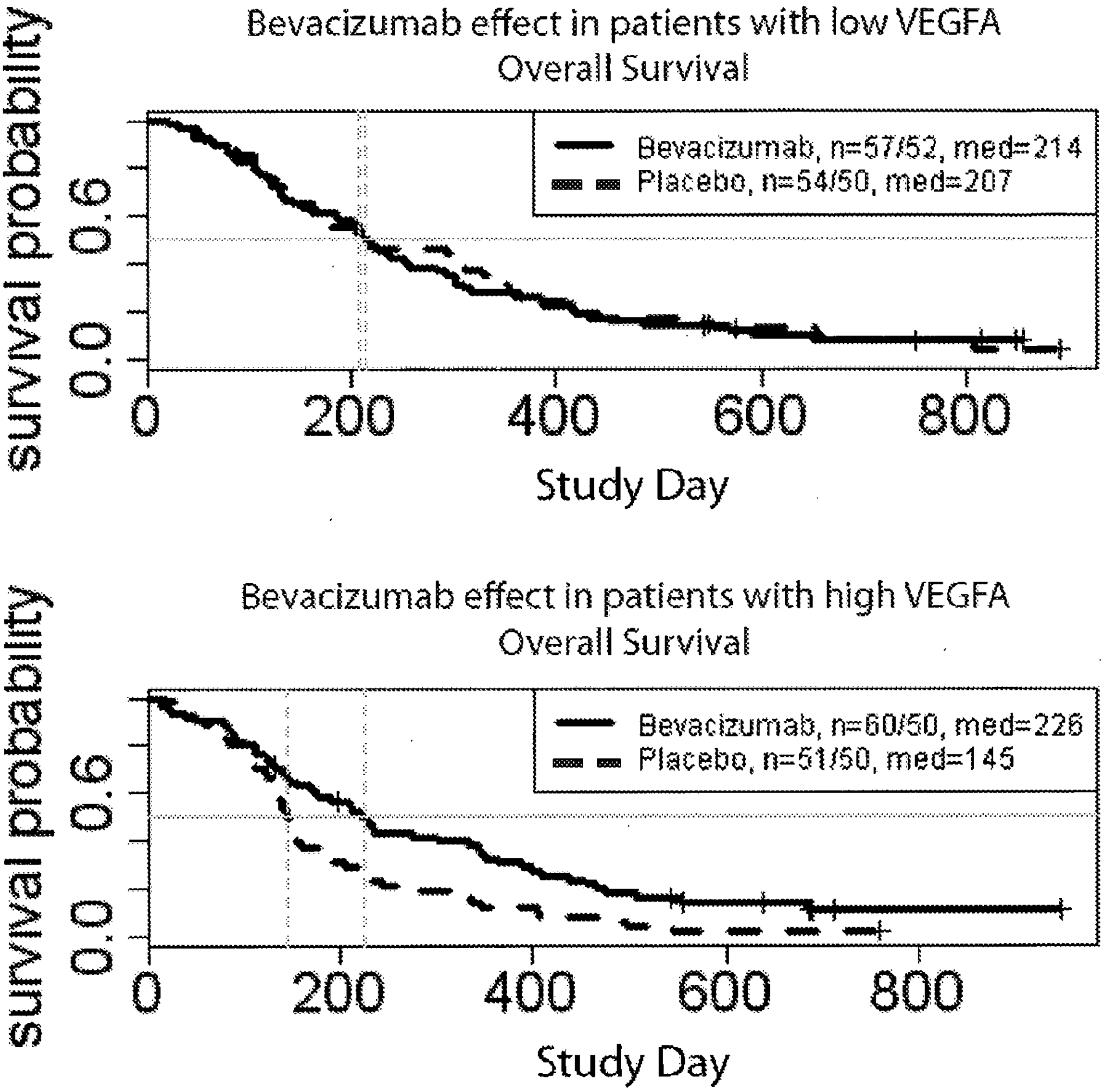
Bevacizumab effect in patients with low VEGFA
Overall Survival
Bevacizumab, n=57/52, med=214
Placebo, n=54/50, med=207
survival probability
0.0
0.6
0
200
400
600
800
Study Day
Bevacizumab effect in patients with high VEGFA
Overall Survival
Bevacizumab, n=60/50, med=226
Placebo, n=51/50, med=145
survival probability
0.0
0.6
0
200
400
600
800
Study Day

Figure 17B

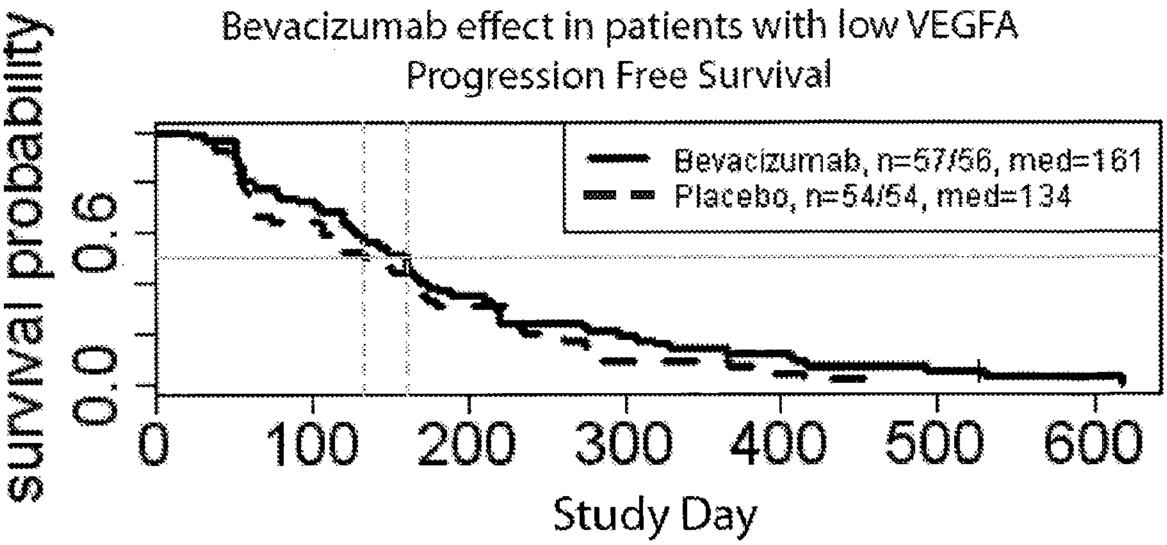
Bevacizumab effect in patients with low VEGFA
Progression Free Survival
survival probability
0.0
0.6
Bevacizumab, n=57/56, med=161
Placebo, n=54/54, med=134
0
100
200
300
400
500
600
Study Day

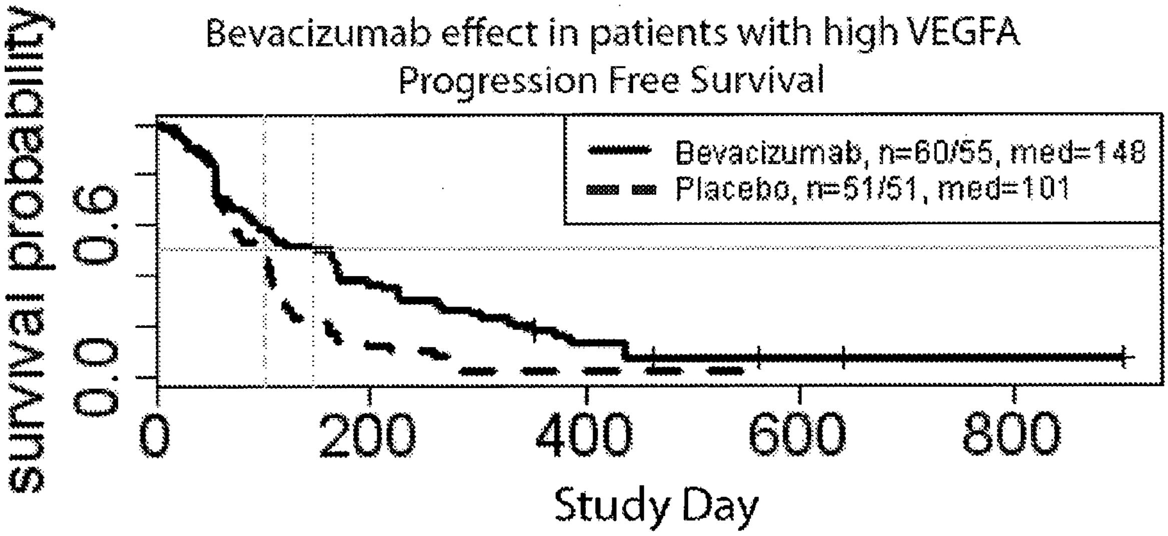
Bevacizumab effect in patients with high VEGFA
Progression Free Survival
survival probability
0.0
0.6
Bevacizumab, n=60/55, med=148
Placebo, n=51/51, med=101
0
200
400
600
800
Study Day

Figure 18A

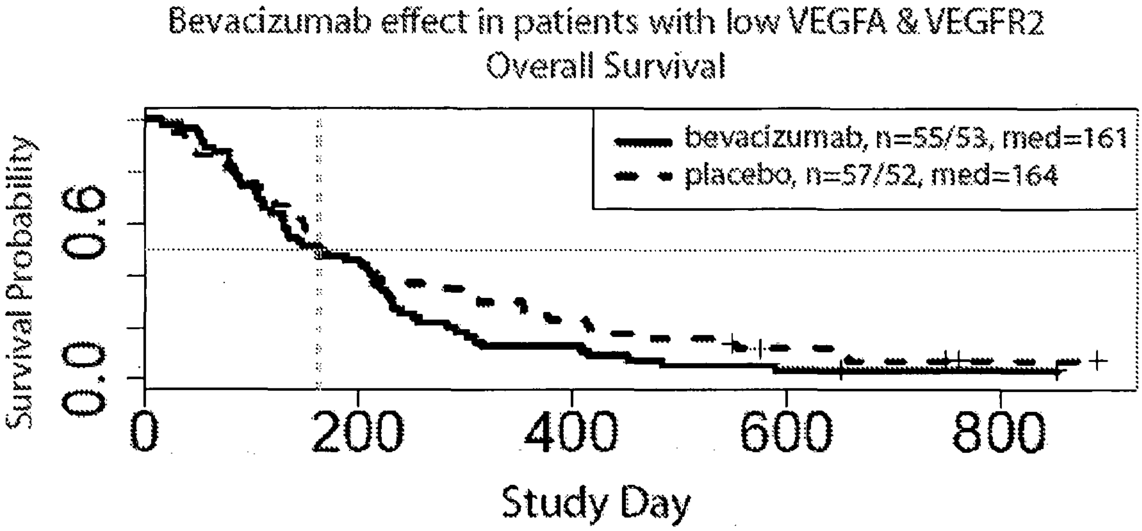
Bevacizumab effect in patients with low VEGFA & VEGFR2
Overall Survival
bevacizumab, n=55/53, med=161
placebo, n=57/52, med=164
Survival Probability
0.0
0.6
0
200
400
600
800
Study Day

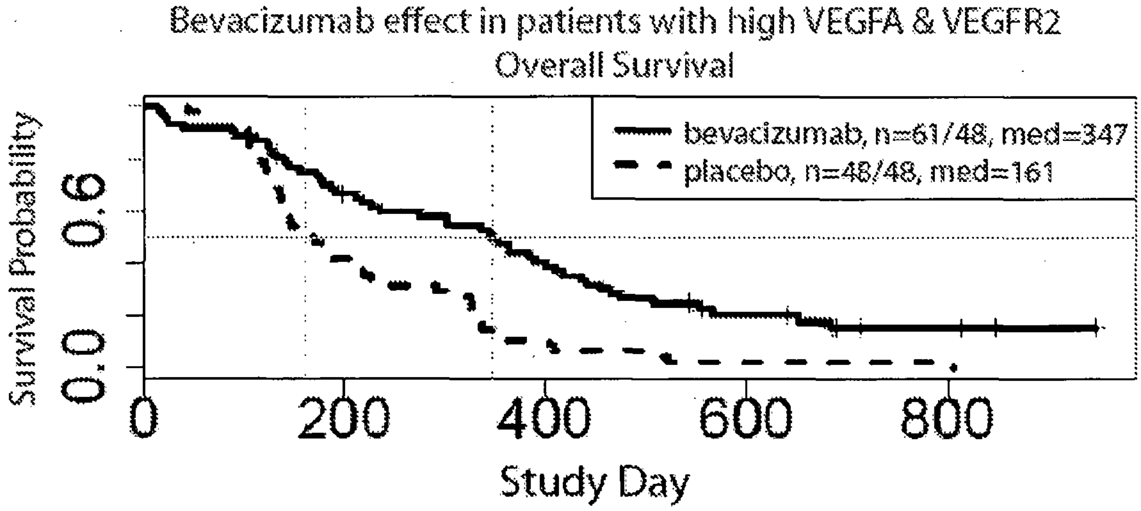
Bevacizumab effect in patients with high VEGFA & VEGFR2
Overall Survival
bevacizumab, n=61/48, med=347
placebo, n=48/48, med=161
Survival Probability
0.0
0.6
0
200
400
600
800
Study Day

Figure 18B

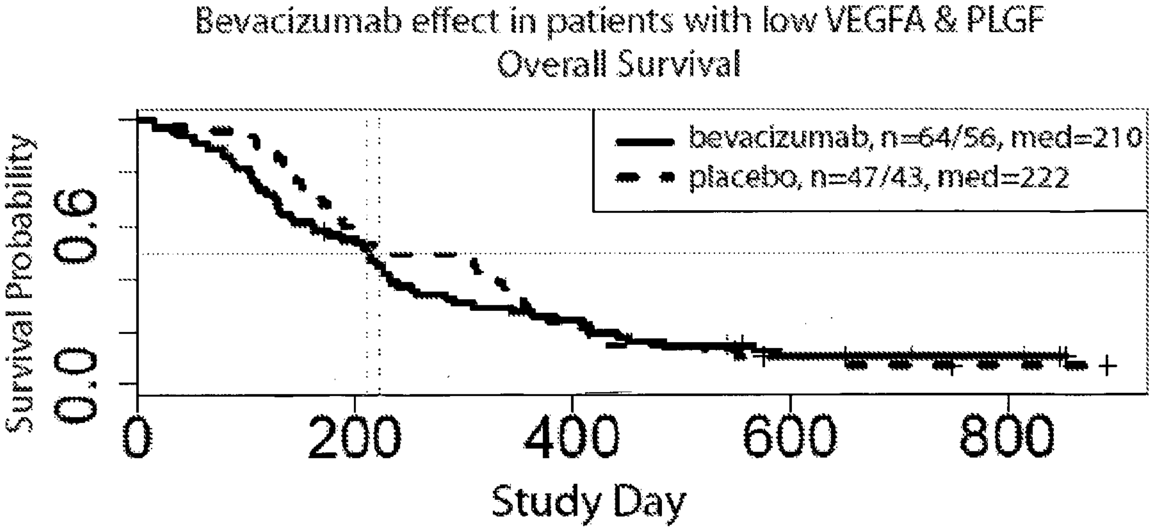
Bevacizumab effect in patients with low VEGFA & PLGF
Overall Survival
bevacizumab, n=64/56, med=210
placebo, n=47/43, med=222
Survival Probability
0.0
0.6
0
200
400
600
800
Study Day

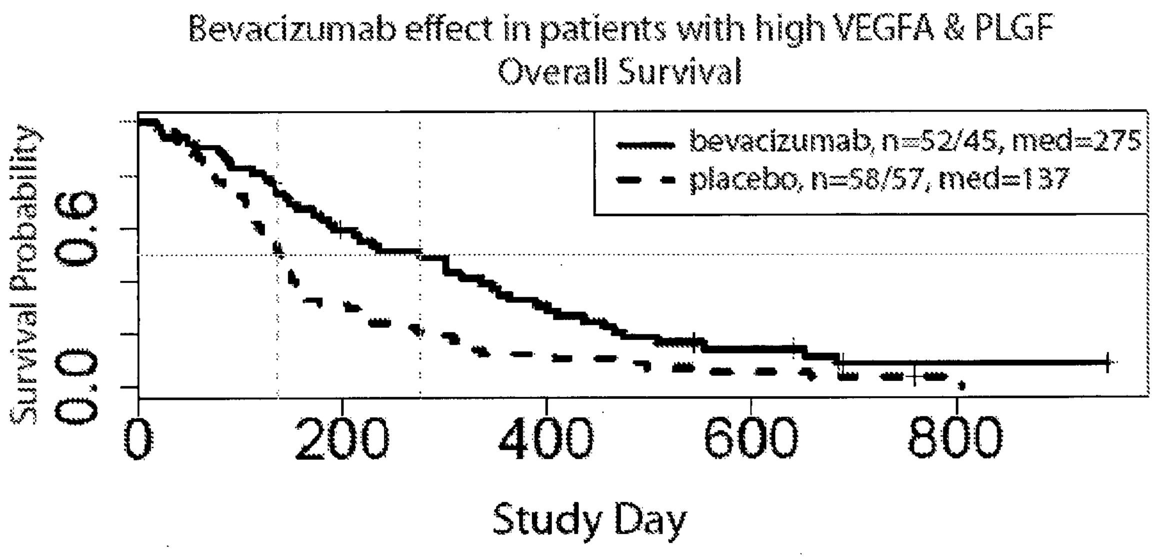
Bevacizumab effect in patients with high VEGFA & PLGF
Overall Survival
bevacizumab, n=52/45, med=275
placebo, n=58/57, med=137
Survival Probability
0.0
0.6
0
200
400
600
800
Study Day

**Figure 19A**

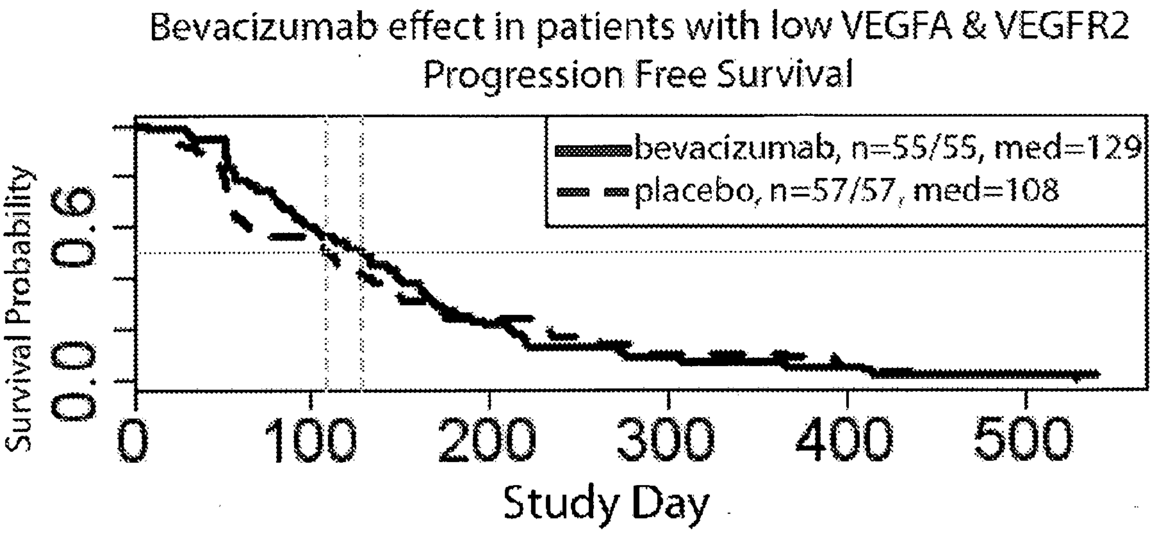
Bevacizumab effect in patients with low VEGFA & VEGFR2
Progression Free Survival
bevacizumab, n=55/55, med=129
placebo, n=57/57, med=108
Survival Probability
0.0
0.6
0
100
200
300
400
500
Study Day

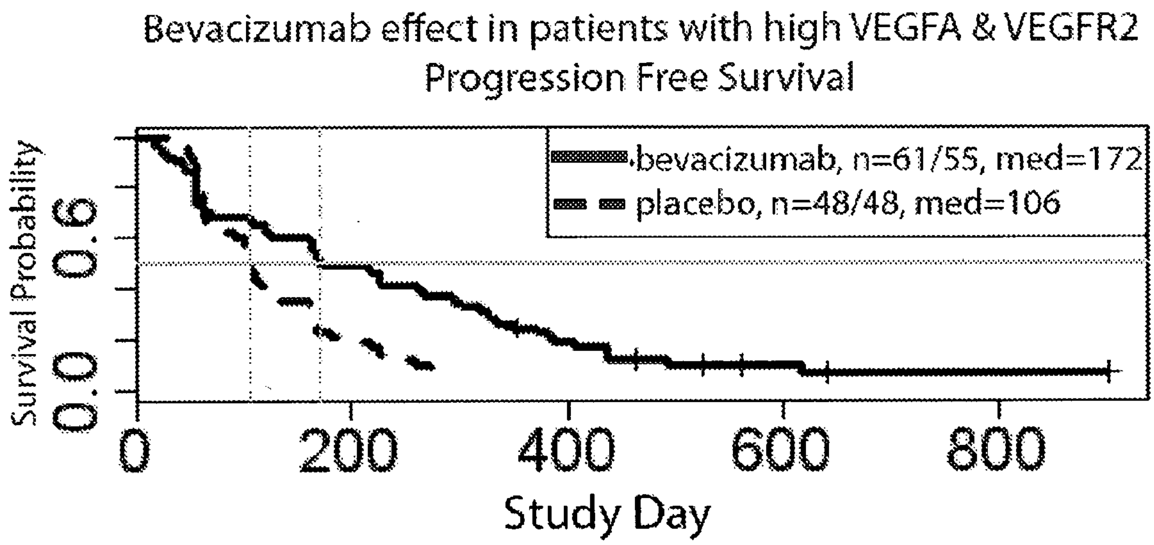
Bevacizumab effect in patients with high VEGFA & VEGFR2
Progression Free Survival
bevacizumab, n=61/55, med=172
placebo, n=48/48, med=106
Survival Probability
0.0
0.6
0
200
400
600
800
Study Day

Figure 19B

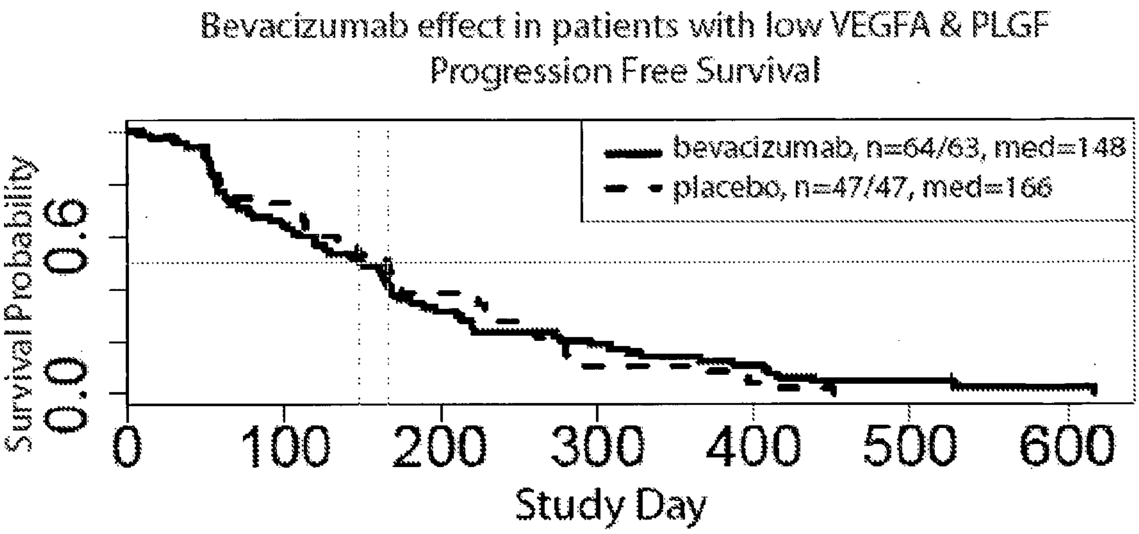
Bevacizumab effect in patients with low VEGFA & PLGF
Progression Free Survival
bevacizumab, n=64/63, med=148
placebo, n=47/47, med=166
Survival Probability
0.0
0.6
0
100
200
300
400
500
600
Study Day

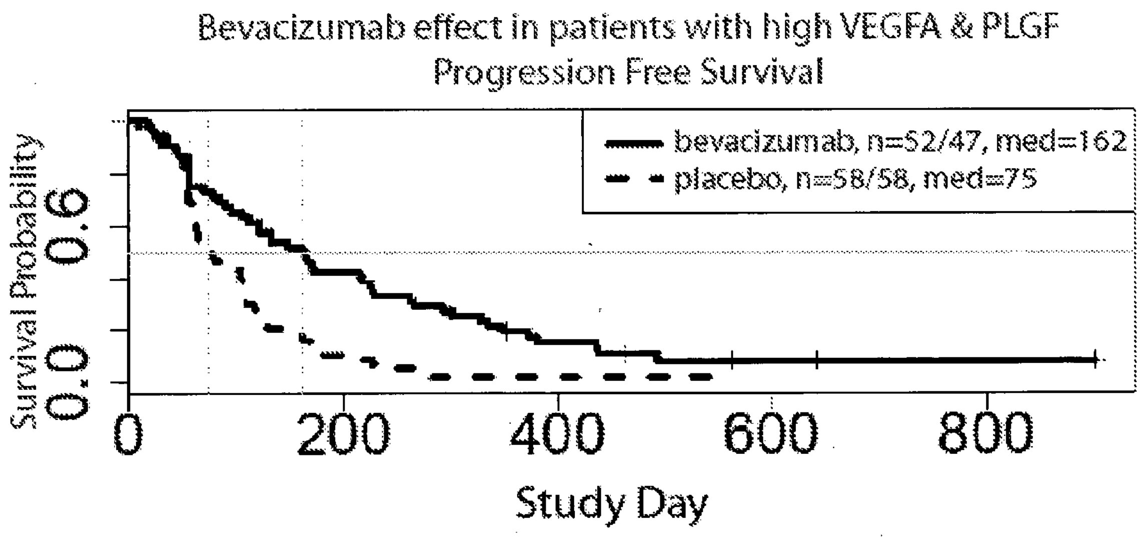
Bevacizumab effect in patients with high VEGFA & PLGF
Progression Free Survival
bevacizumab, n=52/47, med=162
placebo, n=58/58, med=75
Survival Probability
0.0
0.6
0
200
400
600
800
Study Day

**Figure 20A**

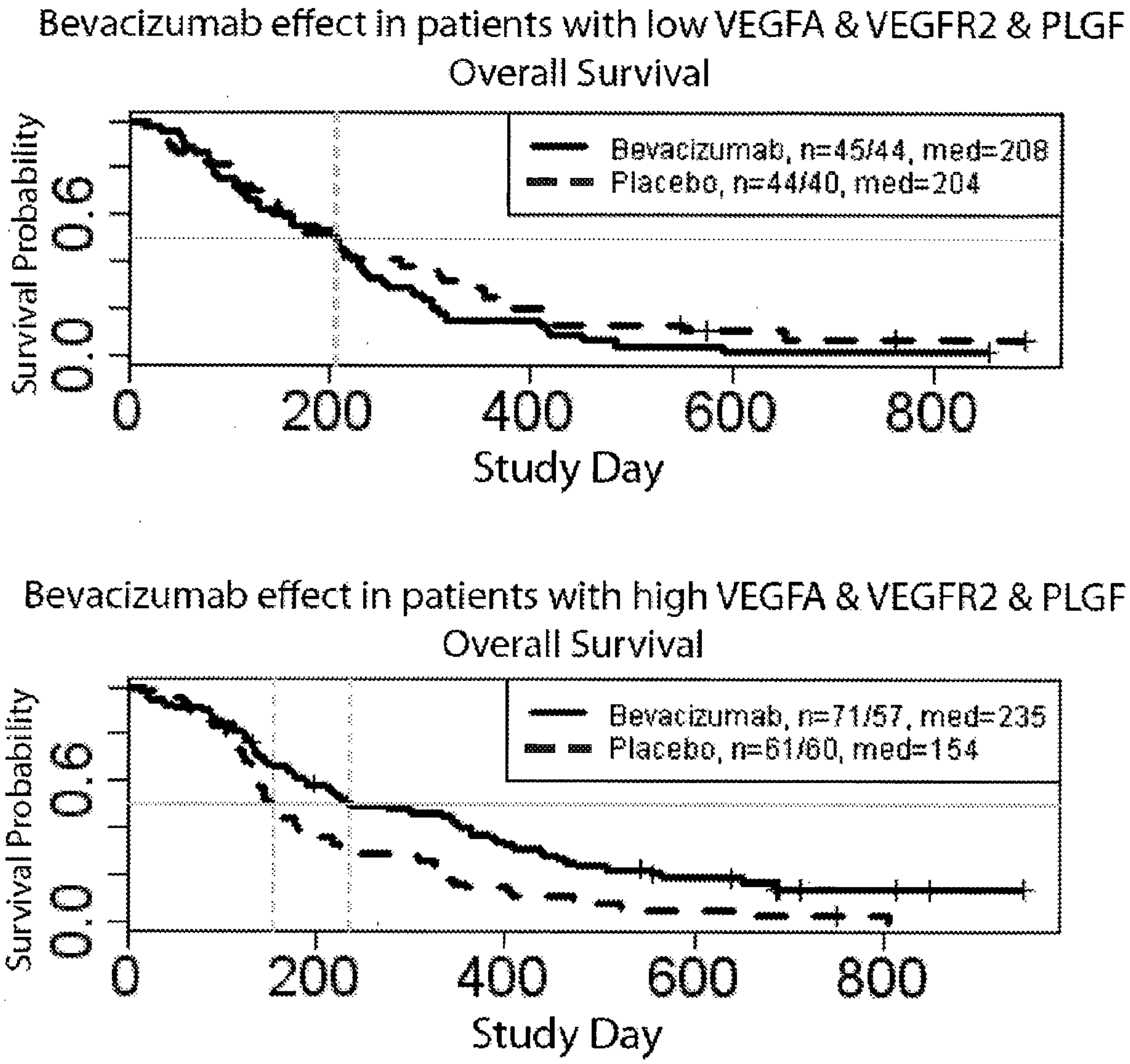
Bevacizumab effect in patients with low VEGFA & VEGFR2 & PLGF
Overall Survival
Bevacizumab, n=45/44, med=208
Placebo, n=44/40, med=204
Survival Probability
0.0
0.6
0
200
400
600
800
Study Day
Bevacizumab effect in patients with high VEGFA & VEGFR2 & PLGF
Overall Survival
Bevacizumab, n=71/57, med=235
Placebo, n=61/60, med=154
Survival Probability
0.0
0.6
0
200
400
600
800
Study Day

**Figure 20B**

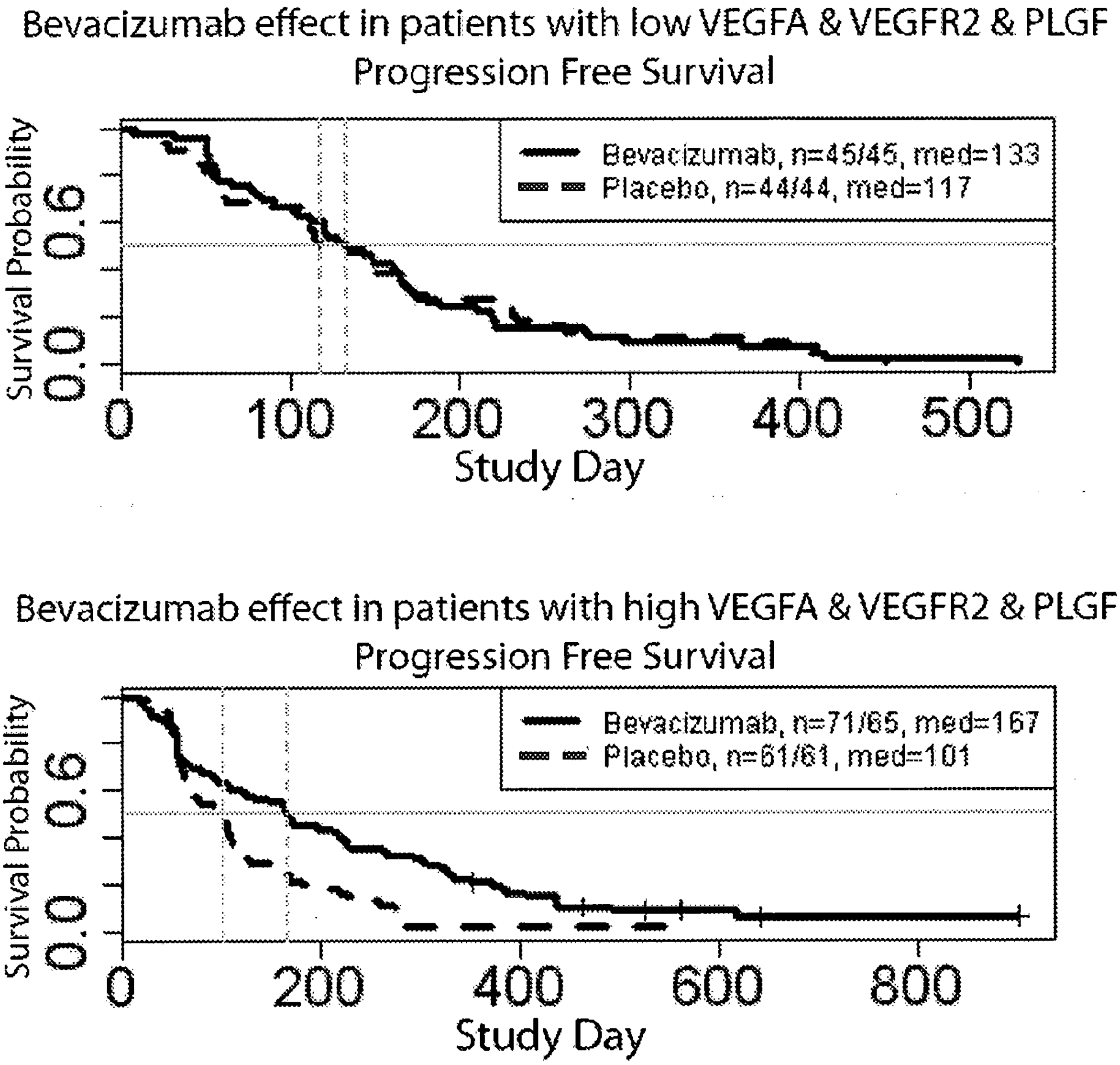
Bevacizumab effect in patients with low VEGFA & VEGFR2 & PLGF
Progression Free Survival
Survival Probability
0.0
0.6
Bevacizumab, n=45/45, med=133
Placebo, n=44/44, med=117
0
100
200
300
400
500
Study Day
Bevacizumab effect in patients with high VEGFA & VEGFR2 & PLGF
Progression Free Survival
Survival Probability
0.0
0.6
Bevacizumab, n=71/65, med=167
Placebo, n=61/61, med=101
0
200
400
600
800
Study Day

**Figure 21**

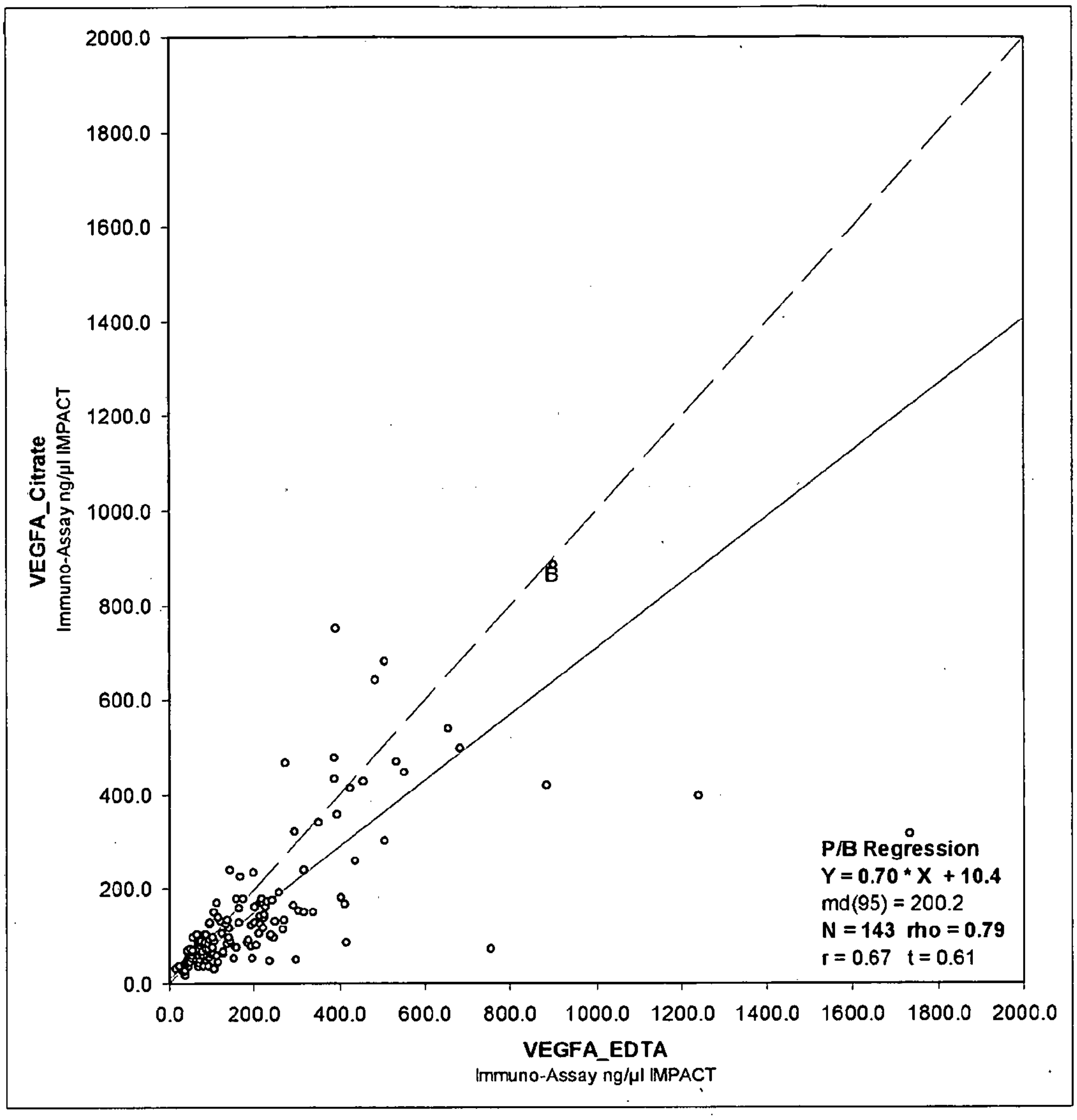
2000.0
1800.0
1600.0
1400.0
1200.0
1000.0
800.0
600.0
400.0
200.0
0.0
VEGFA_Citrate
Immuno-Assay ng/µl IMPACT
0.0 200.0 400.0 600.0 800.0 1000.0 1200.0 1400.0 1600.0 1800.0 2000.0
VEGFA_EDTA
Immuno-Assay ng/µl IMPACT
B
P/B Regression
Y = 0.70 * X + 10.4
md(95) = 200.2
N = 143 rho = 0.79
r = 0.67 t = 0.61

Europäisches Patentamt
European Patent Office
Office européen des brevets

# EUROPEAN SEARCH REPORT

Application Number
EP 14 18 8048

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/060777 A2 (GENENTECH INC [US]; MENG YU-JU G [US]; HONG KYU H [US]; GUTIERREZ JOHN) 22 May 2008 (2008-05-22) * paragraphs [0013], [0015], [0017], [0085], [0099] - [0112] * ----- | 10-15 | INV. G01N33/574 |
| X | KIM K J ET AL: "THE VASCULAR ENDOTHELIAL GROWTH FACTOR PROTEINS: IDENTIFICATION OF BIOLOGICALLY RELEVANT REGIONS BY NEUTRALIZING MONOCLONAL ANTIBODIES", GROWTH FACTORS, HARWOOD ACADEMIC PUBLISHERS GMBH, XX, vol. 7, no. 1, 1 January 1992 (1992-01-01), pages 53-64, XP000612291, ISSN: 0897-7194 * abstract * * page 59, column 1 * ----- | 10-15 | |
| X | Anonymous: "Anti-human VEGF-A (#3C5)", CoaChrom Diagnostica GmbH , 2009, XP002660177, Retrieved from the Internet: URL:http://www.coachrom.com/docs/rel/101-M56.pdf [retrieved on 2011-09-27] * the whole document * ----- | 10-15 | TECHNICAL FIELDS SEARCHED (IPC) G01N |
| X | R&D Systems: "Human VEGF Antibody", , 2009, XP002660178, Retrieved from the Internet: URL:http://www.rndsystems.com/pdf/mab293.pdf [retrieved on 2011-09-27] * the whole document * ----- -/-- | 10-15 | |

The present search report has been drawn up for all claims

1

| Place of search | Date of completion of the search | Examiner |
|---|---|---|
| Munich | 9 February 2015 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 18 8048

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MILES DAVID W ET AL: "Phase III study of bevacizumab plus docetaxel compared with placebo plus docetaxel for the first-line treatment of human epidermal growth factor receptor 2-negative metastatic breast cancer.", JOURNAL OF CLINICAL ONCOLOGY : OFFICIAL JOURNAL OF THE AMERICAN SOCIETY OF CLINICAL ONCOLOGY 10 JUL 2010 LNKD- PUBMED:20498403, vol. 28, no. 20, 10 July 2010 (2010-07-10), pages 3239-3247, XP002660179, ISSN: 1527-7755 * the whole document * ----- | 1-9 | |
| Y | BERNARD PAULE ET AL: "Soluble Isoforms of Vascular Endothelial Growth Factor Are Predictors of Response to Sunitinib in Metastatic Renal Cell Carcinomas", PLOS ONE, vol. 5, no. 5, E10715, 1 January 2010 (2010-01-01), pages 1-5, XP055008441, ISSN: 1932-6203, DOI: 10.1371/journal.pone.0010715 * the whole document * * in particular page E10715, right hand column, last paragraph. * ----- | 1-9 | TECHNICAL FIELDS SEARCHED (IPC) |
| A | LONGO R ET AL: "Challenges for patient selection with VEGF inhibitors", CANCER CHEMOTHERAPY AND PHARMACOLOGY, vol. 60, no. 2, 17 March 2007 (2007-03-17), pages 151-170, XP019514786, SPRINGER, BERLIN, DE ISSN: 1432-0843, DOI: 10.1007/S00280-006-0403-6 * the whole document * * page 158, column 1, paragraph 3 * ----- | 1-9 | |

1

The present search report has been drawn up for all claims

| Place of search | Date of completion of the search | Examiner |
|---|---|---|
| Munich | 9 February 2015 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 18 8048

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2015

| Patent document cited in search report | Publication date | Patent family member(s) | Publication date |
|---|---|---|---|
| WO 2008060777 A2 | 22-05-2008 | AR 063125 A1 | 30-12-2008 |
| | | AU 2007319654 A1 | 22-05-2008 |
| | | BR PI0715239 A2 | 25-06-2013 |
| | | CA 2663012 A1 | 22-05-2008 |
| | | CN 101523220 A | 02-09-2009 |
| | | CN 103454434 A | 18-12-2013 |
| | | DK 2069798 T3 | 16-06-2014 |
| | | EP 2069798 A2 | 17-06-2009 |
| | | EP 2457929 A1 | 30-05-2012 |
| | | ES 2470682 T3 | 24-06-2014 |
| | | HK 1130090 A1 | 19-12-2014 |
| | | HR P20140675 T1 | 10-10-2014 |
| | | JP 2010506171 A | 25-02-2010 |
| | | KR 20090091695 A | 28-08-2009 |
| | | PT 2069798 E | 24-06-2014 |
| | | RS 53387 B | 31-10-2014 |
| | | RU 2009116611 A | 10-11-2010 |
| | | SI 2069798 T1 | 31-07-2014 |
| | | TW 200823233 A | 01-06-2008 |
| | | TW 201406778 A | 16-02-2014 |
| | | US 2008227119 A1 | 18-09-2008 |
| | | US 2010255515 A1 | 07-10-2010 |
| | | US 2013045495 A1 | 21-02-2013 |
| | | WO 2008060777 A2 | 22-05-2008 |
| | | ZA 200901518 A | 26-05-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 10170004 A
- EP 10170008 A
- US 61414853 A
- US 61414859 A
- US 61414861 A
- WO 2005012359 A
- WO 8906692 A
- US 5500362 A
- WO 9527062 A
- US 6884879 B
- WO 2005044853 A
- WO 9845331 A
- US 20030190317 A
- WO 9744453 A
- WO 9924440 A
- WO 9900797 W
- WO 9521613 A
- WO 9961422 A
- US 6534524 B
- US 5834504 A
- WO 9850356 A
- US 5883113 A
- US 5886020 A
- US 5792783 A
- US 6653308 B
- WO 9910349 A
- WO 9732856 A
- WO 9722596 A
- WO 9854093 A
- WO 9802438 A
- WO 9916755 A
- WO 9802437 A
- US 20060280747 A
- US 20070141065 A
- US 20070020267 A
- US 60991302 B
- US 7060269 B
- US 6582959 B
- US 6703020 B
- US 6054297 A
- WO 9845332 A
- WO 9630046 A
- WO 9410202 A
- EP 0666868 B1
- US 2006009360 A
- US 20050186208 A
- US 20030206899 A
- US 20030203409 A
- US 20050112126 A
- WO 9308829 A
- US 5731168 A
- WO 2009089004 A
- US 4676980 A
- US 20060025576 A
- US 20080069820 A
- WO 2009080251 A
- WO 2009080252 A
- WO 2009080253 A
- WO 2009080254 A
- WO 2010112193 A
- WO 2010115589 A
- WO 2010136172 A
- WO 2010145792 A
- WO 2010145793 A
- WO 2010040508 A
- EP 2011054504 W
- WO 2005087812 A
- WO 2009120922 A
- WO 2011039370 A
- EP 404097 A
- WO 199301161 A
- US 4816567 A
- WO 199824893 A
- WO 199634096 A
- WO 199633735 A
- WO 199110741 A
- US 5545807 A
- US 5545806 A
- US 5569825 A
- US 5625126 A
- US 5633425 A
- US 5661016 A
- US 6982321 B
- US 7087409 B
- US 6075181 A
- US 6150584 A
- WO 200492219 A, Hinton
- WO 200042072 A, Presta
- US 4016043 A
- US 4424279 A
- US 4018653 A
- US 6855508 B
- US 7541160 B
- US 20100255515 A
- EP 0939319 A
- EP 1610129 A
- WO 199607321 A
- US 4892538 A

- US 5283187 A
- WO 199325673 A
- US 6949245 B
- US 6267958 B, Andya
- US 20020136719 A1, Shenoy
- US 3773919 A

**Non-patent literature cited in the description**


- **FERRARA.** *Mol. Biol. Cell,* 2010, vol. 21, 687
- **LEUNG et al.** *Science,* 1989, vol. 246, 1306
- **HOUCK et al.** *Mol. Endocrin.,* 1991, vol. 5, 1806
- **KIM et al.** *Growth Factors,* 1992, vol. 7 (1), 53-64
- **FERRARA ; DAVIS-SMYTH.** *Endocrine Rev.,* 1997, vol. 18, 4-25
- **FERRARA.** *J. Mol. Med.,* 1999, vol. 77, 527-543
- **CARMELIET et al.** *Nature,* 1996, vol. 380, 435-439
- **FERRARA et al.** *Nature,* 1996, vol. 380, 439-442
- **FERRARA et al.** *Nature Med.,* 1998, vol. 4, 336-340
- **GERBER et al.** *Nature Med.,* 1999, vol. 5, 623-628
- **CEBE-SUAREZ et al.** *Cell. Mol. Life Sci.,* 2006, vol. 63, 601-615
- **GUERRIN et al.** *J. Cell Physiol.,* 1995, vol. 164, 385-394
- **OBERG-WELSH et al.** *Mol. Cell. Endocrinol.,* 1997, vol. 126, 125-132
- **SONDELL et al.** *J. Neurosci.,* 1999, vol. 19, 5731-5740
- **PRESTA et al.** *Cancer Res.,* 1997, vol. 57, 4593-4599
- **PRESTA et al.** *Cancer Res,* 1997, vol. 57, 4593-4599
- **POPKOV et al.** *Journal of Immunological Methods,* 2004, vol. 288, 149-164
- **LIANG et al.** *J Biol Chem,* 2006, vol. 281, 951-961
- **MILSTEIN, C. ; CUELLO, A.C.** *Nature,* 1983, vol. 305, 537-540
- **TRAUNECKER, A. et al.** *EMBO J.,* 1991, vol. 10, 3655-3659
- **BRENNAN, M. et al.** *Science,* 1985, vol. 229, 81-83
- **KOSTELNY, S.A. et al.** *J. Immunol.,* 1992, vol. 148, 1547-1553
- **HOLLIGER, P. et al.** *Proc. Natl. Acad. Sci. USA,* 1993, vol. 90, 6444-6448
- **GRUBER, M et al.** *J. Immunol.,* 1994, vol. 152, 5368-5374
- **TUTT, A. et al.** *J. Immunol.,* 1991, vol. 147, 60-69
- **KABAT et al.** Sequences of Proteins of Immunological Interest. National Institute of Health, 1991
- **ABBAS et al.** Cellular and Mol. Immunology. W. B. Saunders, Co, 2000
- **PLUECKTHUN.** The Pharmacology of Mono-clonal Antibodies. Springer-Verlag, 1994, vol. 113, 269-315
- **HUDSON et al.** *Nat. Med.,* 2003, vol. 9, 129-134
- **HOLLIGER et al.** *PNAS USA,* 1993, vol. 90, 6444-6448
- **KOHLER ; MILSTEIN.** *Nature,* 1975, vol. 256, 495-97
- **HONGO et al.** *Hybridoma,* 1995, vol. 14 (3), 253-260
- **HARLOW et al.** Antibodies: A Laboratory Manual. Cold Spring Harbor Laboratory Press, 1988
- **HAMMERLING et al.** Monoclonal Antibodies and T-Cell Hybridomas. Elsevier, 1981, 563-681
- **CLACKSON et al.** *Nature,* 1991, vol. 352, 624-628
- **MARKS et al.** *J. Mol. Biol.,* 1992, vol. 222, 581-597
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *PNAS USA,* 2004, vol. 101 (34), 12467-12472
- **LEE et al.** *J. Immunol. Methods,* 2004, vol. 284 (1-2), 119-132
- **JAKOBOVITS et al.** *PNAS USA,* 1993, vol. 90, 2551
- **JAKOBOVITS et al.** *Nature,* 1993, vol. 362, 255-258
- **BRUGGEMANN et al.** *Year in Immunol.,* 1993, vol. 7, 33
- **MARKS et al.** *Bio/Technology,* 1992, vol. 10, 779-783
- **LONBERG et al.** *Nature,* 1994, vol. 368, 856-859
- **MORRISON.** *Nature,* 1994, vol. 368, 812-813
- **FISHWILD et al.** *Nature Biotechnol,* 1996, vol. 14, 845-851
- **NEUBERGER.** *Nature Biotechnol.,* 1996, vol. 14, 826
- **LONBERG ; HUSZAR.** *Intern. Rev. Immunol.,* 1995, vol. 13, 65-93
- **MORRISON et al.** *PNAS USA,* 1984, vol. 81, 6851-6855
- **JONES et al.** *Nature,* 1986, vol. 321, 522-525
- **RIECHMANN et al.** *Nature,* 1988, vol. 332, 323-329

- **PRESTA.** *Curr. Op. Struct. Biol.,* 1992, vol. 2, 593-596
- **VASWANI ; HAMILTON.** *Ann. Allergy, Asthma & Immunol.,* 1998, vol. 1, 105-115
- **HARRIS.** *Biochem. Soc. Transactions,* 1995, vol. 23, 1035-1038
- **HURLE ; GROSS.** *Curr. Op. Biotech.,* 1994, vol. 5, 428-433
- **HOOGENBOOM ; WINTER.** *J. Mol. Biol.,* 1991, vol. 227, 381
- **MARKS et al.** *J. Mol. Biol.,* 1991, vol. 222, 581
- **COLE et al.** Monoclonal Antibodies and Cancer Therapy. Alan R. Liss, 1985, 77
- **BOERNER et al.** *J. Immunol.,* 1991, vol. 147 (1), 86-95
- **VAN DIJK ; VAN DE WINKEL.** *Curr. Opin. Pharmacol.,* 2001, vol. 5, 368-74
- **LI et al.** *PNAS USA,* 2006, vol. 103, 3557-3562
- **XU et al.** *Immunity,* 2000, vol. 13, 37-45
- **JOHNSON ; WU.** Methods in Molecular Biology. Human Press, 2003, vol. 248, 1-25
- **HAMERS-CASTERMAN et al.** *Nature,* 1993, vol. 363, 446-448
- **SHERIFF et al.** *Nature Struct. Biol.,* 1996, vol. 3, 733-736
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917
- **BARBAS et al.** *Proc Nat. Acad. Sci. USA,* 1994, vol. 91, 3809-3813
- **SCHIER et al.** *Gene,* 1995, vol. 169, 147-155
- **YELTON et al.** *J. Immunol.,* 1995, vol. 155, 1994-2004
- **JACKSON et al.** *J. Immunol.,* 1995, vol. 154 (7), 3310-9
- **HAWKINS et al.** *J. Mol. Biol.,* 1992, vol. 226, 889-896
- **DAËRON.** *Annu. Rev. Immunol.,* 1997, vol. 15, 203-234
- **RAVETCH ; KINET.** *Annu. Rev. Immunol,* 1991, vol. 9, 457-92
- **CAPEL et al.** *Immunomethods,* 1994, vol. 4, 25-34
- **DE HAAS et al.** *J. Lab. Clin. Med.,* 1995, vol. 126, 330-41
- **GUYER et al.** *J. Immunol.,* 1976, vol. 117, 587
- **KIM et al.** *J. Immunol.,* 1994, vol. 24, 249
- **GHETIE ; WARD.** *Immunology Today,* 1997, vol. 18 (12), 592-8
- **GHETIE et al.** *Nature Biotechnology,* 1997, vol. 15 (7), 637-40
- **HINTON et al.** *J. Biol. Chem.,* 2004, vol. 279 (8), 6213-6
- **SHIELDS et al.** *J. Biol. Chem.,* 2001, vol. 9 (2), 6591-6604
- **CHEN et al.** *J. Mol. Biol.,* 1999, vol. 293, 865-881
- *Angew. Chem Intl. Ed. Engl.,* 1994, vol. 33, 183-186
- Cell cycle regulation, oncogenes, and anti-neoplastic drugs. **MURAKAMI et al.** The Molecular Basis of Cancer. WB Saunders, 1995, 13
- **KOHLER et al.** Hybridoma Techniques. Cold Spring Harbor Laboratory, 1980
- **TIJSSEN.** Practice and Theory of Enzyme Immunoassays. Elsevier, 1985
- **CAMPBELL.** Monoclonal Antibody Technology. Elsevier, 1984
- **HURRELL.** Monoclonal Hybridoma Antibodies: Techniques and Applications. CRC Press, 1982
- **ZOLA.** Monoclonal Antibodies: A Manual of Techniques. CRC Press, Inc, 1987, 147-158
- **WU et al.** *J. Biol. Chem.,* 1987, vol. 262, 4429-4432
- **WAGNER et al.** *PNAS USA,* 1990, vol. 87, 3410-3414
- **ANDERSON et al.** *Science,* 1992, vol. 256, 808-813
- Chemotherapy Service. Williams & Wilkins, 1992
- The Pharmacological Bases of Therapeutics. Pergamon Press, 1990
- Remington's Pharmaceutical Sciences. Mack Publishing Co, 1990
- Pharmaceutical Dosage Forms: Parenteral Medications. Dekker, 1993
- Pharmaceutical Dosage Forms: Tablets. Dekker, 1990
- Pharmaceutical Dosage Forms: Disperse Systems. Dekker, 1990
- Dermatological and Transdermal Formulations. Marcel Dekker, 2002, vol. 119
- Remington's Pharmaceutical Sciences. 1980
- **MILES.** *J. Clin. Oncol.,* 24 May 2010
- **KANG et al.** *J. Clin. Oncol.,* 2010, vol. 28 (18S), LBA4007
- **VAN CUTSEM.** *J. Clin. Oncol.,* 2009, vol. 27, 2231-2237
- **ZHANG, H.T. et al.** *Brit. J. Cancer,* 2000, vol. 83, 63-68
- **RELF, M. et al.** *Cancer Res.,* 1997, vol. 57, 963-969